# EUROPEAN PATENT APPLICATION

(11) **EP 1 997 493 A1**
(43) Date of publication of application: **03.12.2008**
(21) Application number: 07384026.6
(22) Date of filing: 28.05.2007
(51) Int. Cl.: A61K 31/485, A61K 31/415, A61P 25/04

(54) **Combination of a 5-HT7 receptor ligand and an opioid receptor ligand**

(71) Applicant: Laboratorios del Dr. Esteve S.A., 08041 Barcelona (ES)
(72) Inventor: Romero Alonso, Luz, 08904 L'Hospitalet de Llobregat (Barcelona) (ES); Zamanillo Castanedo, Daniel, 08041 Barcelona (ES); Vela Hernández, José Miguel, 08028 Barcelona (ES); Buschmann, Helmut H, 08960 Sant Just Desvern (Barcelona) (ES)
(74) Representative: Peters, Hajo

(57) **Abstract**

The present invention refers to a combination of a 5HT7 receptor ligand and an opioid recptor ligand, especially of a 5HT7 receptor agonist and an opioid recptor agonist, a medicament comprising this combination, or the use of this combination for the treatment of the symptoms of pain, the prevention or the prophylaxis of the symptoms of pain.

## Description

### Field of the invention

The present invention refers to a combination of a 5HT7 receptor ligand and an opioid receptor ligand, especially of a 5HT7 receptor agonist and an opioid recptor agonist, a medicament comprising this combination, or the use of this combination for the treatment of the symptoms of pain, the prevention or the prophylaxis of the symptoms of pain.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

A long-standing way of treating pain - which is also currently the most common therapeutic approach - is the use of opioids, compounds binding to the receptors of the opioid system (µ, κ, δ and ORL1) with compounds binding to the µ-opioid receptor, like morphine, having the highest clinical relevance. Nevertheless, even though being highly effective as pain-killers, some of these µ-opioids (as they arev also called) do show some severe drawbacks, namely dependency and tolerance. So, for some of these µ-opioids persons having been exposed for an extended time to these compounds like morphine - especially in higher dosages - did shows signs of dependency from the continuous exposure. On the other hand, in some patients following a prolonged treatment with some of these µ-opioids like morphine the analgesic effect of the treatment severely declined, which is a dramatic situation for these patients given the circumstances of their suffering.

Many different neurotransmitter systems, ion channels and enzymes have been implicated in pain transmission, processing and controlling. Among them, serotonin (5-hydroxtryptamine [5-HT]), produced by central and peripheral serotonergic neurons but also by platelets and mast cells after tissue injury, has been described to exert algesic or analgesic effects depending on the site of action and the receptor subtype it acts on (Eide and Hole, 1993; Millan, 2002). At the peripheral level, after nerve injury, 5-HT is released in increased amounts and interacts with different 5-HT receptors present on C-fibers (Sommer, 2004). Acting in combination with other inflammatory mediators, 5-HT may ectopically excite and sensitize afferent nerve fibers, thus contributing to peripheral sensitization and hyperalgesia following inflammation and nerve injury (Beck and Handwerker, 1974; Obata et al., 2000). The central serotonin system has also been the subject of considerable research over the last twenty years. Both 5-HT and noradrenaline (NE)-mediated pathways of descending inhibition have been described extensively. These monoaminergic tracts arise from the midbrain and brainstem and terminate on the spinal cord to suppress sensory transmission and consequently produce analgesia. However, descending pathways projecting to the dorsal horn not only suppress (descending inhibition) but they may also potentiate (descending facilitation) nociceptive messages. In the case of the descending 5-HT system, both inhibition and facilitation have been described depending on the 5-HT receptor involved and on their divergent neuronal localization (Millan, 2002; Suzuki et al., 2004; Oyama et al., 1996).

Different drugs acting on the central 5-HT system have been investigated and/or used as analgesics. Among them, antidepressants are frequently used as adjuvants for analgesic treatment of pain. A significant amount of evidence supports the use of tricyclic antidepressants (TCAs) and other 5-HT and NE reuptake inhibitors (SNRIs) in the management of chronic pain, but because of their effects on multiple systems, they are associated with numerous undesirable side effects (Carter and Sullivan, 2002; Mattia et al., 2002; Barkin and Barkin, 2005). The newer selective 5-HT reuptake inhibitors (SSRIs), having only 5-HT-receptor-mediated side effects, have not been thoroughly studied but they seem to be less efficacious than TCAs and SNRIs in treating chronic pain (Barkin and Fawcett, 2000; Bomholt et al., 2005; Briley, 2004; Maizels and McCarberg, 2005; Sindrup et al., 2005; Stahl et al., 2005). In addition, available data suggest that increased 5-HT levels do not invariably inhibit nociceptive processing. Rather, it elicits a spectrum of pro- and antinociceptive actions that are receptor-dependent and a function of stimulus quality and modality (Millan, 2002; Suzuki et al., 2004).

As reviewed by Millan (2002), different 5-HT receptors have been implicated in analgesia. Among them, 5-HT_{1A}, 5-HT_{1B/1D}, 5-HT_{2A}, 5-HT_{2C} and 5-HT₃ have received most attention (Eide and Hole, 1993; Oyama et al., 1996; Obata et al., 2000; Kayser et al., 2002; Colpaert, 2006).

A recent Immunocytochemical study investigating 5-HT₇ receptor distribution at the lumbar level revealed that 5-HT₇ immunolabelling is localized mainly in the two superficial laminae of the dorsal horn and in small and medium-sized dorsal root ganglion cells, which is consistent with a predominant role in nociception (Doly et al., 2005). In rat and human dorsal root ganglia, the presence of 5-HT₇ receptor messenger RNA has also been detected (Pierce et al., 1996, 1997). Electron microscopic examination of the dorsal horn revealed three main localizations: 1) a postsynaptic localization on peptidergic cell bodies in laminae I-III and in numerous dendrites; 2) a presynaptic localization on unmyelinated (presumably of primary afferent origin) and thin myelinated peptidergic fibers (from intrinsic cells); and 3) a localization on astrocytes in lamina I and II (Doly et al., 2005).

Regarding its function, a possible pronociceptive role of peripheral and spinal 5-HT₇ receptors has been described in the formalin test based on local paw and intrathecal administration of 5-HT or the non-selective 5-carboxamidotryptamine (5-CT) serotonergic agonist and reversion of their effects by the 5-HT₇ receptor antagonist SB-269970 (Rocha-González, 2005).

As pain is still considered as a major health problem in broad areas of the population and needs a very specific treatment, it was the underlying problem solved by this invention to find new ways of treating pain. Especially it was it was the underlying problem solved by this invention to improve treatment with µ-opioids especially in regards to the risks of tolerance and dependency.

So, the main object of this invention is a combination of compounds comprising
a) at least one compound A selected from compounds binding to the 5-HT7 receptor;
b) at least one compound B selected from compounds binding to the µ-opoid receptor.

This/these compound/s may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable ratio.

While working on compounds binding to the 5HT7 receptor and its combination with compounds binding to the µ-opioid receptor (µ-opioids) it was surprisingly found out that the combination of these compounds either used simultaneously or consecutively could act on the treatment of pain with a high potency, especially showing a super-additive effect in a number of treatments in pain symptoms. Thus this combination was not only strongly ameliorating pain treatment, but seems also to be able to reduce the risk of the development of dependency in a patient by allowing a reduction in dose of the opioids and to reduce/deny the effect of development of tolerance - under certain circumstances.

"Treating" or "treatment" as used in this application are defined as including the treatment of the symptoms of pain, as well as treatment of the disease or disease consequences causing the symptoms, the prevention or the prophylaxis of the symptoms of pain, as well as the prevention or the prophylaxis of the disease or disease consequences causing the symptoms. Preferably "treating" or "treatment" as used in this application are defined as including the treatment of the symptoms of pain, as well as treatment of the disease consequences causing the symptoms, the prevention or the prophylaxis of the symptoms of pain, as well as the prevention or the prophylaxis of the disease consequences causing the symptoms. Most preferably "treating" or "treatment" as used in this application are defined as including the treatment of the symptoms of pain, and the prevention or the prophylaxis of the symptoms of pain.

The superfamily of serotonin receptors (5-HT) includes 7 classes (5-HT₁-5-HT₇) encompassing 14 human subclasses are described in many positions in literature especially in D. Hoyer, et al., Neuropharmacology, 1997, 36, 419. Thus:
"The 5HT7 receptor" as used in this application is well known and defined.
"The 5HT1A receptor" as used in this application is well known and defined.
All other "5HT receptors" as used in this application are well known and defined.

"Compound binding to the 5 HT7 receptor" as used in this application is/are defined as having a Kᵢ Value in their binding to the 5HT7-receptor ≤ 2 µM.

Assays that may be used for determining the affinity and selectivity of a 5-HT7 receptor agonist and/or other affinities to 5-HT receptors are well known in the art and especially measuring the affinities to these receptors are offered by service companies like CEREP (128, rue Danton, 92500 Rueil. Malmaison, France) or or MDS Pharma Services (Rosa de Lima 1bis, 2° 28290 Las Matas (Madrid), Spain). It is possible to classify a compound with 5-HT receptor affinity as agonist (also as inverse agonist or antagonist) according to the reference of S. M. Stahl, Essential Psychopharmacology, Neuroscientific basis and practical applications, Ed. Cambridge, 1996, Chapter 3. The respective part of the literature is hereby incorporated by reference and forms part of the disclosure.

"Opioids" is the common name for all compounds which have the same mode of action as the constituents of opium, the dried milky liquid of the poppy seed, Papaver somniferum (Brownstein, 1993). All opioids interact in biological systems with the same type of receptor, the so-called opioid receptor. According to the analgesia and side-effect profile four types of opioid receptors, the µ-receptor (ligand = morphine), the κ-receptor (ligand = ketazocine) and the σ-receptor (ligand = SKF 10081), as well as the later-added ORL1-receptor are known. Corresponding to other receptor systems, binding studies as well as functional investigations indicate that subtypes of opioid receptors exist (Wood, 1982; Pastemak and Wood, 1986). Within the µ- and δ-receptor type 2 subtypes, the µ-1 and µ-2 and δ-1 and δ-2 have been described. The κ-receptor contains an additional κ-3 subtype. Especially in regards to the µ-opioid receptor its two subtypes are included in tis invention.

"Compound binding to the µ-opioid receptor" as used in this application is/are defined as having a Kᵢ Value in their binding to the µ-opioid-receptor ≤ 1 µM.

Assays that may be used for determining the affinity and selectivity of a compound binding to the µ-opioid receptor especially a µ-receptor agonist are well known in the art and especially measuring the affinities to these receptors are offered by service companies like CEREP (128, rue Danton, 92500 Rueil. Malmaison, France) or or MDS Pharma Services (Rosa de Lima 1bis, 2° 28290 Las Matas (Madrid), Spain). A complete comprehensive list of compounds binding to the µ-opioid receptor together with their Ki-values for different opioid receptors are given in H.Buschmann et al, Handbook of Pain, (2002), Wiley, and is hereby incorporated by reference and forms part of the disclosure.

An "Agonist" is defined as a compound that binds to a receptor and has an intrinsic effect, and thus, increases the basal activity of a receptor when it contacts the receptor. Full agonists show the maximum effect like in this case 5CT or AS-19 on the 5-HT7 receptor, whereas a partial agonist like MSD-5a is giving less (e.g. 80%) of the response of the full agonist as a maximum. Included in this definition are also compounds acting as mixed agonists/antagonists on the receptor, thus showing agonistic and also antagonistic activity.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of a compound in the invention in which this compound has attached to it via non-covalent binding another molecule (most likely a polar solvent) especially including hydrates and alcoholates, e.g. methanolate.

An especially preferred embodiment of the invention encompasses combinations according to the invention, wherein the compound A is acting as an agonist, preferably a full or partial agonist on the 5-HT7 receptor, preferably as a full agonist.

Another especially preferred embodiment of the invention encompasses combinations according to the invention, wherein the compound B is acting as an agonist, preferably a full or a partial agonist or mixed agonist/antagonist on the µ-opioid receptor.

A highly preferred embodiment of the invention encompasses combinations according to the invention, wherein
the compound A is acting as an agonist, preferably a full or partial agonist on the 5HT7 receptor;
   and
the compound B is acting as an agonist, preferably a full or partial agonist or mixed agonist/antagonist on the µ opioid receptor.

In another preferred embodiment of the combination according to the invention, the compound B binding to the µ-opioid receptor is binding to the µ-opioid receptor with a Ki-value lower than 1000 nM, preferably lower than 200 nM, more preferably lower than 100 nM, even more preferably lower than 50 nM.

In another preferred embodiment of the combination according to the invention, the compound B binding to the µ-opioid receptor is binding to the µ-opioid receptor with a Ki-value lower than 1000 nM, preferably lower than 200 nM, more preferably lower than 100 nM, even more preferably lower than 50 nM, highly preferably lower than 10 nM.

In another preferred embodiment of the combination according to the invention, the compound B binding to the µ-opioid receptor
is acting as a full or partial agonist on the µ opioid receptor;
   and
is binding to the µ-opioid receptor with a Ki-value lower than 1000 nM, preferably lower than 200 nM, more preferably lower than 100 nM, even more preferably lower than 50 nM.

In another preferred embodiment of the combination according to the invention, the compound B binding to the µ-opioid receptor
is acting as a full or partial agonist on the µ opioid receptor;
   and
is binding to the µ-opioid receptor with a Ki-value lower than 1000 nM, preferably lower than 200 nM, more preferably lower than 100 nM, even more preferably lower than 50 nM, highly preferably lower than 10 nM.

In another preferred embodiment of the combination according to the invention, the compound B binding to the µ-opioid receptor is selected from
- natural products including semi-synthetic derivatives of natural products, like morphine, codeine and thebain;
- fully synthetic compounds;
- peptides.

In another preferred embodiment of the combination according to the invention, the compound B binding to the µ-opioid receptor is selected from
- morphine, codeine, thebain, papaverin, narcotine,
- heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fantanyl, lofentanil, alfentanil, ohmefentanil, remifentanil, pitramide, benztriamide, diphenoxylate, loperamide, tramadol, tilidine, U-50488, 1-Benzyl-4-(4-bromo-phenyl)-4-dimethylamino-cyclohexanol;
- alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
- Met-enkephalin, Leu-enkephalin, nociceptin, β-endorphin, endomorphin-1, endomorphin-2, metorphamid, dynorphin-A, dynorphin-B, α-neoendorphin.

Especially preferred embodiments of the invention encompass a combination according to the invention wherein the Compound A is a compound with a very specific binding to the 5HT7 receptor being in its binding profile more specific in its affinity (thus showing a lower Ki) to the 5HT7 receptor than in its affinity to other 5HT Receptors.

Therefore, another preferred combination according to the invention is a combination wherein the compound A binding to the 5HT7 receptor is binding with a higher affinity - expressed as a lower Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

Exemplifying the above paragraph would be a compound X, specific to the 5HT7 receptor, assumed to have an affinity - expressed as a Ki value- of 1 nM and an affinity to the 5HT1A receptor of a Ki value of 42 nM.

Another preferred combination according to the invention is a combination wherein the compound A binding to the 5HT7 receptor is binding with a higher affinity - expressed as a lower Ki-value - to the 5HT7 receptor than to the any other 5-HT receptor; especially binding with an affinity higher by a factor of at least 10, preferably with an affinity higher by a factor of at least 30, more preferably with an affinity higher by a factor of at least 50, most preferably with an affinity higher by a factor of at least 100.

Another preferred combination according to the invention is a combination wherein the compound A binding to the 5HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM.

Another preferred combination according to the invention is a combination wherein the compound A binding to the 5HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

Another preferred combination according to the invention is a combination wherein the compound A binding to the 5HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100;
and
is acting as an agonist, preferably as a full or partial agonist on the 5-HT7 receptor, more preferably as a full agonist.

Another preferred combination according to the invention is a combination wherein the compound A binding to the 5HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to any other 5HT receptor; especially binding with an affinity higher by a factor of at least 10, preferably with an affinity higher by a factor of at least 30, more preferably with an affinity higher by a factor of at least 50, most preferably with an affinity higher by a factor of at least 100.

Another preferred combination according to the invention is a combination wherein the compound A binding to the 5HT7 receptor is binding to the 5HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a lower Ki-value - to the 5HT7 receptor than to any other 5HT receptor; especially binding with an affinity higher by a factor of at least 10, preferably with an affinity higher by a factor of at least 30, more preferably with an affinity higher by a factor of at least 50, most preferably with an affinity higher by a factor of at least 100;
and
is acting as an agonist, preferably as a full or partial agonist on the 5-HT7 receptor, more preferably as a full agonist.

Another preferred combination according to the invention is a combination wherein the compound A binding to the 5HT7 receptor is either AS-19 or MSD5a, especially AS-19, optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate.

AS-19 [(2*S*)-(+)-8-(1,3,5-trimethylpyrazolin-4-yl)-2-(dimethylamino)tetralin] is defined by below sketched formula and is commercially available (e.g. through TOCRIS). 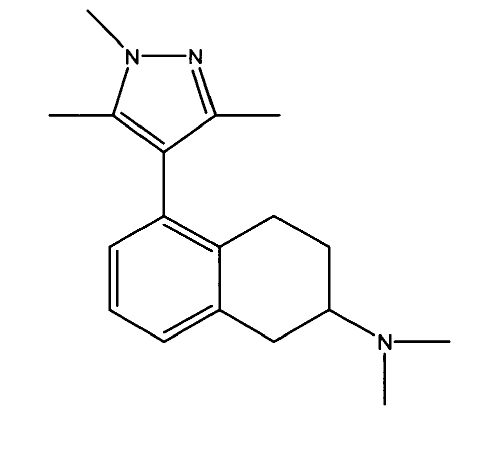 MSD-5a (corresponds to compound 5a in Thomson et al., 2004; Merck Sharp & Dohme Research Laboratories) [2-(6-phenylpyridin-2-ylthio)-*N*,*N*-dimethyl ethanamine] is described in this literature including its synthesis.

Another preferred combination according to the invention is a combination wherein the compound A binding to the 5HT7 receptor is selected from
Compounds of Chemical Group 1, Heterocyclyl-substituted-ethylamino-phenyl derivative of general formula (l/1) 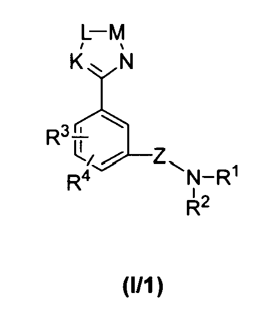 wherein
   K-L-M-N together form
   - =CH-X-Y=CH-; in which any suitable H may be substituted by R⁶ and/or R⁷, and in which X is selected from NR⁸, O or S, while Y is selected from N or CH;
   - =CH-X-Y-C(O)-; in which any suitable H may be substituted by R⁶ and in which one of X and Y is NR⁸, while the other is selected from NR^{8a}, S or O;
   - =CH-X-Y-C(O)-; in which one of X and Y is CH₂, while the other is selected from NR⁸, S or O, in which any suitable H may be substituted by R⁶ and/or R⁷;
   - =CR⁶-N=N-C(O)-;
   - =CR⁹-CH=CH-CH=CH-; in which any suitable H may be substituted by R⁶;
   - =CR⁹-CH=CH-CH=CR^{9a}-; in which any suitable H may be substituted by R⁶;
   - =CH-X=Y-CH=CH-; in which any suitable H may be substituted by R⁶ and/or R⁷, and in which one of X or Y is selected from N, while the other is selected from N or CH;
   - =CH-X=Y-CH₂-CH₂-; in which any suitable H may be substituted by R⁶ and/or R⁷, and in which one of X or Y is selected from N, while the other is selected from N or CH;
   - =CH-X-Y-CH=CH-; in which any suitable H may be substituted by R⁶ and/or R⁷, and in which one of X or Y is selected from NR⁸, O or S while the other is selected from NR^{8a} or CH₂;
   - =CH-X-Y-CH₂-CH₂-; in which any suitable H may be substituted by R⁶ and/or R⁷, and in which one of X or Y is selected from NR⁸, O or S while the other is selected from NR^{8a} or CH₂;
   - =CH-X-CH₂-Y=CH-; in which any suitable H may be substituted by R⁶ and/or R⁷, and in which X is selected from NR⁸, O or S while Y is selected from N or CH;
   - =CH-X-CH=Y-CH₂-; in which any suitable H may be substituted by R⁶ and/or R⁷, and in which X is selected from NR⁸, O or S while Y is selected from N or CH;
   - =CH-N=CH-Y=CH-; in which any suitable H may be substituted by R⁶ and/or R⁷;
   - =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;
   R¹ and R² each are independently selected from the group consisting of hydrogen; or a linear or branched, saturated or unsaturated, optionally at least monosubstituted aliphatic radical; or
   R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
   Z is selected from
   - -(CH₂)n-, with n being 1, 2, 3 or 4;
   - -0-(CH₂)n-, with n being 1, 2, 3 or 4;
   - -S-(CH₂)ₙ-, with n being 1, 2, 3 or 4;
   - (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
   R³ and R⁴ are independently from each other selected from hydrogen; halogen, OH, SH, NH₂; a linear or branched, saturated or unsaturated, optionally at least monosubstituted aliphatic radical; or O-R with R being a linear or branched, saturated or unsaturated, optionally at least mono-substituted aliphatic radical;
   R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
   R⁸ and R^{8a} are independently from each other selected from hydrogen; or an aliphatic radical, which is linear or branched, saturated or unsaturated, and optionally at least mono-substituted by F, Cl, Br, I, SH or OH;
   R⁹ and R^{9a} are independently from each other selected from an aliphatic radical, which is linear or branched, saturated or unsaturated, and optionally at least monosubstituted by F, Cl, Br, I, SH or OH; ; or O-R with R being an aliphatic radical, which is linear or branched, saturated or unsaturated, and optionally at least monosubstituted by F, Cl, Br, I, SH or OH;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
   or
Compounds of Chemical Group 2, heterocyclyl-substituted- tetrahydro-naphthalen derivative of general formula (I/2) or its benzyl-substituted analogue of general formula (Iₚᵣₒₜ/2) 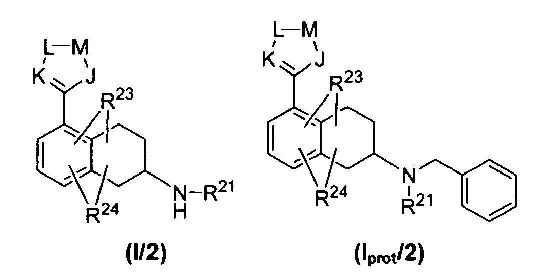 wherein
   K-L-M-J together form
   - =CH-X-Y=CH-, in which any suitable H may be substituted by R²⁶ and/or R²⁷, and in which X is selected from NR²⁸, O or S, while Y is selected from N
      or
      CH;
   - =CH-X-Y-C(O)-, in which any suitable H may be substituted by R²⁶ and in which one of X and Y is NR²⁸, while the other is selected from NR^{28a}, S or O;
   - =CH-X-Y-C(O)-, in which one of X and Y is CH₂, while the other is selected from NR²⁸, S or O, in which any suitable H may be substituted by R²⁶ and/or R²⁷;
   - =CR²⁶-N=N-C(O)-; or
   - =CR²⁹-X₁=Y-X₂=CR^{29a}-, in which two of Y, X₁ and X₂ are CH, while the other is selected from CH or N, in which any suitable H may be substituted by R²⁶;
   R²¹ is selected from the group consisting of hydrogen; a linear or branched, saturated or unsaturated, optionally at least mono-substituted aliphatic radical; or an optionally at least mono-substituted alkyl-aryl;
   R²³ and R²⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
   R²⁶ and R²⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
   R²⁸ and R^{28a} are independently from each other selected from hydrogen; or an aliphatic radical, which is linear or branched, saturated or unsaturated, and optionally at least mono-substituted by F, Cl, Br, I, SH or OH;
   R²⁹ and R^{29a} are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
   or
Compounds of Chemical Group 3, heterocyclyl-substituted-tetrahydro-naphthalen-amine derivatives of general formula (I/3) 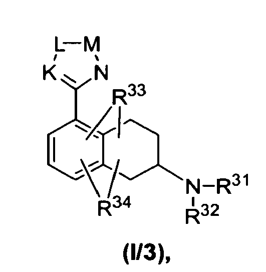 wherein
   K-L-M-J together form
   - =CH-X-Y=CH-, in which any suitable H may be substituted by R³⁶ and/or R³⁷, and in which X is selected from NR³⁸, O or S, while Y is selected from N or
      CH;
   - =CH-X-Y-C(O)-, in which any suitable H may be substituted by R³⁶ and in which one of X and Y is NR³⁸, while the other is selected from NR^{38a}, S or O;
   - =CH-X-Y-C(O)-, in which one of X and Y is CH₂, while the other is selected from NR³⁸, S or O, in which any suitable H may be substituted by R³⁶ and/or
   - =CR³⁶-N=N-C(O)-; or
   - =CR³⁹-X₁=Y-X₂=CR^{39a}-, in which two of Y, X₁ and X₂ are CH, while the other is selected from CH or N, in which any suitable H may be substituted by R³⁶;
   R³¹ and R³² are independently from each other a linear or branched, saturated or unsaturated, optionally at least mono-substituted aliphatic radical;
   or
   R³¹ and R³² together with their connecting nitrogen are forming an optionally at least mono-substituted heterocyclic ring system;
   R³³ and R³⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical; R³⁶ and R³⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Of, Br, I, SH or OH;
   R³⁸ and R^{38a} are independently from each other selected from hydrogen; or an aliphatic radical, which is linear or branched, saturated or unsaturated, and optionally at least mono-substituted by F, Cl, Br, I, SH or OH;
   R³⁹ and R^{39a} are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
   optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

A "mono- or polycyclic ring-system" according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

An "aryl", "aryl radical" or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention "cycloalkyl radical" or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C₃- or C₄-cycloalkyl, C₃₋₅-cycloalkyl represents C₃-, C₄- or C₅-cycloalkyl, C₃₋₆-cycloalkyl represents C₃-, C₄-, C₅- or C₆-cycloalkyl, C₃₋₇-cycloalkyl represents C₃-, C₄-, C₅-, C₆- or C₇-cycloalkyl, C₃₋₈-cycloalkyl represents C₃-, C₄-, C₅-, C₆-, C₇- or C₈-cycloalkyl, C₄₋₅-cycloalkyl represents C₄- or C₅-cycloalkyl, C₄₋₆-cycloalkyl represents C₄-, C₅- or C₆-cycloalkyl, C₄₋₇-cycloalkyl represents C₄-, C₅-, C₆- or C₇-cycloalkyl, C₄₋₈-cycloalkyl represents C₄-, C₅-, C₆- C₇- or C₈-cycloalkyl C₅₋₆-cycloalkyl represents C₅- or C₆-cycloalkyl and C₅₋₇-cycloalkyl represents C₅-, C₆- or C₇-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

A "heterocyclyl", a "heterocyclyl radical" or group or "heterocyclic ring system" is understood as meaning heterocyclic ring systems which contain one or more heteroatoms from the group consisting of nitrogen, oxygen and/or sulfur in the ring or ringsystem, and can also be mono- or polysubstituted. The ringsystem may consist either of only one saturated or unsaturated or even aromatic ring or may consist of 2, 3 or 4 saturated or unsaturated or even aromatic rings, which are condensed in that between two or more of the rings ring members are shared. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, imidazo-thiazole, benzothiazole, indole, benzotriazole, benzodioxolane, benzodioxane, carbazole and quinazoline.

In connection with mono- or polycyclic ring-system, aryl radical, cycloalkyl radical, or heterocyclyl radical, "substituted" is understood - unless defined otherwise - as meaning replacement of at least one hydrogen radical on the ring-system of the mono- or polycyclic ring-system, the aryl radical, the cycloalkyl radical, or the heterocyclyl radical by OH, SH, =O, halogen (F, Cl, Br, I), CN, NO₂, COOH; NRₓR_{y}, with Rₓ and R_{y} independently being either H or a saturated or unsaturated, linear or branched, substituted or unsubstituted C₁₋₆-alkyl; by a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted - S-C₁₋₆₋alkyl; a saturated or unsaturated, linear or branched, substituted or unsubstituted - C(O)-C₁₋₆₋alkyl; a saturated or unsaturated, linear or branched, substituted or unsubstituted - C(O)-O-C₁₋₆₋alkyl; a substituted or unsubstituted phenyl. Within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents. Therefore, "optionally at least monsubstituted" means either "not substituted" if the option is not fulfilled, "monosubstituted" or "polysubstituted".

In connection with aryl radical, cycloalkyl radical, or heterocyclyl radical, "condensed with" is understood as meaning that the ring-system of the aryl radical, the cycloalkyl radical, or the heterocyclyl radical is sharing two atoms (one) of its ring(s) with a ring of the mono- or polycyclic ring-system it is condensed with.

Aliphatic radicals/groups, as referred to in the present invention, are optionally mono- or polysubstituted and may be branched or linear, saturated or unsaturated. Aliphatic radicals, as defined in the present invention, include alkyl, alkenyl and alkinyl radicals. Unsaturated aliphatic radicals, as defined in the present invention, include alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, "alkyl", "alkyl radical" or group is understood as meaning saturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C≡C-CH₃, while saturated alkyl encompasses e.g. -CH₃ and -CH₂-CH₃. In these radicals, C₁₋₂-alkyl represents C₁- or C₂-alkyl, C₁₋₃-alkyl represents C₁-, C₂- or C₃-alkyl, C₁₋₄-alky) represents C₁-, C₂-, C₃- or C₄-alkyl, C₁₋₅-alkyl represents C₁-, C₂-, C₃-, C₄-, or C₅-alkyl, C₁₋₆-alkyl represents C₁-, C₂-, C₃-, C₄-, C₅- or C₆-alkyl, C₁₋₇-alkyl represents C₁-, C₂-, C₃-, C₄-, C₅-, C₆- or C₇-alkyl, C₁₋₈-alkyl represents C₁-, C₂-, C₃-, C₄-, C₅-, C₆-, C₇- or C₈-alkyl, C₁₋₁₀-alkyl represents C₁-, C₂-, C₃-, C₄-, C₅-, C₆-, C₇-, C₈-, C₉- or C₁₀-alkyl and C₁₋₁₈-alkyl represents C₁-, C₂-, C₃-, C₄-, C₅-, C₆-, C₇-, C₈-, C₉-, C₁₀-, C₁₁-, C₁₂-, C₁₃-, C₁₄-, C₁₅-, C₁₆-, C₁₇- or C₁₈-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkylene, alkyl or aliphatic radical or group - unless defined otherwise - the term "substituted" in the context of this invention is understood as meaning replacement of at least one hydrogen radical by F, Cl, Br, 1, NH₂, SH or OH; within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

The term "alkylene" is understood as meaning a divalent alkyl group like -CH₂- or -CH₂-CH₂-, with (CH₂)₃₋₆ being understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning - CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc. An "alkylene" may also be unsaturated.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic- especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid. The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

Any combination of compounds according to the invention also includes combinations with a compound which is a prodrug of this compound and thus is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

Unless otherwise stated, the compounds used in the combination according to the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Other possibilities for the compound A binding to the 5HT7 receptor are known from the art, might include compounds according to WO2006/069776 A1, W02006/069775 A1, WO2006/018309 A1 and WO 2006/018308 A1, included here by reference, or is selected from substituted tetrahydroisoquinoline according to general formula I/4: 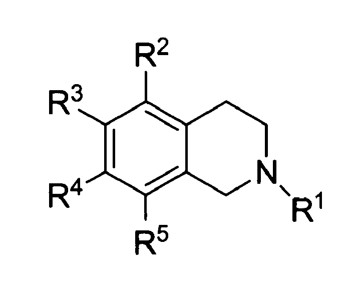 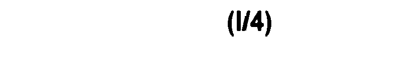 wherein
- R¹: represents a hydrogen atom; a -C(=O)-OR³⁷ moiety;
a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
or a saturated or unsaturated 3- to 9-membered cycloaliphatic radical, which may contain 1, 2 or 3 heteroatom(s) independently selected from the group consisting of nitrogen, oxygen and sulfur as ring member(s) and which may be unsubstituted or optionally substituted with 1, 2, 3, 4 or 5 substituent(s) independently selected from the group consisting of oxo (=O), thioxo (=S), C₁-₅-alkyl, -O-C₁₋₅-alkyl, -S-C₁₋₅-alkyl, -C(=OYC₁₋₅-alkyl, -O-C(=O)-C₁₋₅-alkyl, F, Cl, Br, I, -CN, -CF₃, -OCF₃, -SCF₃, -OH, -SH, -NH₂, -NH(C₁₋₅-alkyl), -N(C₁₋₅-alkyl)₂, -NO₂, -CHO, -CF₂H, -CFH₂, -C(=OYNH₂, -C(=O)-NH(C₁₋₅-alkyl), -C(=O)-N(C₁₋₅-alkyl)₂, -S(=O)₂-C₁₋₅-alkyl, -S(=O)₂-phenyl, phenyl, phenoxy and benzyl and which may be condensed with an unsubstituted or at least mono-substituted saturated, unsaturated or aromatic mono- or bicyclic ring system and which may be bonded via a linear or branched C₁₋₆ alkylene, C₂₋₆ alkenylene or C₂₋₆ alkinylene group which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
- R², R³, R⁴ and R⁵,: independently of one another, each represent a hydrogen atom; F, Cl, Br, I, -NO₂ -NH₂; -SH; -OH; -CN; -C(=O)-OH; -C(=O)-H; -S(=O)₂-OH; -C(=O)-NH₂; -S(=O)₂-NH₂; -C(=O)-R⁶; -S(=O)-R⁷; -S(=O)₂-R⁷; -OR⁸; -SR⁹; -C(=O)-OR¹⁰; -N(R¹¹)-S(=O)₂-R¹²; -NR¹³R¹⁴; -NH-R¹⁵ ; -C(=O)-NR¹⁶R¹⁷; C(=O)-NHR¹⁸;
a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
with the proviso that at least one of the substituents R², R³, R⁴ and R⁵ represents a-N(R¹¹)-S(=O)₂-R¹² moiety;
- R⁶ R⁷, R⁸, R⁹, R¹⁰, R¹³, R¹⁴, R¹⁵, R¹⁶, R¹⁷ and R¹⁸,: independently of one another, each represent a linear or branched, saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
- R¹¹: represents a hydrogen atom, -S(=O)₂-R¹² or a linear or branched, saturated or unsaturated, unsubstituted or at least mono-substituted aliphatic radical;
- R¹²: represents a phenyl radical of general formula (A), 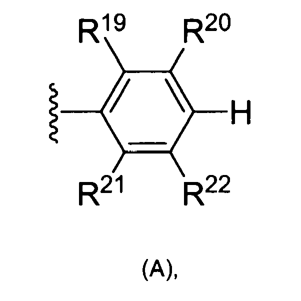 wherein
R¹⁹, R²⁰, R²¹ and R²². independently of one another, each represent a hydrogen atom; F, Cl, Br, I, -NO₂; -NH₂; -SH; -OH; -CN; -C(=O)-OH;-C(=O)-H; -S(=O)₂-OH; -C(=O)-NH₂; -S(=O)₂-NH₂; -C(=O)-R²³; -S(=O)-R²⁴; -S(=O)₂-R²⁴; -OR²⁵; -SR²⁶; -C(=O)-OR²⁷; -N(R²⁸)-S(=O)₂-R²⁹; -NH-S(=O)₂-R³⁰; -NR³¹R³²; -NH-R³³; -C(=O)-NHR³⁴; -C(=O)NR³⁵R³⁶; a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; or a saturated or unsaturated, unsubstituted or at least mono-substituted, optionally at least one heteroatom as a ring member containing cycloaliphatic radical;
with the proviso that at least one of the substituents R¹⁹, R²⁰, R²¹ and R²² is unlike hydrogen;
an unsubstituted or at least mono-substituted 10-membered aryl radical;
a monocyclic heteroaryl radical, which may be unsubstituted or optionally substituted with 1, 2, 3, 4 or 5 substituent(s) independently selected from the group consisting of F, Cl, Br, I, -NO₂; -NH₂; -SH; -OH; -CN; -C(=O)-OH;-C(=O)-H; -S(=O)₂-OH; -C(=O)-NH₂; -S(=O)₂-NH₂; -C(=O)-R²³; -S(=O)-R²⁴;-S(=O)₂-R²⁴; -OR²⁵; -SR²⁶; -C(=O)-OR²⁷; -N(R²⁸)-S(=O)₂-R²⁹; -NH-S(=O)₂-R³⁰;-NR³¹R³²; -NH-R³³; -C(=O)-NHR³⁴; -C(=O)-NR³⁵R³⁶; a linear or branched, saturated or unsaturated C₁₋₁₀ aliphatic radical which may be unsubstituted or substituted with 1, 2 or 3 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂; and a saturated or unsaturated, unsubstituted or at least mono-substituted, optionally at least one heteroatom as a ring member containing cycloaliphatic radical;
an unsubstituted or at least mono-substituted monocyclic heteroaryl radical, which is condensed with an unsubstituted or at least mono-substituted saturated or unsaturated, but not aromatic, mono- or bicyclic ring system; an unsubstituted or at least mono-substituted bi- or tricyclic heteroaryl radical, which may be condensed with an unsubstituted or at least mono-substituted saturated or unsaturated, but not aromatic, mono- or bicyclic ring system;
or a saturated or unsaturated, unsubstituted or at least mono-substituted, optionally at least one heteroatom as a ring member containing cycloaliphatic radical, which may be condensed with an unsubstituted or at least mono-substituted saturated, unsaturated or aromatic mono- or bicyclic ring system;
- R²³, R²⁷, R²⁸, R²⁹ and R³⁰,: independently of one another, each represent a linear or branched, saturated or unsaturated C₁₋₁₀aliphatic radical which may be unsubstituted or optionally substituted with 1, 2, 3, 4 or 5 substituent(s) independently selected from the group consisting of F, Cl, Br, I, -CN, -CF₃,-OCF₃, -SCF₃, -OH, -SH, -NH₂, -O-C₁₋₅-alkyl, -S-C₁₋₅-alkyl, -NH(C₁₋₅-alkyl) and-N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
- R²⁴, R²⁶, R³¹, R³² and R³³,: each represent a linear or branched, saturated or unsaturated C₁₋₁₀ aliphatic radical which may be unsubstituted or substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be condensed with an unsubstituted or at least mono-substituted saturated or unsaturated, but not aromatic, mono- or bicyclic ring system;
- R²⁵, R³⁴, R³⁵ and R³⁶,: represents a linear or branched, saturated or unsaturated C₁₋₁₀ aliphatic radical which may be unsubstituted or optionally substituted with 1, 2, 3, 4 or 5 substituent(s) independently selected from the group consisting of F, Cl, Br, I, -CN, -CF₃, -OCF₃, -SCF₃, -OH, -SH, -NH₂, -O-C₁₋₅-alkyl, -S-C₁₋₅-alkyl, -NH(C₁₋₅-alkyl) and -N(C₁₋₅-alkyl)₂;
- and R³⁷: represents a linear or branched, saturated or unsaturated C₁₋₁₀ aliphatic radical,
a saturated or unsaturated, unsubstituted or at least mono-substituted, optionally at least one heteroatom as a ring member containing cycloaliphatic radical, which may be bonded via a linear or branched alkylene, alkenylene or alkinylene group and which may be condensed with an unsubstituted or at least mono-substituted saturated, unsaturated or aromatic mono- or bicyclic ring system;
or an unsubstituted or at least mono-substituted aryl or heteroaryl radical, which may be bonded via a linear or branched alkylene, alkenylene or alkinylene group and which may be condensed with an unsubstituted or at least mono-substituted saturated or unsaturated, but not aromatic, mono- or bicyclic ring system;
option ally in form of one of its stereoisomers, preferably enantiomers or diasteromers, a racemate or in form of a mixture of at least two of its stereoisomers, preferably enantiomers and/or diastereomers, in any mixing ratio, or a salt thereof, or a corresponding solvate thereof;
and optionally at least one physiologically acceptable auxiliary agent.

Another preferred combination according to the invention is a combination wherein the compound A binding to the 5HT7 receptor is selected from
compounds of Chemical Group 1, preferably from compounds of Chemical Group 1 according to formula la/1 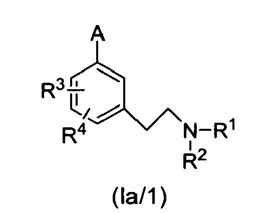 , wherein
   A is a compound selected from the following group 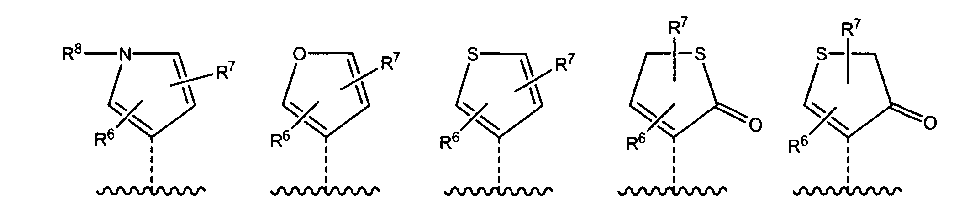 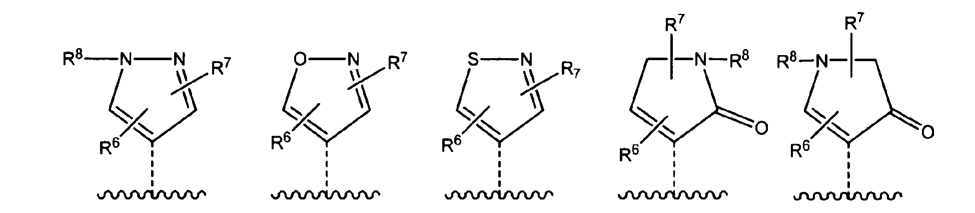 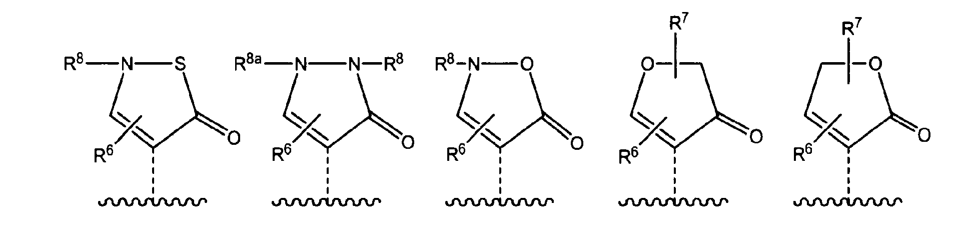 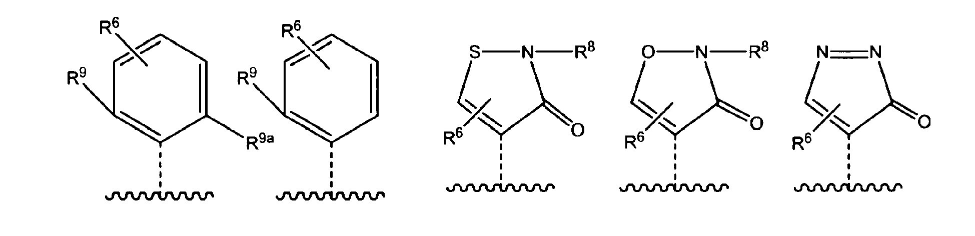 R¹ and R² each are independently selected from the group consisting of hydrogen; or a linear or branched, saturated or unsaturated, optionally at least mono-substituted aliphatic radical; or
   R¹ and R² together with the bridging nitrogen atom form an saturated or unsaturated. optionally at least mono-substituted 5- or 6-membered-heterocyclic ring, which may be condensed with an optionally at least mono-substituted mono- or polycyclic ringsystem,
   R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
   R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
   R⁸ and R^{8a} are independently from each other selected from hydrogen; or an aliphatic radical, which is linear or branched, saturated or unsaturated, and optionally at least mono-substituted by F, Cl, Br, I, SH or OH;
   R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
   most preferably from compounds of Chemical Group 1, selected from
   - Dimethyl-{2-[3-(1,3,5-tdmethyl-1H-pyrazol-4-yl)-phenyl]-ethyl)-amine,
   - Methyl-{2-[3-(1,3,5-trimethyl-1H-pyrazol-4-yl)-phenyl]-ethy)}-amine,
   - Diethyl-{2-[3-(1,3,5-trimethyl-1H-pyrazol-4-yl)-phenyl]-ethyl}-amine,
   - Dipropyl-{2-[3-(1,3,5-trimethyl-1H-pyrazol-4-yl)-phenyl]-ethyl}-amine,
   - {2-[3-(3,5-Dimethyl-1H-pyrazol-4-yl)-phenyl]-ethyl}-dimethyl-amine,
   - {2-[3-(3,5-Dimethyl-1H-pyrazol-4-yl)-phenyl]-ethyl}-methyl-amine,
   - {2-[3-(3,5-Dimethyl-1H-pyrazol-4-yl)-phenyl]-ethyl}-diethyl-amine,
   - {2-[3-(3,5-Dimethyl-1H-pyrazol-4-yl)-phenyl]-ethyl}-dipropyl-amine,
   - Dimethyl-{2-[3-(1-methyl-1H-pyrazol-4-yl)-phenyl]-ethyl}-amine,
   - Methyl-{2-[3-(1-methyl-1H-pyrazol-4-yl)-phenyl]-ethyl}-amine,
   - Diethyl-{2-[3-(1-methyl-1H-pyrazol-4-yl)-phenyl]-ethyl}-amine,
   - {2-[3-(1-Methyl-1H-pyrazol-4-yl)-phenyl]-ethyl}-dipropyl-amine.
   - {2-[3-(3,5-Dimethyl-isoxazol-4-yl)-phenyl]-ethyl}-dimethyl-amine,
   - {2-[3-(3,5-Dimethyl-isoxazol-4-yl)-phenyl]-ethyl}-methyl-amine,
   - {2-[3-(3,S-Dimethyl-isoxazol-4-yl)-phenyl]-ethyl}-diethyl-amine, or
   - {2-[3-(3,5-Dimethyl-isoxazo1-4-yl)-phenyl]-ethyl}-dipropyl-amine,
   optionally in form of a salt, preferably a physiologically acceptable salt, more preferably in form of a physiologically acceptable acid addition salt, most preferably a hydrochloride salt, or a corresponding solvate.

Another preferred combination according to the invention is a combination wherein the compound A binding to the 5HT7 receptor is selected from
compounds of Chemical Group 2, preferably from compounds of Chemical Group 2 according to formula la/2 or laₚᵣₒₜ/2 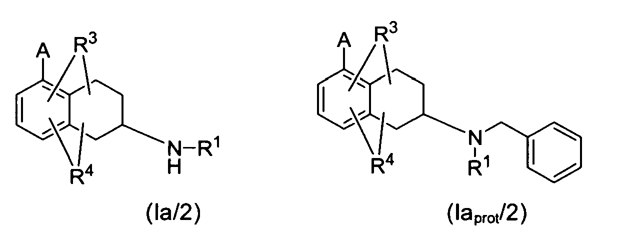 , wherein
   A is a compound selected from the following group 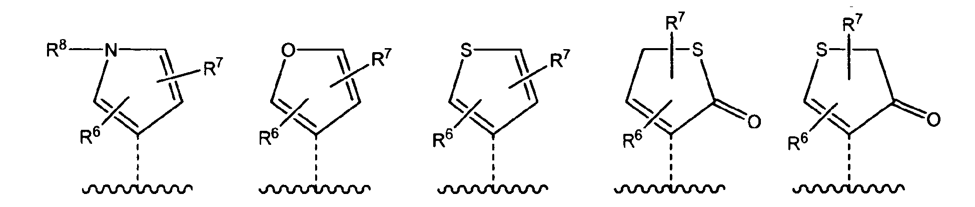 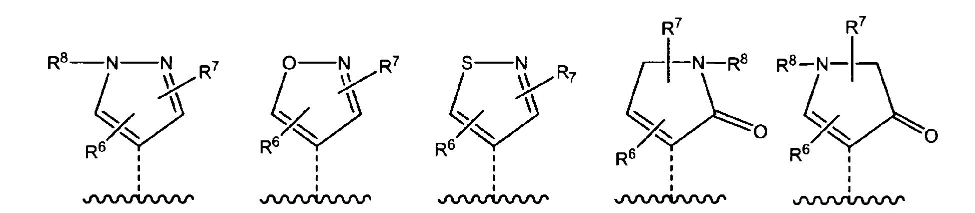 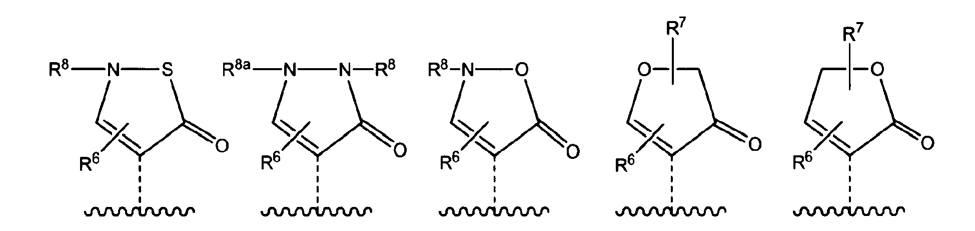 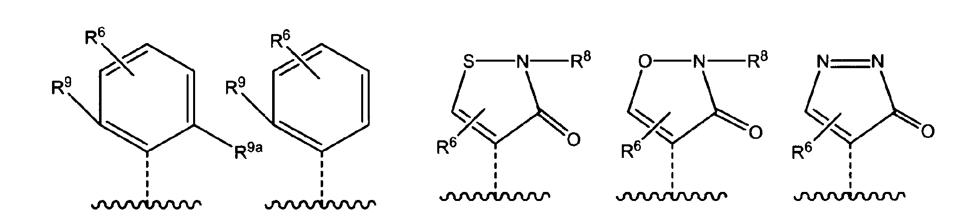 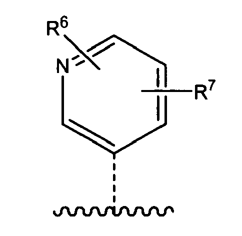 R²¹ is selected from the group consisting of hydrogen; a linear or branched, saturated or unsaturated, optionally at least mono-substituted aliphatic radical; or an optionally at least mono-substituted alkyl-aryl;
   R²³ and R²⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
   R²⁶ and R²⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
   R²⁸ and R^{28a} are independently from each other selected from hydrogen; or an aliphatic radical, which is linear or branched, saturated or unsaturated, and optionally at least mono-substituted by F, Cl, Br, I, SH or OH;
   R²⁹ and R^{29a} are independently from each other selected from halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
   most preferably from compounds of Chemical Group 2 selected from
   • Methyl-[5-(1,3,5-trimethyl-1 H-pyrazo)-4-yl)-1,2,3,4-tetrahydro-naphthalen-2-yl]-amine,
   • (2S)-Methyl-[5-(1,3,5-trimethyl-1H-pyrazol-4-yl)-1,2,3,4-tetrahydro-naphthalen-2-yl]-amine,
   • Benzyl-[5-(1,3,5-trimethyl-1 H-pyrazol-4-yl)-1,2,3,4-tetrahydro-naphthalen-2-yl]-amine,
   • (2*S*)-Benzyl-[5-(1,3,5-trimethyl-1H-pyrazol-4-yl)-1,2,3,4-tetrahydro-naphthalen-2-yl]-amine,
   • Benzyl-methyl-[5-(1,3,5-trimethyl-1 H-pyrazol-4-yl)-1,2,3,4-tetrahydro-naphthalen-2-yl]-amine,
   • (2*S*)-Benzyl-methyl-[5-(1,3,5-trimethy)-1H-pyrazol-4-yl)-1,2,3,4-tetrahydro-naphthalen-2-yl]-amine;
   • 5-(1,3,5-Trimethyl-1H-pyrazol-4-yl)-1,2,3,4-tetrahydro-naphthalen-2-ylamine hydrochloride;
      or
   • (2S)-5-(1,3.5-Trimethy)-1H-pyrazol-4-yl)-1,2.3,4-tetrahydro-naphthalen-2-ylamine hydrochloride;
   optionally in form of a salt, preferably a physiologically acceptable salt, more preferably in form of a physiologically acceptable acid addition salt, most preferably a hydrochloride salt, or a corresponding solvate.

Another preferred combination according to the invention is a combination wherein the compound A binding to the 5HT7 receptor is selected from
compounds of Chemical Group 3, preferably from compounds of Chemical Group 3 according to formula la/3 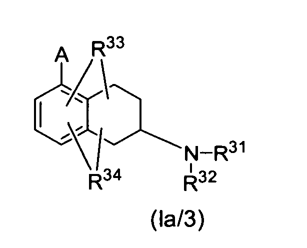 , wherein
   A is a compound selected from the following group 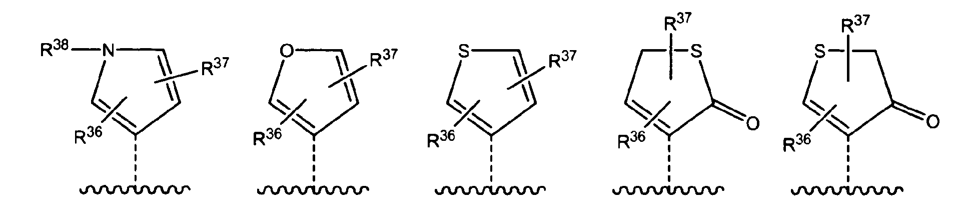 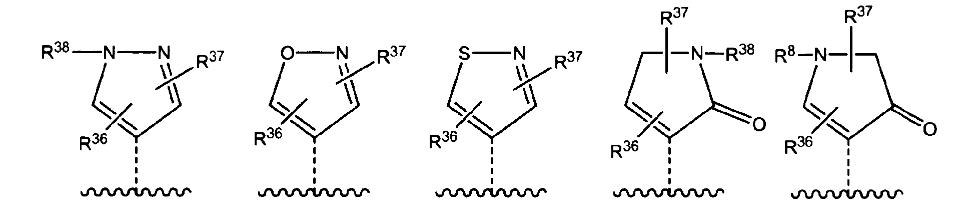 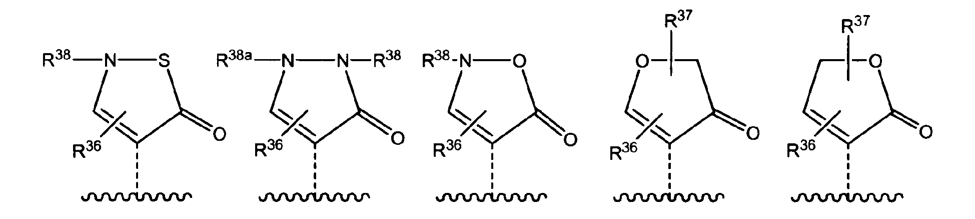 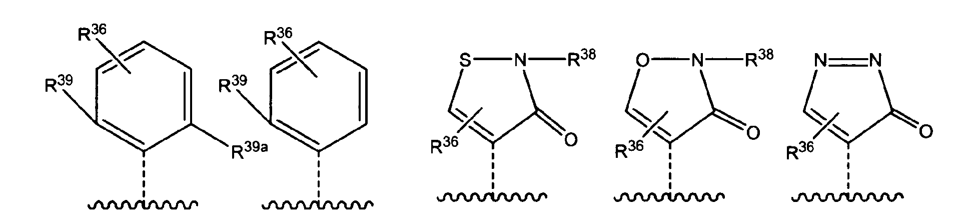 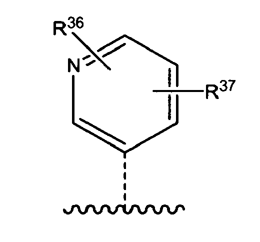 R³¹ and R³² are independently from each other a linear or branched, saturated or unsaturated, optionally at least mono-substituted aliphatic radical;
   or
   R³¹ and R³² together with their connecting nitrogen are forming an optionally at least mono-substituted heterocyclic ring system;
   R³³ and R³⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
   R³⁶ and R³⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
   R³⁸ and R^{38a} are independently from each other selected from hydrogen; or an aliphatic radical, which is linear or branched, saturated or unsaturated, and optionally at least mono-substituted by F, Cl, Br, I, SH or OH;
   R³⁹ and R^{39a} are independently from each other selected from halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
   most preferably from compounds of Chemical Group 3 selected from
   - 1-[5-(1,3,5-Trimethy)-1H-pyrazo)-4-yl)-1,2,3,4-tetrahydro-naphthalen-2-yl]-piperazine;
   - 1-Methyl-4-[5-(1,3,5-trimethyl-1H-pyrazol-4-yl)-1,2,3,4-tetrahydro-naphthalen-2-yl]-piperazine;
   - 1,3,5-Trimethyl-4-(6-pyrrolidin-1-yl-5,6,7,8-tetrahydro-naphthalen-1-yl)-1H-pyrazole;
   - 1-[5-(1,3,5-Trimethyl-1H-pyrazol-4-yl)-1,2,3,4-tetrahydro-naphthalen-2-yl]-piperidine;
   - Dipropyl-[5-(1,3,5-trimethyl-1 H-pyrazol-4-yl)-1,2,3,4-tetrahydro-naphthalen-2-yl]-amine;
   - Methyl-propyl-[5-(1,3,5-trimethyl-1 H-pyrazol-4-yl)-1,2,3,4-tetrahydro-naphthalen-2-yl]-amine;
   - Diethyl-[5-(1,3,5-trimethyl-1 H-pyrazol-4-yl)-1,2,3,4-tetrahydro-naphthalen-2-yl]-amine;
   - Ethyl-methyl-[5-(1,3,5-trimethyl-1 H-pyrazol-4-yl)-1,2,3,4-tetrahydro-naphthalen-2-yl]-amine;
   - Dimethyl-[5-(1-methyl-1H-pyrazol-4-yl)-1,2,3,4-tetrahydro-naphthalen-2-yl]-amine;
   - [5-(3,5-Dimethyl-isoxazol-4-yl)-1,2,3,4-tetrahydro-naphthalen-2-yl]-dimethyl-amine;
   - (5-Furan-3-yl-1,2,3,4-tetrahydro-naphthalen-2-yl)-dimethyl-amine;
   - Dimethyl-(5-thiophen-3-yl-1,2,3,4-tetrahydro-naphthalen-2-yl)-amine;
   - [5-(2,6-Dimethyl)-phenyl)-1,2,3,4-tetrahydro-naphtha)en-2-yl]-dimethyl-amine;
   - [5-(2,6-Difluoro-phenyl}-1,2,3,4-tetrahydro-naphthalen-2-yl]-dimethyl-amine;
   - Dimethyl-(5-pyridin-3-yl-1,2,3,4-tetrahydro-naphthalen-2-yl)-amine;
   optionally in form of one of its stereoisomers, preferably enantiomers or diastereomers, its racemate or in form of a mixture of at least two of its stereoisomers, preferably enantiomers or diastereomers, in any mixing ratio, optionally in form of a salt, preferably a physiologically acceptable salt, more preferably in form of a physiologically acceptable acid addition salt, most preferably a hydrochloride salt, or a corresponding solvate.

The combination according to the invention is toxicologically unproblematic. Thus a further aspect of the invention refers to a medicament comprising a combination according to the invention, and optionally one or more pharmaceutically acceptable adjuvants.

In human therapeutics, the dose administered can be quite low depending on the route of administration. Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.1 to 1000 mg/kg/day.

Any medicament according to the invention contains the active ingredient as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration can include intramuscular injection, intraveneous injection, subcutaneous injection, sublingual, bucal, patch through skin, oral ingestion, implantable osmotic pump, collagen implants, aerosols or suppository.

Another aspect of the invention is a use of at least one combination according to the invention for the manufacture of a medicament for the treatment of pain, preferably visceral pain, chronic pain, cancer pain, migraine, acute pain or neuropathic pain, allodynia or hyperalgesia.

In another embodiment of the invention the medicament is used for the treatment of pain in which the stimulus evoking the pain is thermal.

In another embodiment of the invention the medicament is used for the treatment of neuropathic pain - or its subgroups - in which the stimulus evoking the neuropathic pain is thermal.

In another embodiment of the invention the medicament is used for the treatment of pain in which the stimulus evoking the pain is mechanical.

In another embodiment of the invention the medicament is used for the treatment of neuropathic pain - or its subgroups - in which the stimulus evoking the neuropathic pain is mechanical.

Some known subgroups of pain are acute pain, chronic pain, visceral pain and including also headaches, especially migraine. Other subgroups of pain are neuropathic pain, hyperalgesia and allodynia.

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system". One of the many reasons causing neuropathic pain is diabetes, especially diabetes II.

According to the IASP "allodynia" is defined as "a pain due to a stimulus which does not normally provoke pain" (IASP, Classification of chronic pain, 2nd Edition, IASP Press (2002), 210).

According to the IASP "hyperalgesia" is defined as "an increased response to a stimulus which is normally painful(IASP, Classification of chronic pain, 2nd Edition, IASP Press (2002), 211).

In another preferred embodiment of the use according to the invention the neuropathic pain, is selected from central pain, hyperpathia, peripheral neuropathic pain or peripheral neurogenic pain, causalgia, hyperesthesia, neuralgia, neuritis, or neuropathy.

According to the IASP "central pain" is defined as "a pain initiated or caused by a primary lesion or dysfunction in the central nervous system" (IASP, Classification of chronic pain, 2nd Edition, IASP Press (2002), 211).

According to the IASP "causalgia" is defined as "a syndrome of sustained burning pain, allodynia and hyperpathia after a traumatic nerve lesion, often combined with vasomotor and sudomotor dysfunction and later trophic changes" (IASP, Classification of chronic pain, 2nd Edition, IASP Press (2002), 210).

According to the IASP "hyperesthesia" is defined as "increased sensitivity to stimulation, excluding the senses" (IASP, Classification of chronic pain, 2nd Edition, IASP Press (2002), 211).

According to the IASP "neuralgia" is defined as "Pain in the distribution of a nerve or nerves" (IASP, Classification of chronic pain, 2nd Edition, IASP Press (2002), 212).

According to the IASP "neuritis" is defined as "Inflammation of a nerve or nerves" (IASP, Classification of chronic pain, 2nd Edition, IASP Press (2002), 212).

According to the IASP "neuropathy" is defined as "a disturbance of function or pathological change in a nerve: in one nerve mononeuropathy, in several nerves mononeuropthy multiplex, if diffuse and bilateral, polyneuropathy" (IASP, Classification of chronic pain, 2nd Edition, IASP Press (2002), 212).

According to the IASP "hyperpathia" is defined as "a painful syndrome characterized by an abnormally painful reaction to a stimulus, especially a repetitive stimulus, as well as an increased threshold" (IASP, Classification of chronic pain, 2nd Edition, IASP Press (2002), 212).

The IASP draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

Another aspect of the ionvention refers to the use of at least one combination according to the invention for the manufacture of a medicament for the treatment of sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

Included in this invention as a very preferred aspect is especially also a method of treatment of a patient or a mammal, including men, suffering from pain by applying to the patient or mammal either a suitable amount of the combination according to the invention comprising at least one Compound A and at least one Compound B or by applying to the patient or mammal first a suitable amount of Compound A followed by applying to the patient or mammal a suitable amount of Compound B or by applying to the patient or mammal first a suitable amount of Compound B followed by applying to the patient or mammal a suitable amount of Compound A.

### Pharmacological methods:

### Method 1: Radioligand binding 5HT7

Radioligand binding assays were performed using the Cloned Human Serotonin Receptor, Subtype 7 (h5HT₇), expressed in CHO cells, coated on Flashplate (Basic FlashPlate Cat.: SMP200) from PerkinElmer (Cat.: 6120512). The protocol assay was essentially the recommended protocol in the Technical Data Sheet by PerkinEmer Life and Analytical Sciences. The Mass membrane protein/well was typically 12 µg and the Receptor/well was about 9-10 fmoles. The Flashplate were let equilibrate at room temperature for one hour before the addition of the components of the assay mixture. The binding buffer was: 50 mM Tris-HCl, pH 7.4, containing 10 mM MgCl₂, 0.5 mM EDTA and 0.5% BSA. The radioligand was [²⁵I]LSD at a final concentration of 0.82 nM. Nonspecific binding was determined with 50 µM of Clozapine. The assay volume was 25 µl. TopSeal-A were applied onto Flashplate microplates and they were incubated at room temperature for 240 minutes in darkness. The radioactivity were quantified by liquid scintillation spectrophotometry (Wallac 1450 Microbeta Trilux) with a count delay of 4 minutes prior to counting and a counting time of 30 seconds per well. Competition binding data were analyzed by using the LIGAND program (Munson and Rodbard, LIGAND: A versatile, computerized approach for characterization of ligand-binding systems. Anal. Biochem. 107: 220-239, 1980) and assays were performed in triplicate determinations for each point.

Functionality assay on the 5HT7 receptor were done according to those known in the state of the art. Classification of a compound with 5-HT receptor affinity as agonist (also as inverse agonist or antagonist) can be done according to the reference of S. M. Stahl, Essential Psychopharmacology, Neuroscientific basis and practical applications, Ed. Cambridge, 1996, Chapter 3.

### Method 2: Hot-Plate Test

### MATERIAL AND METHODS

### Animals

Male CD1 mice aged from 6 to 8 weeks old were used in these studies. Animals were housed in groups of five, provided with food and water ad libitum and kept in controlled laboratory conditions with the temperature maintained at 21 ± 1 °C and light in 12 h cycles (on at 07:00 h and off at 19:00 h). Experiments were carried out in a soundproof and air-regulated experimental room. The number of mice ranged from 8 to 16 per group in each individual experiment.

### Drugs

Drugs used for treatments were: Morphine; AS-19 [(2*S*)-(+)-8-(1,3,5-trimethylpyrazolin-4-yl)-2-(dimethylamino) tetralin]. Compound 1 [Dimethyl-{2-[3-(1,3,5-trimethyl-1H-pyrazol-4-yl)-phenyl]-ethyl}-amine]. AS-19 as well as Compound 1 were dissolved in 1% DMSO and the rest of compounds were prepared as aqueous solutions. Doses were calculated based on the molecular weight of the free base. All the compounds and vehicles (1% DMSO or physiological saline) were administered in a volume of 5 ml/kg through the subcutaneous (s.c.) route. When two compounds were co-administered, they were injected s.c. in opposite flanks of the mice.

### EXPERIMENTAL PROCEDURE:

The analgesic activity of the inventive active substance combination is determined in male CDR1 following and adapting the description in the publication of G. Woolfe and A. D. MacDonald, J. Pharm. Exp. Ther. 1944, 80, pages 300-307. The respective description of this publication is incorporated by reference and forms part of the present disclosure.

In accordance to this test the mice were put onto a plate, which was heated to 50 °C and the time was determined until the mice showed signs of pain such as vigorous and repeated licking of the hind or front paws, jumping or pulling back the paws. The mice were kept on the hot plate for no longer than 40 seconds in order to avoid the development of cutaneous lesions. The vehicle, the active substances and the inventive active substance combination to be tested were administered - sometimes in differing concentrations - by s.c. injection to different groups of mice. 0.5 hours after the administration the animals are put onto the hot plate and the time is measured until they show signs of pain, divided by Front Paw Licking (FPL), Hind Paw Licking (HPL) and Jumping, to allow conclusions on differentiations of pain types through the different pain symptoms/signs of pain. The analgesic efficacy of the active substances or active substance combination is calculated on the basis of the values obtained for the comparison group of mice to which is only administered the vehicle (baseline). By comparing to baseline (defined as 0% analgesia) and to a failure of pain signs (defined as 100% analgesia) Percentage of analgesia was determined. In some cases based on this calculation and with different concentrations of the active substances or active substance combination ED50 values were determined.

### Method 3: Tail Flick Test

The test was performed as previously described (Moncada et al. (2003), Eur. J. Pharmacol. 465, 53-60). Briefly, the animals (male CD1 mice) were restrained in a Plexiglas tube and placed on the tail flick apparatus (LI 7100, Letica, S.A., Spain). A noxious beam of light was focussed on the tail about 4 cm from the tip, and the tail flick latency was recorded automatically to the nearest 0.1 s. The intensity of the radiant heat source was adjusted to yield baseline latencies between 3 and 5 s; this intensity was never changed and any animal whose baseline latency was outside the pre-established limits was excluded from the experiments. In order to minimise injury in the animals, a cut-off time of 10 s was used. The animals received an s.c. injection of the vehicle (saline), drug or drug combination under study, and tail flick latencies were measured and are measured at 45 min after drug or vehicle administration. Baseline tail flick latencies were recorded after saline/vehicle injection. By comparing to baseline (defined as 0% analgesia) and to a failure of pain reaction/tail flick (defined as 100% analgesia) Percentage of analgesia was determined.

The examples and figures in the following section describing pharmacological trials are merely illustrative and the invention cannot be considered in any way as being restricted to these applications.

### Figures:

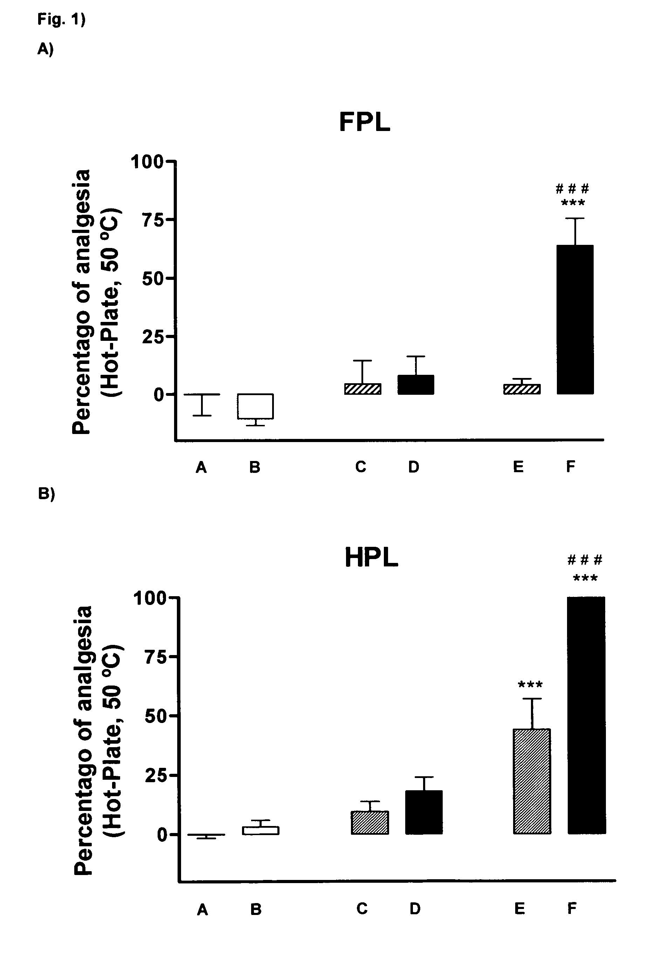

**Figure 1A****) Hot Plate Test: Morphine in combination with AS-19, FPL**
   Figure 1A) refers to P-Example 2), a Hot-Plate-Test at 50°C. Male CD1 Mice were receiving s.c. injections of test substances/combinations 30 min before measurement.
   The test results refer to:
   A = saline (vehicle/baseline)
   B = AS-19, [10 mg/kg]
   C = morphine [2 mg/kg]
   D = morphine [2 mg/kg] + AS-19 [10 mg/kg]
   E = morphine [8 mg/kg]
   F = morphine [8 mg/kg] + AS-19 [10 mg/kg]
   The sign of pain shown here is Front Paw Licking (FPL). The Baseline was determined based on saline injection. Significance values were:
   * *vs.* saline group
   # *vs.* respective morphine group
   The Combination according to the invention did show a highly super-additive effect.
**Figure 1B****) Hot Plate Test: Morphine in combination with AS-19, HPL**
   Figure 1 B) refers to P-Example 2), a Hot-Plate-Test at 50°C. Male CD1 Mice were receiving s.c. injections of test substances/combinations 30 min before measurement. The test results refer to:
   A = saline (vehicle/baseline)
   B = AS-19, [10 mg/kg]
   C = morphine [2 mg/kg]
   D = morphine [2 mg/kg] + AS-19 [10 mg/kg]
   E = morphine [8 mg/kg]
   F = morphine [8 mg/kg] + AS-19 [10 mg/kg]
   The sign of pain shown here is Hind Paw Licking (HPL). The Baseline was determined based on saline injection. Significance values were:
   * *vs.* saline group
   # *vs.* respective morphine group
   The Combination according to the invention did show a highly super-additive effect.

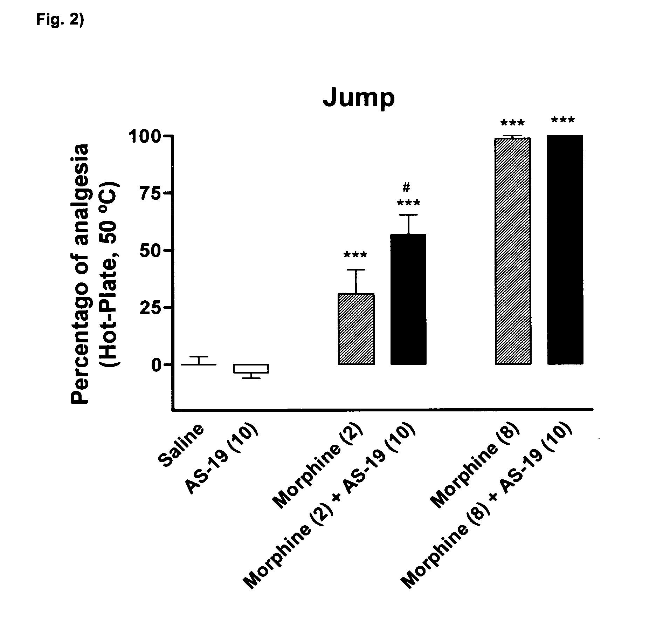

**Figure 2****) Hot Plate Test: Morphine in combination with AS-19, Jumping**
   Figure 2) refers to P-Example 2), a Hot-Plate-Test at 50°C. Male CD1 Mice were receiving s.c. injections of test substances/combinations 30 min before measurement. The sign of pain shown here is Jumping (Jump). The Baseline was determined based on saline injection. Significance values were:
   * *vs.* saline group
   # *vs.* respective morphine group
   The Combination according to the invention did show a highly super-additive effect.
**Figure 3****) Tail-Flick-Test: Morphine in combination with AS-19**

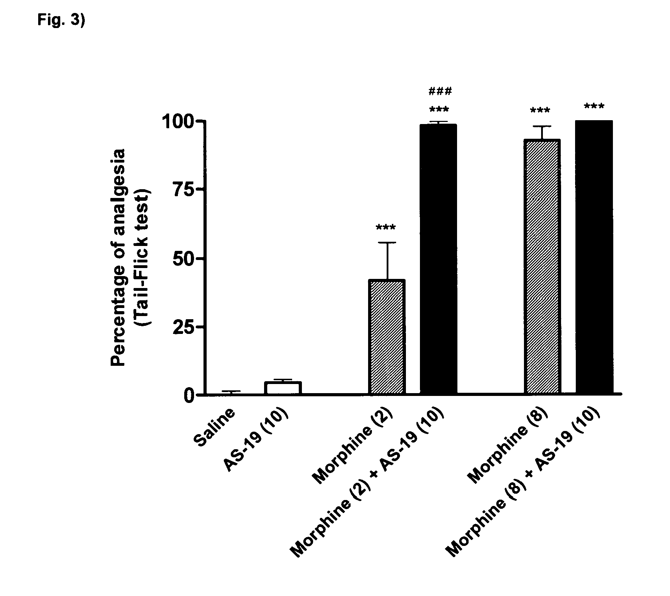

   Figure 3) refers to P-Example 3), a Tail-Flick-Test. Male CD1 Mice were receiving s.c. injections of test substances/combinations 45 min before measurement. The Baseline was determined based on saline injection. Significance values were:
   * *vs*. saline group
   # vs. respective morphine group
   The Combination according to the invention did show a highly super-additive effect at low concentrations of morphine.

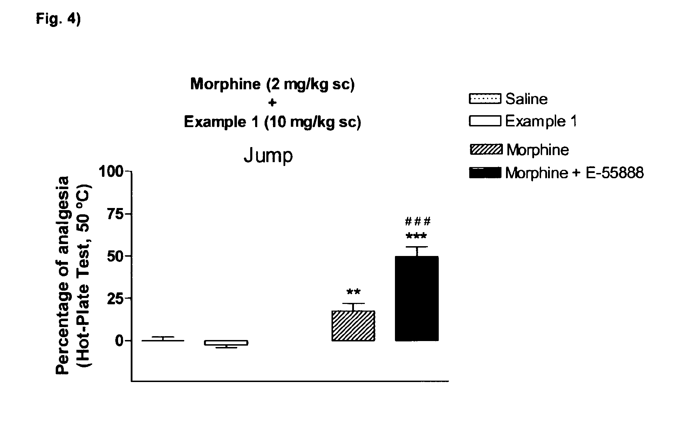

**Figure 4****) Hot Plate Test: Morphine in combination with Chemical Example 1: Jumping**
   Figure 4) refers to P-Example 4), a Hot-Plate-Test at 50°C. Male CD1 Mice were receiving s.c. injections of test substances/combinations 30 min before measurement. The tested substances were:
   - saline (vehicle/baseline)
   - Chemical Example 1, Dimethyl-(2-[3-(1,3,5-timethyl-1H-pyrazol-4-yl)-phenyl]-ethyl}-amine [10 mg/kg]
   - morphine [2 mg/kg]
   - morphine [2 mg/kg] + Chemical Example 1, Dimethyl-{2-[3-(1,3,5-trimethyl-1 H-pyrazol-4-yl)-phenyl]-ethyl}-amine [10 mg/kg]
   The sign of pain shown here is Jumping (Jump). The Baseline was determined based on saline injection. Significance values were:
   * *vs.* saline group
   # vs. respective morphine group
   The Combination according to the invention did show a highly super-additive effect.

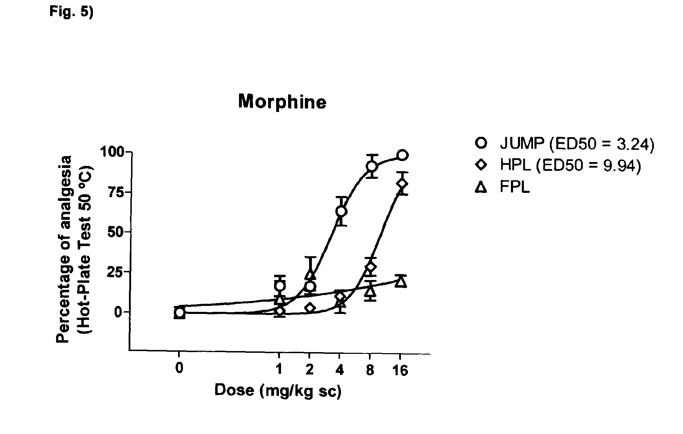

**Figure 5****) Hot Plate Test: Morphine; ED50**
   Figure 5) refers to P-Example 4), a Hot-Plate-Test at 50°C determining the ED50 of morphine in the three different signs of pain/symptoms. Male CD1 Mice were receiving s.c. injections of vehicle or morphine 30 min before measurement. The tested substances were - besides saline, the vehicle - 5 different concentrations of morphine [1, 2, 4, 8 or 16 mg/kg]. The sign of pain shown here is Front Paw Licking (FPL), Hind Paw Licking (HPL), jumping (Jump). The Baseline was determined based on saline injection and the 3 different ED50s were calculated.
   Fig. 5) shows these ED50s with no ED50 being determined for FPL.

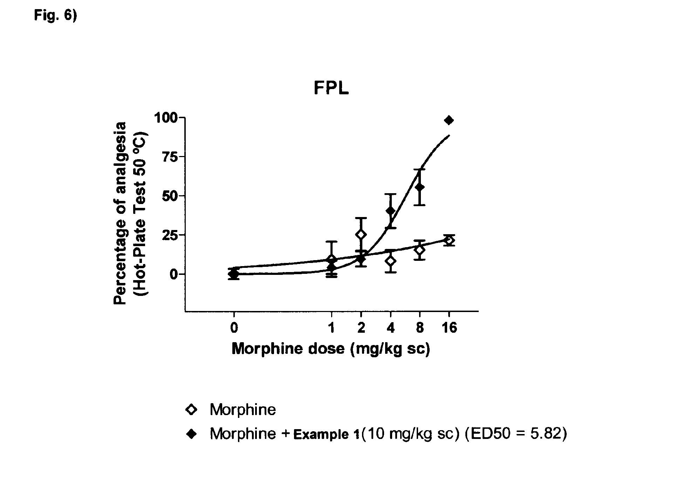

**Figure 6****) Hot Plate Test: Morphine in combination with Chemical Example 1; FPL;** ED50
   Figure 6) refers to P-Example 4), a Hot-Plate-Test at 50°C determining the ED50 of the combination in question. Male CD1 Mice were receiving s.c. injections of test substances/combinations 30 min before measurement. The tested substances were - besides saline the vehicle - 5 different concentrations of morphine [1, 2, 4, 8 or 16 mg/kg] with Chemical Example 1, Dimethyl-{2-[3-(1,3,5-trimethyl-1H-pyrazol-4-yl)-phenyl]-ethyl}-amine [10 mg/kg] as combination.
   The sign of pain shown here is Front Paw Licking (FPL). The Baseline was determined based on saline injection and the ED50 was calculated.
   Fig. 6) shows a pronounced lowering of the ED50 value due to the combination (ED50 = 5.82 mg/kg) compared to morphine alone, for which no ED50 could be determined (see Fig. 5).

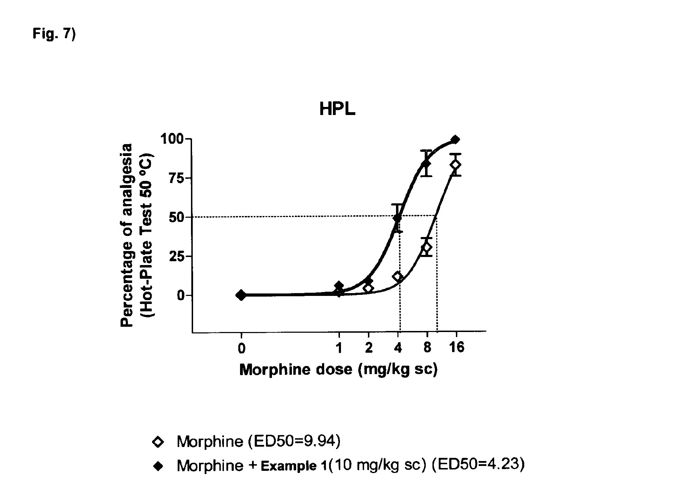

**Figure 7****) Hot Plate Test: Morphine in combination with Chemical Example 1: HPL: ED50**
   Figure 7) refers to P-Example 4), a Hot-Plate-Test at 50°C determining the ED50 of the combination in question. Male CD1 Mice were receiving s.c. injections of test substances/combinations 30 min before measurement. The tested substances were - besides saline the vehicle - 5 different concentrations of morphine [1, 2, 4, 8 or 16 mg/kg] with Chemical Example 1, Dimethyl-{2-[3-(1,3,5-trimethyl-1H-pyrazol-4-yl)-phenyl]-ethyl)-amine [10 mg/kg] as combination.
   The sign of pain shown here is Hind Paw Licking (HPL). The Baseline was determined based on saline injection and the ED50 was calculated.
   Fig. 7) shows a pronounced lowering of the ED50 value due to the combination (ED50 = 4.23 mg/kg) compared to morphine alone (ED50 = 9.94 mg/kg) (see Fig. 5).

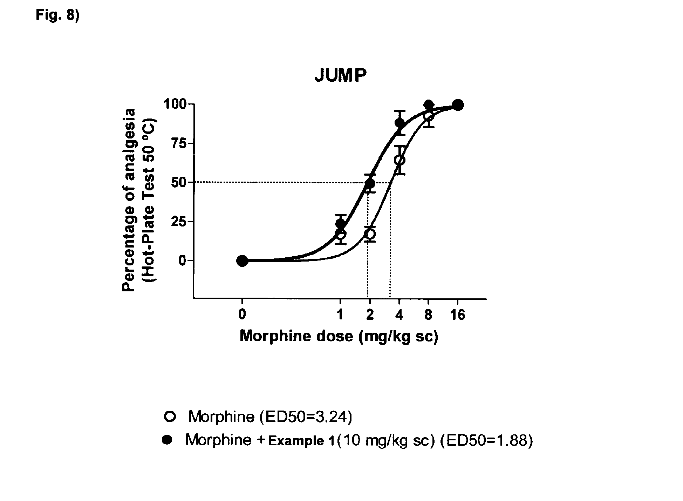

**Figure 8****) Hot Plate Test: Morphine in combination with Chemical Example 1: Jumping; ED50**
   Figure 8) refers to P-Example 4), a Hot-Plate-Test at 50°C determining the ED50 of the combination in question. Male CD1 Mice were receiving s.c. injections of test substances/combinations 30 min before measurement. The tested substances were - besides saline the vehicle - 5 different concentrations of morphine [1, 2, 4, 8 or 16 mg/kg] with Chemical Example 1, Dimethyl-{2-[3-(1,3,5-trimethyl-1H-pyrazol-4-yl)-phenyl]-ethyl}-amine [10 mg/kg] as combination.
   The sign of pain shown here is Jumping (Jump). The Baseline was determined based on saline injection, and the different ED50s was calculated.
   Fig. 8) shows a pronounced lowering of the ED50 value due to the combination (ED50 = 1.88 mg/kg) compared to morphine alone (ED50 = 3.24 mg/kg) (see Fig. 5).

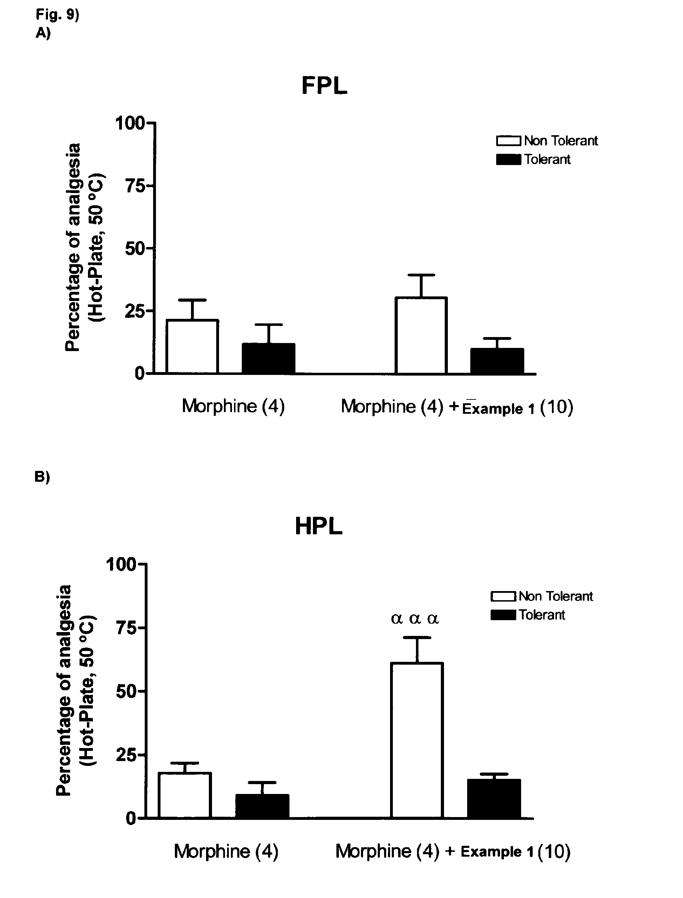

**Figure 9A****) Hot Plate Test: Morphine in combination with Chemical Example 1; FPL; after morphine tolerance expression**
   Figure 9A) refers to P-Example 5), a Hot-Plate-Test at 50°C after development of tolerance against morphine in one group of animals. Male CD1 Mice were devided in 2 groups, the group receiving s.c. injections of morphine in high doses for four days to develop tolerance (hereinafter called Tolerants), and the group receiving only vehicle (callede Non-Tolerants).
   On day five mice of the Tolerant and the Non-Tolerant group were receiving s.c. injections of either morphine at 4 mg/kg or morphine [4 mg/kg] with Chemical Example 1, Dimethyl-{2-[3-(1,3,5-trimethyl-1H-pyrazol-4-yl)-phenyl]-ethyl)-amine [10 mg/kg] as combination.
   The sign of pain shown here is Front Paw Licking (FPL). The Baseline was determined based on saline injection.
   The mice were placed 30 minutes after injection on the hot-plate.
   As shown in Fig. 9A) the analgesic effect of morphine alone was reduced in the Tolerant group compared to the Non-Tolerant group. Also the Combination according to the invention did show no effect on the Tolerant group compared to the group treated only with morphine, but again an effect in FPL in the Non-Tolerant group.
**Figure 9B****) Hot Plate Test: Morphine in combination with Chemical Example 1; HPL; after morphine tolerance expression**
   Figure 9B) refers to P-Example 5), a Hot-Plate-Test at 50°C after development of tolerance against morphine in one group of animals. Male CD1 Mice were devided in 2 groups, the group receiving s.c. injections of morphine in high doses for four days to develop tolerance (hereinafter called Tolerants), and the group receiving only vehicle (callede Non-Tolerants). On day five mice of the Tolerant and the Non-Tolerant group were receiving s.c. injections of either morphine at 4 mg/kg or morphine [4 mg/kg] with Chemical Example 1, Dimethyl-{2-[3-(1,3,5-trimethyl-1H-pyrazol-4-yl)-phenyl]-ethyl}-amine [10 mg/kg] as combination.
   The sign of pain shown here is Hind Paw Licking (HPL). The Baseline was determined based on saline injection.
   The mice were placed 30 minutes after injection on the hot-plate. Significance values were determined vs. respective "non tolerant" group, the "Morphine-tolerant group" or the "Morphine-non tolerant group" with *** p<0.001 vs. respective "non tolerant" group, # # # p<0.001 vs. "Morphine-tolerant group" and α α α p<0.001 *vs.* "Morphine-non tolerant group".
   As shown in Fig. 9B) the analgesic effect of morphine alone was reduced in the Tolerant group compared to the Non-Tolerant group. Also the Combination according to the invention did show no effect on the Tolerant group compared to the group treated only with morphine, but a significant super-additive effect on HPL in the Non-Tolerant group.

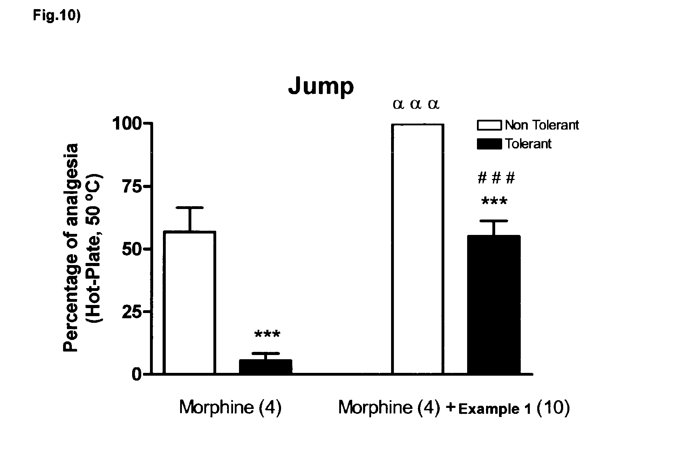

**Figure 10****) Hot Plate Test: Morphine in combination with Chemical Example 1: Jump; after morphine tolerance expression**
   Figure 10) refers to P-Example 5), a Hot-Plate-Test at 50°C after development of tolerance against morphine in one group of animals. Male CD1 Mice were devided in 2 groups, the group receiving s.c. injections of morphine in high doses for four days to develop tolerance (hereinafter called Tolerants), and the group receiving only vehicle (callede Non-Tolerants). On day five mice of the Tolerant and the Non-Tolerant group were receiving s.c. injections of either morphine at 4 mg/kg or morphine [4 mg/kg] with Chemical Example 1, Dimethyl-{2-[3-(1,3,5-trimethyl-1H-pyrazol-4-yl)-phenyl]-ethyl}-amine [10 mg/kg] as combination.
   The sign of pain shown here is Jumping (Jump). The Baseline was determined based on saline injection.
   The mice were placed 30 minutes after injection on the hot-plate. Significance values were determined vs. the respective the "Morphine-non tolerant group" with α α α p<0.001.
   As shown in Fig. 10) the analgesic effect of morphine alone was reduced in the Tolerant group compared to the Non-Tolerant group. Also the Combination according to the invention did show a clear and significant effect on both the Tolerant and the Non-Tolerant Group compared to the respective groups treated only with morphine.

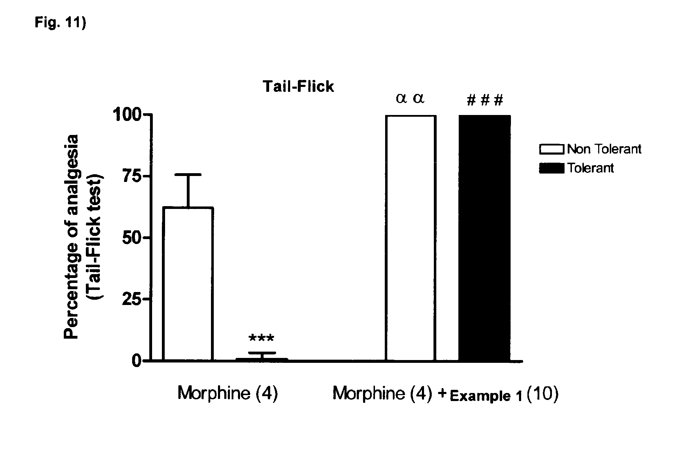

**Figure 11****) Tail-Flick-Test: Morphine in combination with Chemical Example 1; after morphine tolerance expression**
   Figure 11) refers to P-Example 6), a Tail-Flick-Test after development of tolerance against morphine in one group of animals. Male CD1 Mice were devided in 2 groups, the group receiving s.c. injections of morphine in high doses for four days to develop tolerance (hereinafter called Tolerants), and the group receiving only vehicle (callede Non-Tolerants). On day five mice of the Tolerant and the Non-Tolerant group were receiving s.c. injections of either morphine at 4 mg/kg or morphine [4 mg/kg] with Chemical Example 1, Dimethyl-{2-[3-(1,3,5-trimethyl-1H-pyrazol-4-yl)-phenyl]-ethyl}-amine [10 mg/kg] as combination. The Baseline was determined based on saline injection.
   The mice were placed 45 minutes after injection in the tail-flick-apparatus. Significance values were determined vs. respective "non tolerant" group, the "Morphine-tolerant group" or the "Morphine-non tolerant group" with *** p<0.001 *vs.* respective "non tolerant" group, # # # p<0.001 *vs.* "Morphine-tolerant group" and α α α p<0.001 *vs.* "Morphine-non tolerant group".
   As shown in Fig. 11) the analgesic effect of morphine alone was significantly reduced in the Tolerant group compared to the Non-Tolerant group. Also the combination does show a clear and significant effect on both the Tolerant and the Non-Tolerant Group compared to the respective groups treated only with morphine.

### Examples:

### CHEMICAL EXAMPLES:

### 1^{st} Group of Chemical Examples:

### C-Example 1

### Dimethyl-{2-[3-(1,3,5-trimethyl-1H-pyrazol-4-yl)-phenyl]-ethyl}-amine

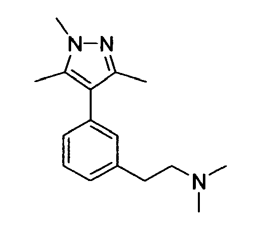

In a more general form Chemical Example 1 was prepared according to the following Scheme:

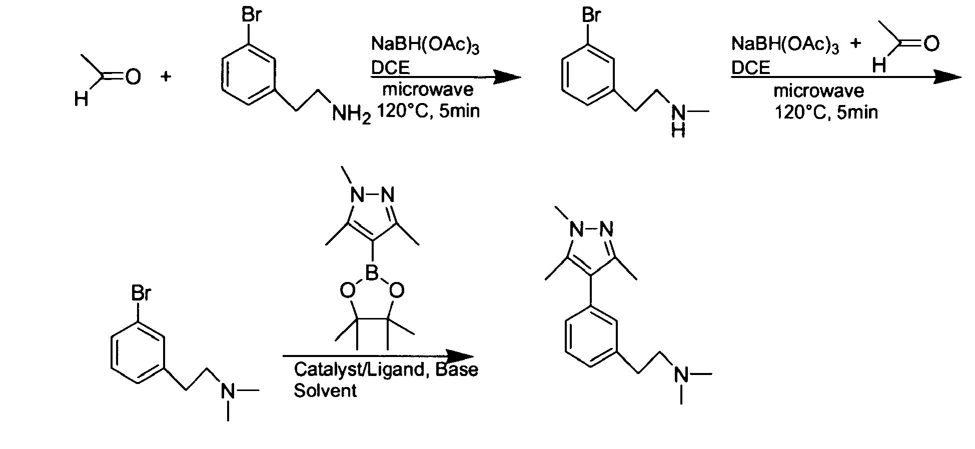

Base, Catalyst, Solvent, Temperature and reaction time in the last step, a so-called "Suzuki Reaction" were varied and in part orientated on literature such as JACS, 2002, 1162 and Angew. Chem. Int. Ed. 2006, 1282.

Thus the base was selected from K₂CO₃, K₃PO₄ and used in amounts between 1.7 and 5 eq. based on the amount of [2-(3-Bromo-phenyl)-ethyl]-dimethyl-amine introduced.
The Solvent was selected from DME/H₂O 1/1, and Dioxane/H₂O 2/1.
The Catalyst/ligand was selected from 1. (Pd₂(dba)₃, 4.3mol% + DPEPhos, 10mol%), 2. (Pd(PPh₃)₄, 10 mol%), 3. (Pd₂(dba)₃, 5 mol% + DPEPhos, 6 mol%) and 4.(Pd₂(dba)₃, 2 mol% + PCy₃ 4.8 mol%).
The Temperature was usually 10°C and the Reaction Time varied between a few minutes and 20 hours and even microwave irridation was used.
Yields varied between 8% and 78%.

After its precursors (A and B) were prepared Chemical Example 1 was synthesized following different methods explained more in detail below:

### Chemical Example A:

### [2-(3-Bromo-phenyl)-ethyl]-methyl-amine

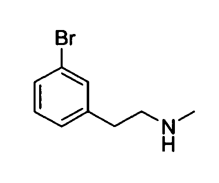

2-(3-Bromo-phenyl)-ethylamine (0.5 mmol) and formaldehyde (0.42 mmol) were mixed in 3 ml of 1,2-dichloroethane in a process vial, which was sealed with a septum. Sodium triacetoxyborohydride (0.84 mmol) was added under argon atmosphere. The suspension was subjected to microwave irradiating conditions (CEM Discover^{®} equipped with a CEM Explorer^{®} automated reaction handling module). The reaction mixture was heated for 5 min at 90°C and then cooled. The crude was evaporated to dryness and then suspended in aqueous NaHCO₃. The product was extracted with CH₂Cl₂ and washed with aqueous NaHCO₃. The CH₂Cl₂ extract was dried with anhydrous Na₂SO₄, filtered and evaporated to dryness to give the crude product [2-(3-bromo-phenyl)-ethyl]-methyl-amine. The crude was purified by flash column chromatography (CH₂Cl₂-MeOH as eluents) by using a CombiFlash Companion^{™} system to yield the title compound (75%) as colourless oil.

### Chemical Example B:

### [2-(3-Bromo-phenyl)-ethyl]-dimethyl-amine

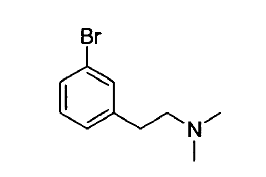

2-(3-Bromo-phenyl)-ethylamine (0.5 mmol) and formaldehyde (2.5 mmol) were mixed in 5 ml of 1,2-dichloroethane in a process vial, which was sealed with a septum. Sodium triacetoxyborohydride (1 mmol) was added under argon atmosphere. The suspension was subjected to microwave irradiating conditions (CEM Discover^{®} equipped with a CEM Explorer^{®} automated reaction handling module). The reaction mixture was heated for 5 min at 120°C and then cooled. The crude was evaporated to dryness and then suspended in aqueous NaHCO₃. The product was extracted with CH₂Cl₂ and washed with aqueous NaHCO₃. The CH₂Cl₂ extract was dried with anhydrous Na₂SO₄, filtered and evaporated to dryness to give the crude product [2-(3-bromo-phenyl)-ethyl]-dimethyl-amine. The crude was purified by flash column chromatography (CH₂Cl₂-MeOH as eluents) by using a CombiFlash Companion^{™} system to yield the title compound (86%) as colourless oil.

### C-Example 1

### Dimethyl-{2-[3-(1,3,5-trimethyl-1H-pyrazol-4-yl)-phenyl]-ethyl}-amine

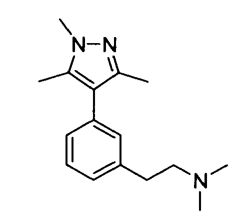

**(Method 1):** [2-(3-Bromo-phenyl)-ethyl]-dimethyl-amine (0.44 mmol) was dissolved in DME/H₂O 1/1 (8 mL) under argon atmosphere. 1,3,5-Trimethyl-1H-pyrazole-4-boronic acid pinacol ester (0.66 mmol), K₂CO₃ (2.19 mmol), and tetrakis-(triphenylphosphine)palladium (10 mol%, 0.044 mmol) were added and the reaction mixture was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion^{™} system to yield the title compound (60%) as colourless oil.

**(Method 2):** [2-(3-Bromo-phenyl)-ethyl]-dimethyl-amine (0.22 mmol) was dissolved in DME/H₂O 1/1 (4 mL) under argon atmosphere in a process vial. 1,3,5-Trimethyl-1H-pyrazole-4-boronic acid pinacol ester (0.33 mmol), K₂CO₃ (1.1 mmol), and tetrakis-(triphenylphosphine)palladium (10 mol%, 0.022 mmol) were added and the vial was sealed with a septum. The reaction mixture was subjected to microwave irradiating conditions, heated for 5 min at 100°C and then cooled. The reaction mixture was evaporated to dryness, then dissolved in CHCI₃ and filtered through Celite® to give the crude product dimethyl-{2-[3-(1,3,5-trimethyl-1H-pyrazol-4-yl)-phenyl}-ethyl)-amine. The crude was purified by flash column chromatography (CH₂Cl₂-MeOH as eluents) by using a CombiFlash Companion^{™} system to yield the title compound (78%) as colourless oil.

**(Method 3):** [2-(3-Bromo-phenylfethyl]-dimethyl-amine (0.44 mmol), 1,3,5-trimethyl-1H-pyrazole-4-boronic acid pinacol ester (0.876 mmol), tris(dibenzylideneacetone)dipalladium (5 mol%, 0.022 mmol) and DPEPhos (6 mol%, 0.026 mmol) were dissolved in dioxane. K₃PO₄ (1.32 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-timethyl-1H-pyrazol-4-yl)phenyl]-ethyl}amine. The crude was purified by flash column chromatography (CH₂Cl₂-MeOH as eluents) by using a CombiFlash Companion^{™} system to yield the title compound (24%) as colourless oil.

**(Method 4):** [2-(3-Bromo-phenyl}-ethyl]-dimethyl-amine (0.44 mmol), 1,3,5-trimethyl-1H-pyrazole-4-boronic acid pinacol ester (0526 mmol), tris(dibenzylideneacetone)dipalladium (2 mol%, 0.009 mmol) and tricyclohexylphosphine (4.8 mol%, 0.021 mmol) were dissolved in 4 mL of dioxane. K₃PO₄ (0.745 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion^{™} system to yield the title compound (8%) as colourless oil.

**(Method 5):** [2-(3-Bromo-phenyl)-ethyl]-dimethyl-amine (0.44 mmol), 1,3,5-trimethyl-1 H-pyrazole-4-boronic acid pinacol ester (0526 mmol), tris(dibenzylideneacetone)dipalladium (4.3 mol%, 0.019 mmol) and DPEPhos (10 mol%, 0.044 mmol) were dissolved in 4 mL of dioxane. K₃PO₄ (2.19 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-trimethy)-1H-pyrazo)-4-yl)-phenyl]-ethyl}-amine. The crude was purified by flash column chromatography (CH₂Cl₂-MeOH as eluents) by using a CombiFlash Companion^{™} system to yield the title compound (34%) as colourless oil.

The other examples were or are prepared according to or analogous to the reaction schemes and descriptions given above and are listed (where applicable) together with their binding data, their Mass and their 1 H-NMR data in the following table:

| **Chemical Example** | **Structure** | **Name** | **IC50 (nM)** | **%inh (10⁻⁶M)** | **%inh (10⁻⁷M)** | **%inh (10⁻⁸M)** | **¹H-NMR** | **MS (APCI (M+H)⁺)** |
|---|---|---|---|---|---|---|---|---|
| 1 | 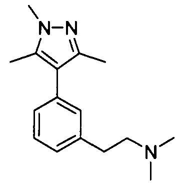 | Dimethyl-{2-[3-(1,3,5-trimethyl-1H-pyrazol-4-yl)-phenyl]-ethyl}-amine | 6.1 | | 98.6 | 60.8 | (300 MHz, METHANOL-*d*₄) δ ppm 2.18 (d, *J*=2.78 Hz, 3 H) 2.25 (d, *J*=2.64 Hz, 3 H) 2.37 (d, *J*=2.64 Hz, 6 H) 2.61 - 2.70 (m, 2 H) 2.81 - 2.90 (m, 2 H) 3.76 (d, *J*=2.64 Hz, 3 H) 7.07 - 7.13 (m, 2 H) 7.17 (d, *J*=7.47 Hz, 1 H) 7.31 - 7.39 (m, 1 H). | 258.08 |
| 2 | 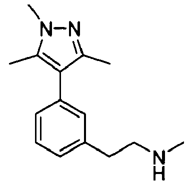 | Methyl-{2-[3-(1,3,5-trimethyl-1H-pyrazol-4-yl)-phenyl]-ethyl}-amine | | | | | (300 MHz, CHLOROFORM-*d*) δ ppm 1.95 (s, 3 H) 2.25 (s, 6 H) 2.85 (t, *J*=6.88 Hz, 2 H) 3.54 (q, *J*=6.88 Hz, 2 H) 3.79 (s, 3 H) 5.49 (br. s., 1 H) 7.06 (s, I H) 7.11 (d, *J*=8.06 Hz, 2 H) 7.35 (t, *J*=7.62 Hz, 1H) | |
| 3 | 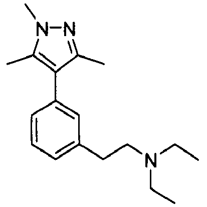 | Diethyl-{2-[3-(1,3,5-trimethyl-1H-pyrazol-4-yl)-phenyl]-ethyl}-amine | 76.2 | 98.1 | 41.5 | 4.8 | (300 MHz, METHANOL-*d*₄) δ ppm 1.11 (t, *J*=7.25 Hz, 6 H) 2.16 (s, 3 H) 2.23 (s, 3 H) 2.71 (q, *J*=7.25 Hz, 4 H) 2.75 - 2.84 (m, 4 H) 3.75 (s, 3 H) 7.01 - 7.11 (m, 2 H) 7.15 (m, 1 H) 7.34 (t, *J*=7.91 Hz, 1H) | 286.18 |
| 4 | 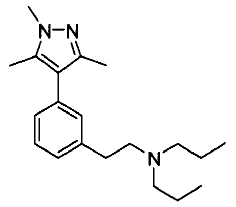 | Dipropyl-{2-[3-(1,3,5-trimethyl-1H-pyrazol-4-yl)-phenyl]-ethyl}-amine | 56.9 ± 17.9 | 90 | 98.7 | 48.6 | (300 MHz, METHANOL-*d*₄) δ ppm 0.92 (t, *J*=7.40 Hz, 6 H) 1.42 - 1.61 (m, 4 H) 2.18 (s, 3 H) 2.25 (s, 3 H) 2.44 - 2.58 (m, 4 H) 2.67 - 2.86 (m, 4 H) 3.77 (s, 3 H) 7.07 (m, 1H) 7.09 (s, 1 H) 7.16 (m, 1 H) 7.34 (t, *J*=7.84 Hz, 1 H) | 314.2 |
| 5 | 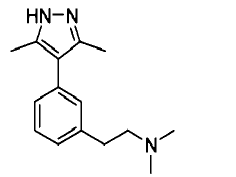 | {2-[3-(3,5-Dimethyl-1H-pyrazol-4-yl)-phenyl]-ethyl} -dimethyl-amine | | | | | (300 MHz, CHLOROFORM-*d*) δ ppm 2.23 (s, 6 H) 2.32 (s, 6 H) 2.59 (t, *J*=8.64 Hz, 2 H) 2.81 (t, *J*=8.64 Hz, 2 H) 6.97 - 7.13 (m, 3 H) 7.27 (t, *J*=7.69 Hz, 1 H) | |
| 6 | 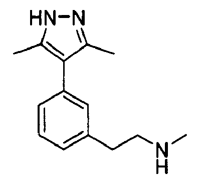 | {2-[3-(3,5-Dimethyl-1H-pyrazol-4-yl)-phenyl]-ethyl}-methyl-amine | | | | | (300 MHz, METHANOL-*d*₄) δ ppm 1.91 (s, 3 H) 2.24 (s, 6 H) 2.83 (t, *J*=7.32 Hz, 2 H) 3.42 (t, *J*=7.32 Hz, 2 H) 7.08 - 7.18 (m, 3 H) 7.34 (t, *J*=7.76 Hz, 1 H) | |
| 7 | 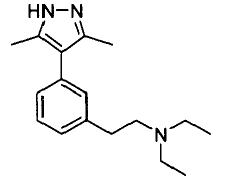 | {2-[3-(3,5-Dimethyl-1H-pyrazol-4-yl)-phenyl]-ethyl}-diethyl-amine | | | | | (300 MHz, CHLOROFORM-*d*) δ ppm 1.09 (t, *J*=7.03 Hz, 6 H) 2.23 (s, 6 H) 2.70 (m, 4 H) 2.81 (br. s., 4 H) 6.98 - 7.13 (m, 3 H) 7.27 (t, *J*=7.76 Hz, 1 H) | |
| 8 | 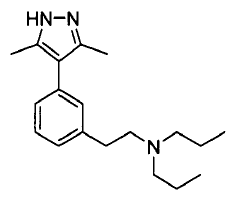 | {2-[3-(3,5-Dimethyl-1H-pyrazol-4-yl)-phenyl]-ethyl}-dipropyl-amine | | | | | (300 MHz, CHLOROFORM-*d*) δ ppm 0.84 (t, *J*=7.32 Hz, 6 H) 1.48 (br. s., 4 H) 2.23 (s, 6 H) 2.47 (m, 4 H) 2.78 (br. s., 4 H) 6.96 - 7.09 (m, 3 H) 7.26 (t, *J*=7.91 Hz, 1 H) | |
| 9 | 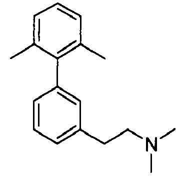 | [2-(2',6'-Dimethyl-biphenyl-3-yl)-ethyl]-dimethyl-amine | 10.1 ± 0.8 | 104.6 | 88.9 | 65.9 | (400 MHz, METHANOL-*d*₄) δ ppm 1.98 (s, 6 H) 2.33 (s, 6 H) 2.58 - 2.65 (m, 2 H) 2.81-2.87 (m, 2 H) 6.93-6.99(m, 2 H) 7.04 - 7.13 (m, 3 H) 7.21 (d, *J*=7.82 Hz, 1 H) 7.36 (t, *J*=7.62 Hz, 1 H) | 254.25 |
| 10 | 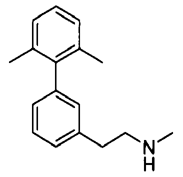 | [2-(2',6'-Dimethyl-biphenyl-3-yl)-ethyl]-methyl-amine | | | | | (300 MHz, METHANOL-*d*₄) δ ppm 1.90 (s, 3 H) 1.99 (s, 6 H) 2.84 (t, *J*=7.32 Hz, 2 H) 3.43 (t, *J*=7.32 Hz, 2 H) 6.91 - 7.00 (m, 2 H) 7.03 - 7.13 (m, 3 H) 7.22 (d, *J*=7.76 Hz, 1 H) 7.37 (t, *J*=7.47 Hz, 1 H) | |
| 11 | 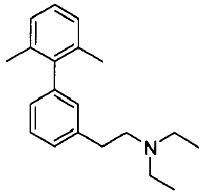 | [2-(2',6'-Dimethyl-biphenyl-3-yl)-ethyl]-diethyl-amine | 43.5 ± 15.9 | 98.2 | 62.3 | -13.7 | (300 MHz, METHANOL-*d*₄) δ ppm 1.11 (t, *J*=7.18 Hz, 6 H) 1.99 (s, 6 H) 2.68 (q, *J*=7.18 Hz, 4 H) 2.74 - 2.86 (m, 4 H) 6.91 - 7.00 (m, 2 H) 7.02 - 7.15 (m, 3 H) 7.21 (d, *J*=7.76 Hz, 1 H) 7.38 (t, *J*=7.47 Hz, 1 H) | 282.1 |
| 12 | 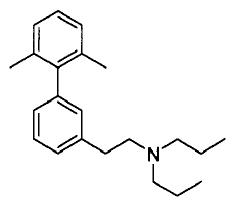 | [2-(2',6'-Dimethyl-biphenyl-3-yl)-ethyl]-dipropyl-amine | 42.6 ± 21.1 | 93.3 | 92.2 | 43.8 | (400 MHz, METHANOL-*d*₄) δ ppm 0.90 (t, *J*=7.42 Hz, 6 H) 1.47 - 1.58 (m, *J*=7.62, 4 H) 1.98 (s, 6 H) 2.54 (m, 4 H) 2.80 (br. s., 4 H) 6.92 - 6.98 (m, 2 H) 7.04 - 7.13 (m, 3 H) 7.20 (d, *J*=7.42 Hz, 1 H) 7.36 (t, *J*=7.42 Hz, 1 H) | 310.28 |
| 13 | 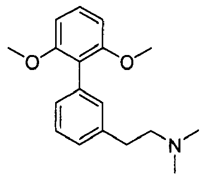 | [2-(2',6'-Dimethoxy-biphenyl-3-yl)-ethyl]-dimethyl-amine | 19.7 ± 1.5 | 104.8 | 60.5 | 54.4 | (300 MHz, METHANOL-*d*₄) δ ppm 2.37 (s, 6 H) 2.67 (m, 2 H) 2.84 (m, 2 H) 3.69 (s, 6 H) 6.71 (d, *J*=8.35 Hz, 2 H) 7.01 - 7.15 (m, 3 H) 7.28 (m, 1 H) 7.26 (d, *J*=7.62 Hz, 1 H) | 286.14 |
| 14 | 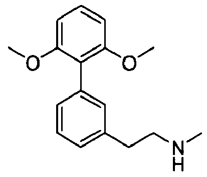 | [2-(2',6'-Dimethoxy-biphenyl-3-yl)-ethyl]-methyl-amine | | | | | (300 MHz, METHANOL-*d*₄) δ ppm 1.90 (s, 3 H) 2.79 (t, *J*=7.32 Hz, 2 H) 3.40 (t, *J*=7.32 Hz, 2 H) 3.68 (s, 6 H) 6.70 (d, *J*=8.50 Hz, 2 H) 6.99 - 7.08 (m, 2 H) 7.12 (d, *J*=7.76 Hz, 1 H) 7.26 (t, *J*=8.35 Hz, 2 H) | |
| 15 | 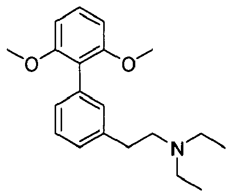 | [2-(2',6'-Dimethoxy-biphenyl-3-yl)-ethyl]-diethyl-amine | | | | | (300 MHz, METHANOL-*d*₄) δ ppm 1.11 (t, *J*=7.18 Hz, 6 H) 2.69 (q, *J*=7.28 Hz, 4 H) 2.78 (s, 4 H) 3.68 (s, 6 H) 6.70 (d, *J*=8.35 Hz, 2 H) 6.98 - 7.17 (m, 3 H) 7.17 - 7.33 (m, 2 H) | 314.15 |
| 16 | 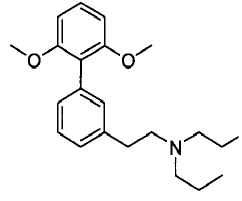 | [2-(2',6'-Dimethoxy-biphenyl-3-yl)-ethyl]-dipropyl-amine | | | | | (300 MHz, METHANOL-*d*₄) δ ppm 0.92 (t, *J*=7.40 Hz, 6 H) 1.46 - 1.64 (m, 4 H) 2.49 - 2.64 (m, 4 H) 2.81 (s, 4 H) 3.68 (s, 6 H) 6.70 (d, *J*=8.35 Hz, 2 H) 7.00 - 7.09 (m, 2 H) 7.11 (d, *J*=7.76 Hz, 1 H) 7.25 (dd, *J*=8.13, 2.27 Hz, 1 H) 7.29 (m, 1 H) | 342.27 |
| 17 | 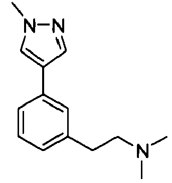 | Dimethyl- {2-[3-(1-methyl-1H-pyrazol-4-yl)-phenyl]-ethyl}-amine | | 46 | 16.9 | -1 | (400 MHz, METHANOL-*d*₄) δ ppm 2.34 (s, 6 H) 2.61 (t, *J*=8.60 Hz, 2 H) 2.80 (t, *J*=8.60 Hz, 2 H) 3.91 (s, 3 H) 7.06 (d, *J*=7.42 Hz, 1 H) 7.26 (t, *J*=7.62 Hz, 1 H) 7.40 (s, 1 H) 7.37 (d, *J*=7.42 Hz, 1 H) 7.80 (s, 1 H) 7.93 (s, 1 H) | 230.32 |
| 18 | 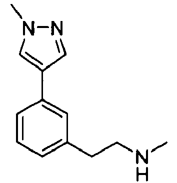 | Methyl-{2-[3-(1-methyl-1H-pyrazol-4-yl)-phenyl]-ethyl}-amine | | | | | | |
| 19 | 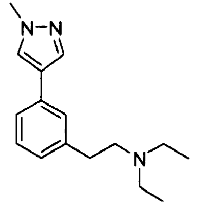 | Diethyl-{2-[3-(1-methyl-1H-pyrazol-4-yl)-phenyl]-ethyl}-amine | | | | | (300 MHz, METHANOL-*d*₄) δ ppm 1.15 (t, *J*=7.18 Hz, 6 H) 2.78 (q, *J*=7.18 Hz, 4 H) 2.84 (s, 4 H) 3.92 (s, 3 H) 7.08 (d, *J*=7.62 Hz, 1 H) 7.28 (t, *J*=7.62 Hz, 1 H) 7.38 (m, 2 H) 7.81 (s, 1 H) 7.95 (s, 1 H) | 258.20 |
| 20 | 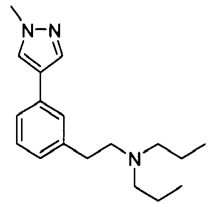 | {2-[3-(1-Methyl-1H-pyrazol-4-yl)-phenyl]-ethyl}-dipropyl-amine | | | | | (300 MHz, METHANOL-*d*₄) δ ppm 0.92 (t, *J*=7.32 Hz, 6 H) 1.41 - 1.61 (m, 4 H) 2.55 (m, J=8.06 Hz, 4 H) 2.78 (s, 4 H) 3.92 (s, 3 H) 7.05 (d, *J*=7.62 Hz, 1 H) 7.26 (t, *J*=7.54 Hz, 1 H) 7.33 - 7.42 (m, 2 H) 7.80 (s, 1 H) 7.94 (s, 1 H) | 286.23 |
| 21 | 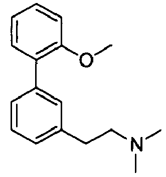 | [2-(2'-Methoxy-biphenyl-3-yl)-ethyl]-dimethyl-amine | 19.9 ± 1.7 | 95.4 | 69.2 | 20 | (400 MHz, METHANOL-*d*₄) δ ppm 2.33 (s, 6 H) 2.59 - 2.65 (m, 2 H) 2.80-2.86 (m, 2 H) 3.77 (s, 3 H) 6.99 (td, *J*=7.42, 1.17 Hz, 1 H) 7.04 (d, *J*=7.82 Hz, 1 H) 7.15 (m, 1 H) 7.23 -7.32 (m, 5 H) | 256.25 |
| 22 | 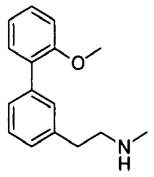 | [2-(2'-Methoxy-biphenyl-3-yl)-ethyl]-methyl-amine | | | | | (300 MHz, CHLOROFORM-*d*) δ ppm 1.95 (s, 3 H) 2.86 (t, *J*=6.81 Hz, 2 H) 3.56 (q, *J*=6.64 Hz, 2 H) 3.82 (s, 3 H) 5.55 (br. s., 1 H) 6.96 - 7.07 (m, 1 H) 7.01 (t, *J*=8.13 Hz, 1 H) 7.16 (d, *J*=7.03 Hz, 1 H) 7.28 - 7.43 (m, 5 H) | |
| 23 | 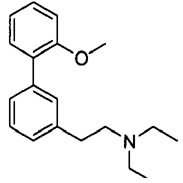 | Diethyl-[2-(2'-methoxy-biphenyl-3-yl)-ethyl]-amine | | | | | (300 MHz, METHANOL-*d*₄) δ ppm 1.13 (t, *J*=7.18 Hz, 6 H) 2.74 (q, *J*=7.18 Hz, 4 H) 2.83 (s, 4 H) 3.78 (s, 3 H) 6.99 (td, *J*=7.43, 1.10 Hz, 1 H) 7.05 (d, *J*=8.20 Hz, 1 H) 7.16 (ddd, *J*=3.84, 2.53, 2.27 Hz, 1 H) 7.22 - 7.36 (m, 5H) | 284.21 |
| 24 | 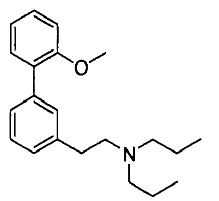 | [2-(2'-Methoxy-biphenyl-3-yl)-ethyl]-dipropyl-amine | 84.9 | 92.4 | 37.8 | -1.8 | (300 MHz, METHANOL-*d*₄) δ ppm 0.92 (t, *J*=7.32 Hz, 6 H) 1.47 - 1.61 (m, 4 H) 2.54 (m, 4 H) 2.79 (s, 4 H) 3.78 (s, 3 H) 7.00 (t, *J*=7.47 Hz, 1 H) 7.06 (d, *J*=8.20 Hz, 1 H) 7.14 (td, *J*=4.21, 1.98 Hz, 1 H) 7.23 - 7.34 (m, 5 H) | 312.22 |
| 25 | 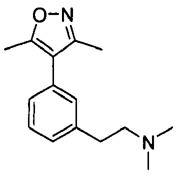 | {2-[3-(3,5-Dimethyl-isoxazol-4-yl)-phenyl]-ethyl}-dimethyl-amine | | | | | (300 MHz, METHANOL-*d*₄) δ ppm 2.26 (s, 3 H) 2.41 (s, 3 H) 2.97 (s, 6 H) 3.13 (m, 2 H) 3.43 (m, 2 H) 7.28 (m, 2 H) 7.36 (d, *J*=7.47 Hz, 1 H) 7.48 (t, *J*=7.54 Hz, 1 H) | 245.20 |
| 26 | 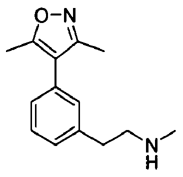 | {2-[3-(3,5-Dimethyl-isoxazol-4-yl)-phenyl]-ethyl}-methyl-amine | | | | | | |
| 27 | 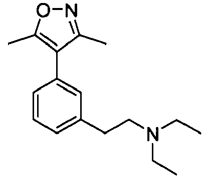 | {2-[3-(3,5-Dimethyl-isoxazol-4-yl)-phenyl]-ethyl}-diethyl-amine | | | | | (300 MHz, METHANOL-*d*₄) δ ppm 1.10 (t, *J*=7.18 Hz, 6 H) 2.24 (s, 3 H) 2.39 (s, 3 H) 2.68 (q, *J*=7.18 Hz, 4 H) 2.72 - 2.86 (m, 4 H) 7.12 - 7.20 (m, 2 H) 7.24 (d, *J*=7.76 Hz, 1 H) 7.39 (t, *J*=7.54 Hz, 1 H) | 273.16 |
| 28 | 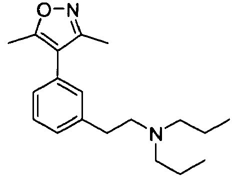 | {2-[3-(3,5-Dimethyl-isoxazol-4-yl)-phenyl]-ethyl }-dipropyl-amine | | | | | (300 MHz, METHANOL-*d*₄) δ ppm 0.91 (t, *J*=7.40 Hz, 6 H) 1.45 - 1.60 (m, 4 H)) 2.25 (s, 3 H) 2.40 (s, 3 H) 2.55 (m, 4 H) 2.72 - 2.87 (m, 4 H) 7.13 - 7.20 (m, 2 H) 7.25 (d, *J*=7.62 Hz, 1 H) 7.40 (t, *J*=7.54 Hz, 1 H) | 301.31 |

### 2^{nd} Group of Chemical Examples:

### Chemical Example G: (2S)-Methyl-[5-(1,3,5-trimethyl-1H-pyrazol-4-yl)-1,2,3,4-tetrahydro-naphthalen-2-yl]-amine

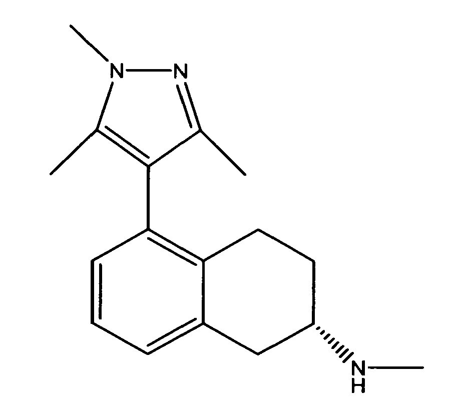

Methyl-[5-(1,3,5-trimethyl-1*H*-pyrazol-4-yl)-1,2,3,4-tetrahydro-naphthalen-2-yl]-amine Chemical Example G was prepared according to the following Scheme 2

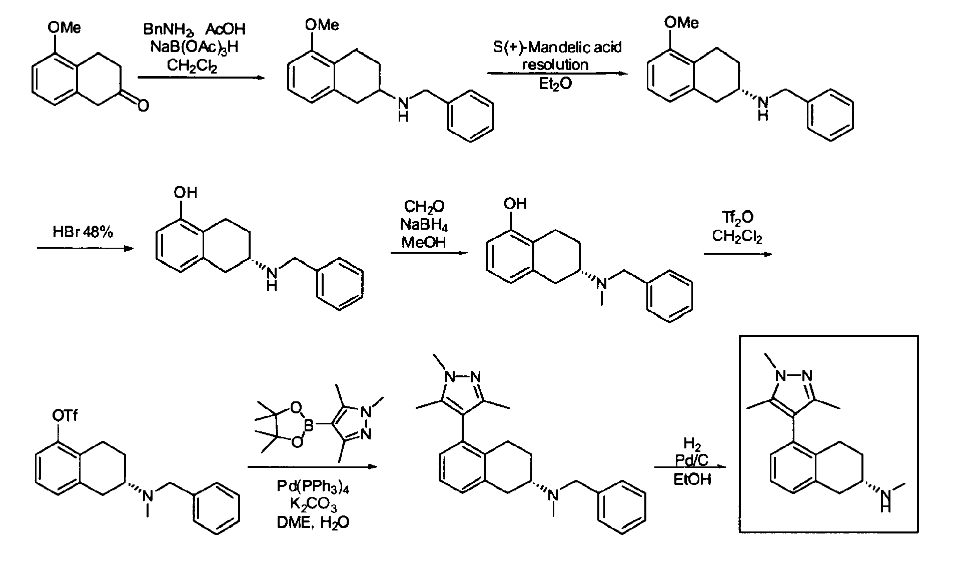

Step by step Chemical Example G was prepared as follows starting from Chemical Example A, whose precursor is readily available and may also be synthesized by anyone skilled in the art:

### Chemical Example A

### N-Benzyl-N-(5-methoxy-1,2,3,4-tetrahydronaphthalen-2-yl)amine

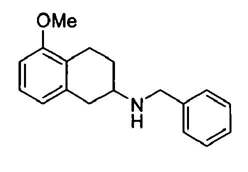

To a solution of 5-methoxy-2-tetralone (30 g, 170.24 mmol) dissolved in CH₂Cl₂ (250 mL) were added benzylamine (23 mL, 212.80 mmol) and AcOH (0.97 mL, 17.02 mmol), and the mixture was stirred for 4 h at room temperature. It was then cooled to 0 °C and NaB(OAc)₃H (0.38 eq, 13.71 g, 64.69 mmol) was added over a period of 20 min. After 1 h stirring at 0 °C, NaB(OAc)₃H (1.07 eq, 38.61 g, 182.16 mmol) was added over a period of 30 min. It was added CH₂CI₂ (100 mL), the reaction mixture warmed to room temperature and stirred for 15 h. The mixture was cooled again to 0°C, and H₂O (200 mL) was added slowly. The pH of the solution was adjusted to 8.0 by adding NaHCO₃ saturated aqueous solution (300 mL), and the mixture was stirred at 0 °C for 15 min. The layers were separated, and the aqueous phase was extracted with CH₂Cl₂ (2x150 mL). All organic phases were combined, dried over anhydrous Na₂SO₄ and concentrated in vacuo. The residue (58.7 g) was purified by flash chromatography on silica gel (40:60:1-100:0:1 AcOEt/Hexane/Et₃N), followed by trituration with hexane, affording 33.87 g of the title compound (R*f*= 0.5 (10% MeOH/CH₂Cl₂), yellow solid, 74% yield).
¹H-NMR (CDCl₃, 250 MHz, δ): 7.26-7.12 (m, 5H, ArH); 7.00 (dd, J = 8.0 y 7.7 Hz, 1H, ArH); 6.60 (m, 2H, ArH); 3.82 (s, 2H, CH₂); 3.72 (s, 3H, CH₃); 2.88 (m, 2H, CH₂); 2.51 (m, 2H, CH₂); 1.99 (m, 1H, CH); 1.48 (m, 2H, CH₂).

### Chemical Example B

### N-benzyl-N-[(2S)-5-methoxy-1,2,3,4-tetrahydronaphthalen-2-yl]amine

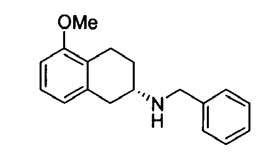

*N*-Benzyl-*N*-(5-methoxy-1,2,3,4-tetrahydronaphthalen-2-yl)amine (11.70 g, 43.76 mmol) was dissolved in Et₂O (350 mL). The reaction mixture was warmed up to reflux and (*S*)-(+)-mandelic acid (6.66 g, 43.76 mmol) was added. It was added CH₂Cl₂ (30 mL), and the mixture was refluxed for 3 h. The mixture was cooled at room temperature, and stirred for 16 h. The resulting solid was filtered, washed with Et₂O (3x20 mL) and dried to give 6.80 g of the diastereosiomeric salt as a white solid. The ee was evaluated in the compound (6S)-6-(dimethylamino)-5,6,7,8-tetrahydronaphthalen-1-ol (column chiralCel OD-H, 5 µm, 4.6 x 250 mm; flow rate: 1 ml/min; mobile phase: MeOH:EtOH (1:1)/hexanes 10:90). The diastereosiomeric salt was recrystallized from Et₂O to improve the ee. The salt was suspended in AcOEt (100 mL), and K₂CO₃ aqueous solution (20%, 80 mL) was added. The mixture was stirred at room temperature for 1 h and the layers were separated. The organic phase was dried over anhydrous Na₂SO₄ and concentrated in vacuo, affording 4.29 g of the title compound (R*f*= 0.5 (10% MeOH/CH₂Cl₂), pale yellow solid, 37% yield).
¹H-NMR (CDCl₃, 250 MHz, *δ*): 7.26-7.12 (m, 5H, ArH); 7.00 (dd, J = 8.0 y 7.7 Hz, 1H, ArH); 6.60 (m, 2H, ArH); 3.82 (s, 2H, CH₂); 3.72 (s, 3H, CH₃); 2.88 (m, 2H, CH₂); 2.51 (m, 2H, CH₂); 1.99 (m, 1H, CH); 1.48 (m, 2H, CH₂).

### Chemical Example C

### (6S)-6-[Benzyl(methyl)amino]-5,6,7,8-tetrahydronaphthalen-1-ol

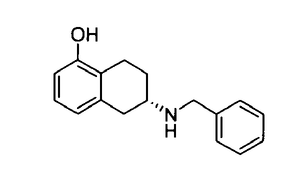

*N*-benzyl-*N*-[(2*S*)-5-methoxy-1,2,3,4-tetrahydronaphthalen-2-yl]amine (1.0 g, 3.74 mmol) was suspended in HBr aq. (48%, 70 mL), and the reaction mixture was refluxed for 4 h. The mixture was allowed to reach room temperature, and it was then cooled to -78°C. The pH of the solution was adjusted to 9.0 by adding NH₃ aqueous solution (25%) slowly. The mixture was allowed to reach room temperature, and was stirred for 30 min. The aqueous phase was extracted with CHCl₃ (3x100 mL). All organic phases were combined, dried over anhydrous Na₂SO₄ and concentrated in vacuo. The residue (1.0 g) was purified by flash chromatography on silica gel (10% MeOH/CH₂Cl₂), affording 0.86 g of the title compound (R*f*= 0.4 (10% MeOH/CH₂Cl₂), off-white solid, 91% yield).
¹H-NMR (CDCl₃, 250 MHz, δ): 9.13 (sa, 1H, OH); 7.38-7.17 (m, 5H, ArH); 6.86 (m, 1H, ArH); 6.54 (d, J = 8.0 Hz, 1H, ArH); 6.47 (d, J = 7.4 Hz, 1H, ArH); 3.80 (s, 2H, CH₂); 2.92-2.67 (m, 2H, CH₂); 2.41 (m, 2H, CH₂); 2.00 (m, 1H, CH); 1.45 (m, 2H, CH₂).

### Chemical Example D

### (6S)-6-[Benzyl(methyl)amino]-5,6,7,8-tetrahydronaphthalen-1-ol

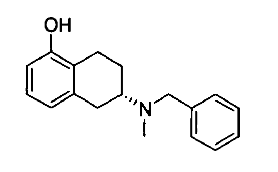

Formaldehyde (13.3 mL 37% aqueous solution, 13.3 mL, 177.62 mmol) and NaBH₄ (2.01 g, 53.29 mmol) were added in three portions (one portion every 20 min) to a 0 °C cooled solution of (6*S*)-6-[benzyl(methyl)amino]-5,6,7,8-tetrahydronaphthalen-1-ol (9.0 g, 35.52 mmol) in MeOH (400 mL).The reaction mixture was stirred at room temperature for 90 min, and solvent was concentrated off. The crude residue was diluted with H₂O (150 mL) and extracted with AcOEt (2x150 mL). The organic layers were dried over anhydrous Na₂SO₄, filtered and concentrated. The crude was flash chromatographed on silica gel (100% AcOEt) to afford 7.13 g of the title compound (R*f*= 0.6 AcOEt/Hexano/Et₃N (10:10:2), white solid, 75% yield).
¹H-NMR (CDCl₃, 300 MHz, δ): 7.42-7.26 (m, 5H, ArH); 7.00 (m, 1H, ArH); 6.71 (d, J = 7.6 Hz, 1H, ArH); 6.53 (d, J = 7.6 Hz, 1H, ArH); 3.74 (m, 2H, CH₂); 3.07-2.88 (m, 4H, CH₂); 2.60 (m, 1H, CH); 2.34 (s, 3H, CH₃); 2.26 (m, 1H, CH); 1.73 (m, 1H, CH).

### Chemical Example E

### (6S)-6-[Benzyl(methyl)amino]-5,6,7,8-tetrahydronaphthalen-1-yl trifluoromethanesulfonate

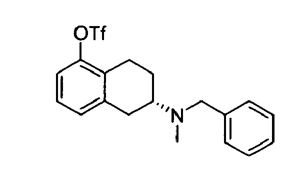

Tf₂O (3.6 mL, 21.309 mmol) was dropwise added to a -78 °C cooled solution of (6*S*)-6-[benzyl(methyl)amino]-5,6,7,8-tetrahydronaphthalen-1-ol (5.30 g, 19.822 mmol) and Et₃N (6 mL, 43.04 mmol) in CH₂Cl₂ (100 mL). Addition time: ca. 5 min. The reaction was completed after 15 min at low temperature (TLC analysis). The reaction mixture was poured into CH₂Cl₂ (250 mL) and washed with H₂O (200 mL). The organic layer was dried over anhydrous Na₂SO₄, filtered and concentrated. The crude residue was flash chromatographed on silica gel (1-2-3% MeOH/CH₂CI₂), to furnish two batches of product: 5.78 g of pure triflate, and 1.90 g of product slightly impure (TLC analysis) (R*f*= 0.8 (10% MeOH/CH₂CI₂), off-white solid, 73% and 24% yield).
¹H-NMR (CDCI₃, 250 MHz, δ): 7.27 - 6.96 (m, 8H, ArH); 3.59 (m, 2H, CH₂); 3.07 - 2.56 (m, 5H, CH₂); 2.20 (s, 3H, CH₃); 2.14 (m, 1H, CH); 1.64 (m, 1H, CH).

### Chemical Example F

### (2S)-Benzyl-methyl-[5-(1,3,5-trimethyl-1H-pyrazol-4-yl)-1,2,3,4-tetrahydro-naphthalen-2-yl]-amine

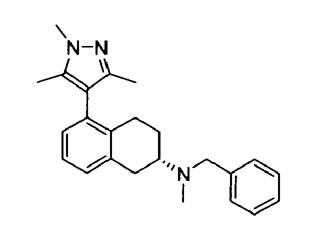

(*6S*)-6-[Benzyl(methyl)amino]-5,6,7,8-tetrahydronaphthalen-1-yl trifluoromethanesulfonate (5.0 g, 12.517 mmol), 1,3,5-trimethyl-1*H*-pyrazole-4-boronic acid pinacol ester (3.60 g, 15.246 mmol) and Pd(PPh₃)₄ (1.70 g, 1.471 mmol) were added to a solution of K₂CO₃ (3.31 g, 23.95 mmol) in a mixture of 1,2,-dimethoxyethane (120 mL) and H₂O (15 mL). The reaction mixture was purged with N₂ (g) for 10 min, and warmed up to reflux. The reaction was completed in 3 h (TLC analysis). It was allowed to reach room temperature, diluted with H₂O (150 mL) and extracted with AcOEt (300 mL). The organic layer was filtered through Celite (washing with AcOEt), dried over anhydrous Na₂SO₄, filtered and concentrated. The residue was purified by flash chromatography on silica gel (0-10-20% Et₃N/AcOEt) to afford the desired product as a brown-colored oil. The material was dissolved in CH₂Cl₂ (100 mL) and acidified with HCl aqueous solution (6 N). The organic layer was discarded, and the aqueous layer was taken to pH > 13 with NaOH aqueous solution (6 N). It was extracted with CH₂Cl₂ (3x200 mL), and the organic layer was dried over anhydrous Na₂SO₄, filtered and concentrated, to give 3.67 g of the coupling product (R*f*= 0.3 (AcOEt), pale yellow oil, 81% yield).
¹H-NMR (CDCl₃, 250 MHz, δ): 7.29 - 7.00 (m, 7H, ArH); 6.82 (m, 1H, ArH); 3.70 (d, *J* = 1.6 Hz, 3H, CH₃); 3.6 (d, *J* = 3.3 Hz, 2H, CH₂); 2.90 (m, 4H, CH₂); 2.60 - 2.24 (m, 2H, CH₂); 2.20 (d, *J* = 1.1 Hz, 3H, CH₃); 1.96 (d, *J* = 1.4 Hz, 6H, CH₃); 1.93 (d, *J* = 3.8 Hz, 6H, CH₃); 1.60 (m, 1H, CH). MS-EI+ *m*/*z:* 359.23

### Chemical Example G

### (2S)-Methyl-[5-(1,3,5-trimethyl-1H-pyrazol-4-yl)-1,2,3,4-tetrahydro-naphthalen-2-yl]-amine

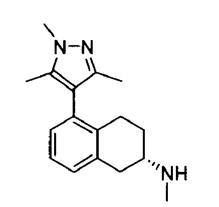

A solution of (2S)-benzy)-methy)-[5-(1,3,5-trimethyl-1H-pyrazol-4-yl)-1,2,3,4-tetrahydro-naphthalen-2-yl]-amine (3.70 g, 10.291 mmol) in THF (20 mL) was added to Pd/C (1.20 g, 10% weight of Pd on activated carbon), and MeOH (120 mL) was added to the suspension. The reaction mixture was stirred under H₂ overnight (ca. 16 h). It was filtered through Celite washing with AcOEt (2x100 mL) and the solvent was concentrated off to give 2.54 g of the methylamine as pale brown oil. The crude was purified by flash chromatography on silica gel (10-20-60% MeOH/CH₂Cl₂), to yield 2.05 g of the title product (R*f*= 0.3 (AcOEt/MeOH/Et₃N 20:3:2), pale yellow coloured oil, 74% yield).
¹H-NMR (CDCl₃, 250 MHz, δ): 7.11 (m, 2H, ArH); 6.92 (m, 1H, ArH); 3.77 (s, 3H, CH₃); 3.19 (m, 1H, CH); 3.08-2.81 (m, 2H, CH₂); 2.64-2.12 (m, 2H, CH₂); 2.60 (s, 3H, CH₃); 2.04 (m, 1 H, CH); 2.02 (s, 3H, CH₃); 2.00 (d, J = 2.2 Hz, 3H, CH₃); 1.55 (m, 1H, CH). MS-EI+ m/z: 269.19.

The other examples were then prepared as follows:

### Chemical Example H

### (2S)-Methyl-[5-(1,3,5-trimethyl-1H-pyrazol-4-yl)-1,2,3,4-tetrahydro-naphthalen-2-yl]-amine hydrochloride

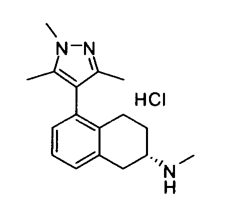

HCl (6.0 mL, 4 M solution in dioxane, 24.03 mmol) was dropwise added to a suspension of (*2S*)-methyl-[5-(1,3,5-trimethyl-1H-pyrazol-4-yl)-1,2,3,4-tetrahydro-naphthalen-2-yl]-amine (2.16 g, 8.01 mmol) in Et₂O (10 mL). The reaction mixture was stirred at room temperature for 2.5 h and then the solvent was concentrated off. The resulting solid was suspended in Et₂O (25 mL) and concentrated, in order to remove excess of HCl. This operation was done for three times, to give 2.42 g of the title product (R*f*= 0.3 (AcOEt/MeOH/Et₃N 20:3:2), white solid, 99% yield).
¹H-NMR (DMSO-d₆+D₂O, 250 MHz, δ): 9.31 (sa, 1H, NH); 7.19 (m, 2H, ArH); 6.95 (m, 1H, ArH); 3.80 (d, *J =* 1.4 Hz, 3H, CH₃); 3.41-3.21 (m, 2H, CH₂); 2.96 (m, 1H, CH); 2.59 (m, 3H, CH₃); 2.44 (m, 2H, CH₂); 2.14 (m, 1H, CH); 2.05-1.97 (m, 6H, CH₃); 1.71 (m, 1H, CH).

### Chemical Example I

### tert-Butyl benzyl [(2S)-5-hydroxy-1,2,3,4-tetrahydronaphthalen-2-yl]carbamate

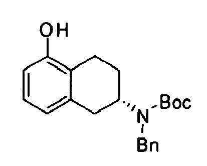

Boc₂O (0.380 g, 1.741 mmol) was added to a solution of (6*S*)-6-[benzyl(methyl)amino]-5,6,7,8-tetrahydronaphthalen-1-ol (Example C) (0.40 g, 1.578 mmol) and Et₃N (1.0 mL, 7.174 mmol) in CH₂CI₂ (20 mL). The reaction mixture was stirred at room temperature for 8 h, poured into CH₂CI₂ (200 mL) and washed with brine (1x100 mL). The organic layer was dried over anhydrous Na₂SO₄, filtered and concentrated. The crude residue was flash chromatographed on silica gel (5-10-20-30% AcOEt/Hexane) to give 0.44 g of the title product (R*f*= 0.8 (10% MeOH/CH₂Cl₂), white solid, 79% yield).
¹H-NMR (CDCl₃, 250 MHz, δ): 7.18 (m, 5H, ArH); 6.82 (m, 1H, ArH); 6.46 (d, J = 7.7 Hz, 2H, ArH); 4.35 (sa, 2H, CH₂); 3.71 (m, 4H, CH₂); 2.44 (m, 1H, CH); 1.82-1.69 (m, 2H, CH₂); 1.33 (sa, 9H, CH₃)

### Chemical Example J

### (6S)-6-[Benzyl(tert-butylcarbonyl)amino]-5,6,7,8-tetrahydronaphthalen-1-yl trifluoromethanesulfonate

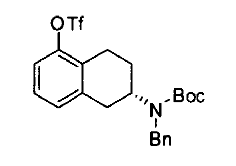

Tf₂O (0.270 mL, 1.598 mmol) was dropwise added to a -78 °C cooled solution of *tert*-butyl benzyl [(2S)-5-hydroxy-1,2,3,4-tetrahydronaphthalen-2-yl]carbamate (0.430 g, 1.217 mmol) and Et₃N (1.0 mL, 7.174 mmol) in CH₂Cl₂ (25 mL). The reaction was completed after 5 min at low temperature (TLC analysis). The reaction mixture was poured into H₂O (150 mL) and extracted with CH₂Cl₂ (1x150 mL). The organic layer was dried over anhydrous Na₂SO₄, filtered and concentrated. The crude residue was flash chromatographed on silica gel (10-20% AcOEt/Hexane) to furnish 0.508 g of the title product (R*f*= 0.8 (30% AcOEt/Hexane), colorless oil, 86% yield).
¹H-NMR (CDCl₃, 250 MHz, δ): 7.27 (m, 5H, ArH); 7.11 (m, 1H, ArH); 7.03 (m, 2H, ArH); 4.45 (sa, 2H, CH₂); 3.04-2.67 (m, 4H, CH₂); 1.97-1.70 (m, 3H, CH, CH₂); 1.44 (sa, 9H, CH₃)

### Chemical Example K

### N-Benzyl-N-tert-butylcarbonyl-N-[(2S)-5-(1,3,5-trimethyl-1H-pyrazol-4-yl)-1,2,3,4-tetrahydronaphthalen-2-yl]amine

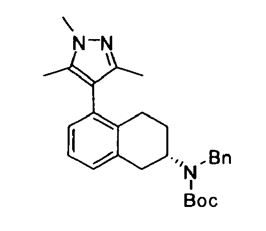

(*6S*)-6-[Benzyl(*tert*-butylcarbonyl)amino]-5,6,7,8-tetrahydronaphthalen-1-yl trifluoromethanesulfonate (0.5 g, 1.028 mmol), 1,3,5-trimethyl-1*H*-pyrazole-4-boronic acid pinacol ester (0.350 g, 1.482 mmol) and Pd(PPh₃)₄ (0.170 g, 0.147 mmol) were added to a solution of K₂CO₃ (0.30 g, 2.17 mmol) in a mixture of 1,2,-dimethoxyethane (30 mL) and H₂O (4 mL). The reaction mixture was purged with N₂ (g) for 10 min, and warmed up to reflux. The reaction was completed in 2 h. It was allowed to reach room temperature, diluted with AcOEt (300 mL) and filtered through Celite (washing with AcOEt). The organic layer was washed with brine (1x200 mL), dried over anhydrous Na₂SO₄, filtered and concentrated.
The residue was purified by flash chromatography on silica gel (80-100% AcOEt/Hexane) to afford 0.38 g of the coupling product (R*f*= 0.1 (30% AcOEt/Hexane), viscous yellow oil, 83% yield).
¹H-NMR (CDCl₃, 250 MHz, δ): 7.27 (m, 5H, ArH); 7.09 (d, J = 7.4 Hz, 1H, ArH); 7.01 (d, *J* = 7.4 Hz, 1H, ArH); 7.88 (m, 1 H, ArH); 4.44 (sa, 2H, CH₂); 3.75 (s, 3H, CH₃); 2.93 (m, 2H, CH₂); 2.45 (m, 2H, CH₂); 1.99 (m, 6H, CH₃); 1.77 (m, 1H, CH); 1.41 (sa, 9H, CH₃)

### Chemical Example L

### (6S)-6-(Benzylamine)-5,6,7,8-tetrahydronaphthalen-1-yl trifluoromethanesulfonate

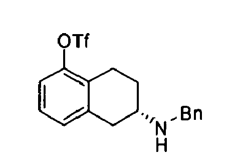

Tf₂O (0.620 mL, 3.734 mmol) was dropwise added to a -78 °C cooled solution of (6*S*)-6-[benzyl(methyl)amine]-5,6,7,8-tetrahydronaphthalen-1-ol (Example C) (0.860 g, 3.395 mmol) in CH₂Cl₂ (120 mL). The reaction was stirred at low temperature for 1.5 h. The reaction mixture was poured into H₂O (100 mL) and extracted with CH₂Cl₂ (2x50 mL). The organic layer was dried over anhydrous Na₂SO₄, filtered and concentrated. The crude residue was flash chromatographed on silica gel (1-5-10% MeOH/CH₂Cl₂), to furnish 0.279 g of triflate (R*f*= 0.8 (10% MeOH/CH₂Cl₂), orange colored oil, 21% yield).
¹H-NMR (CDCl₃, 250 MHz, δ): 7.28 -7.11 (m, 5H, ArH); 7.14-6.97 (m, 3H, ArH); 3.83 (s, 2H, CH₂); 2.98 (m, 2H, CH₂); 2.63 (m, 1H, CH); 2.10 (m, 1H, CH); 1.57 (m, 3H, CH₂, CH)

### Chemical Example M

### (2S)-Benzyl-[5-(1,3,5-trimethyl-1H-pyrazol-4-yl)-1,2,3,4-tetrahydro-naphthalen-2-yl]-amine

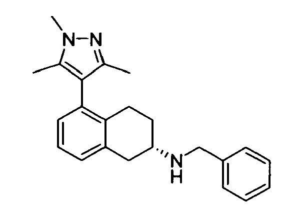

From (6*S*)-6-(benzylamine)-5,6,7,8-tetrahydronaphthalen-1-yl trifluoromethanesulfonate: Triflate, 1,3,5-trimethyl-1*H*-pyrazole-4-boronic acid pinacol ester (0.248 g, 1.051 mmol) and Pd(PPh₃)₄ (0.161 g, 0.140 mmol) were added to a solution of K₂CO₃ (0.194 g, 1.401 mmol) in a mixture of 1,2,-dimethoxyethane (30 mL) and H₂O (3 mL). The reaction mixture was purged with N₂ (g) for 10 min, and warmed up to reflux. The reaction was completed in 6 h. It was allowed to reach room temperature, diluted with H₂O (100 mL) and extracted with AcOEt (1x150 mL). The organic layer was filtered through Celite (washing with AcOEt), dried over anhydrous Na₂SO₄, filtered and concentrated. The residue was purified by flash chromatography on silica gel (0-5-10% MeOH/CH₂Cl₂) to afford 0.135 g of the coupling product (R*f*= 0.5 (10% MeOH/CH₂Cl₂), orange colored oil, 56% yield).

From *N*-benzyl-*N*-*tert*-butylcarbonyl-N-[(*2S*)-5-(1,3,5-trimethyl-1*H*-pyrazol-4-yl)-1,2,3,4-tetrahydronaphthalen-2-yl]amine: TFA (0.330 mL, 4.275 mmol) was added to a 0 °C cooled solution of starting material (0.381 g, 0.855 mmol) in CH₂Cl₂ (15 mL). The reaction was allowed to reach room temperature, and stirred at this temperature for 7 h. It was poured into H₂O (20 mL), extracted with CH₂Cl₂ (1x25 mL) and washed with NaOH aqueous solution (10%, 2x20 mL). The organic layer was dried over anhydrous Na₂SO₄, filtered and concentrated. The crude residue was flash chromatographed on silica gel (0-5-10% MeOH/CH₂Cl₂) to give 0.215 g of the title product (R*f*= 0.2 (10% MeOH/CH₂Cl₂), yellow oil, 73% yield).
¹H NMR (250 MHz, CHLOROFORM-*d*) δ ppm 1.50 (m, 2 H) 1.94 (d, J=3.29 Hz, 3 H) 1.97 (s, 3 H) 2.19 - 2.53 (m, 2 H) 2.66 (dd, *J*=15.09, 8.78 Hz, 1 H) 2.82 - 3.14 (m, 2 H) 3.70 (s, 3 H) 3.84 (s, 2 H) 6.84 (d, *J*=6.86 Hz, 1 H) 6.95 - 7.11 (m, 2 H) 7.20 - 7.31 (m, 5 H). MS-FAB+ m/z: 346.32 (M+1).

### Chemical Example N

### (2S)-5-(1,3,5-Trimethyl-1H-pyrazol-4-yl)-1 ,2,3,4-tetrahydronaphthalen-2-amine

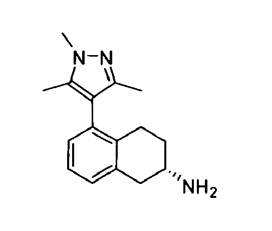

A solution of (2*S*)-benzy)-[5-(1,3,5-trimethyl-1H-pyrazo)-4-yl)-1,2,3,4-tetrahydro-naphthalen-2-yl]-amine (0.287 g, 0.832 mmol) in THF (4 mL) was added to Pd/C (0.10 g, 10% weight of Pd on activated carbon), and MeOH (10 mL) was added to the suspension. The reaction mixture was stirred under H₂ (g) atmosphere (balloon) for 21 h. It was filtered through Celite washing with AcOEt (2x10 mL), the solvent was concentrated off and the crude was purified by flash chromatography on silica gel (CH₂Cl₂/MeOH/NH₄OH 95:5:1), to yield 0.178 g of the title product (R*f*= 0.2 (AcOEt/MeOH/Et₃N 20:3:2), pale yellow colored oil, 84% yield).
¹H-NMR (CDCl₃, 250 MHz, δ): 7.10 (m, 2H, ArH); 6.92 (m, 1H, ArH); 3.77 (d, J= 1.4 Hz, 3H, CH₃); 3.11 (m, 2H, CH₂); 2.67-2.29 (m, 4H, CH₂); 1.93 (m, 6H, CH₃); 1.51 (m, 1H, CH).

### Chemical Example O

### (2S)-5-(1,3,5-Trimethyl-1H-pyrazol-4-yl)-1,2,3,4-tetrahydro-naphthalen-2-ylamine hydrochloride

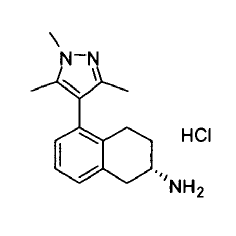

HCl (0.42 mL, 4 M solution in dioxane, 1.688 mmol) was dropwise added to a suspension of (*2S*)-5-(1,3,5-Trimethyl-1*H*-pyrazol-4-yl)-1,2,3,4-tetrahydronaphthalen-2-amine (0.143 g, 0.563 mmol) in Et₂O (5 mL). The reaction mixture was stirred at room temperature for 3 h and then the solvent was concentrated off. The resulting solid was suspended in Et₂O (10 mL) and concentrated, in order to remove excess of HCl. This operation was repeated for three times, to give 0.150 g of the title product (R*f*= 0.2 (AcOEt/MeOH/Et₃N 20:3:2), white solid, 91% yield).
¹H NMR (250 MHz, DMSO-*d*₆) δ ppm 1.56 - 1.76 (m, 1 H) 1.94 (d, *J*=5.49 Hz, 3 H) 2.01 (d, J=6.59 Hz, 3 H) 2.07 (m, 1 H) 2.45 (m, 2 H) 2.85 (dd, *J*=16.05, 10.29 Hz, 1 H) 3.15 (dd, *J*=16.05, 4.53 Hz, 1 H) 3.41 (s, 1 H) 3.74 (s, 3 H) 6.92 (d, *J*=7.14 Hz, 1 H) 7.03 - 7.26 (m, 2 H) 8.21 (br, 2 H). MS-FAB+ *m*/*z*: 256.06 (M+1-HCl).

### 3rd Group of Chemical Examples:

### Chemical Example A

### N-(5-Methoxy-1,2,3,4-tetrahydronaphthalen-2-yl)-N,N-dimethylamine

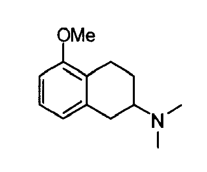

To a solution of 5-methoxy-2-tetralone (10.33 g, 58.62 mmol) dissolved in CH₂Cl₂ (400 mL) were added dimethylamine (5.6 M in EtOH, 14 mL, 76.206 mmol) and AcOH (0.46 mL, 5.862 mmol), and the mixture was stirred for 4 h at room temperature. It was then cooled to 0 °C and NaB(OAc)₃H (0.45 eq, 5.59 g, 26.379 mmol) was added over a period of 20 min. After 1 h stirring at 0 °C, NaB(OAc)₃H (1.0 eq, 12.42 g, 58.62 mmol) was added over a period of 30 min. The reaction mixture was warmed to room temperature and stirred for 16 h. The mixture was cooled again to 0 °C, and H₂O (250 mL) was added slowly. The pH of the solution was adjusted to 8.0 by adding NaHCO₃ saturated aqueous solution, and the mixture was stirred at 0 °C for 15 min. The layers were separated, and the aqueous phase was extracted with CH₂Cl₂ (4x100 mL). All organic phases were combined, dried over anhydrous Na₂SO₄ and concentrated in vacuo. The residue (12.51 g) was purified by flash chromatography on silica gel (1:100:1-100:0:1 AcOEt/Hexane/Et₃N) affording 8.86 g of the title compound (R*f*= 0.5 (AcOEt/Hexane/Et₃N 10:10:2), brown colored oil, 74% yield).
¹H-NMR (CDCl₃, 250 MHz, δ): 7.09 (m, 1H, ArH); 6.72 (d, J = 7.4 Hz, 1H, ArH); 6.65 (d, J = 8.0 Hz, 1H, ArH); 3.81 (s, 3H, CH₃); 2.90-3.04 (m, 2H, CH₂); 2.70-2.81 (m, 1 H, CH); 2.47-2.64 (m, 2H, CH₂); 2.37 (m, 6H, CH₃); 2.10-2.30 (m, 1H, CH); 1.48-1.64 (m, 1H, CH)

### Chemical Example B

### 6-(Dimethylamino)-5,6,7,8-tetrahydronaphthalen-1-ol

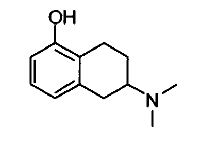

*N*-(5-Methoxy-1,2,3,4-tetrahydronaphthalen-2-yl)-*N,N*-dimethylamine (8.86 g, 43.156 mmol) was dissolved in CH₂Cl₂ (200 mL), cooled to 0 °C and BBr₃ (1.0 M in CH₂Cl₂, 51.8 mL, 51.788 mmol) was added over a period of 20 min. The reaction mixture was allowed to reach r.t. while stirring overnight (ca. 14 h). The mixture was cooled again to 0 °C, NH₃ aq. (25%, 50 mL) was added slowly and the mixture was stirred at 0 °C for 15 min. The salts were filtered off, layers were separated and the aqueous phase was extracted with CH₂Cl₂ (4x40 mL). All organic phases were combined, dried over anhydrous Na₂SO₄ and concentrated in vacuo. The residue (6.99 g) was purified by flash chromatography on silica gel (30:70:2-100:0:2 AcOEt/Hexane/Et₃N and 30:70:2 AcOEt/Hexane/Et₃N-90:10:2 AcOEt/MeOH/Et₃N) affording 2.80 g of the title compound (R*f*= 0.3 (AcOEt/Hexane/Et₃N 10:10:2), off-white solid, 34% yield).
¹H-NMR (CDCl₃, 250 MHz, δ): 6.99 (m, 1 H, ArH); 6.68 (d, J= 7.6 Hz, 1H, ArH); 6.58 (d, J= 7.6 Hz, 1H, ArH); 2.76 (m, 2H, CH₂); 2.58 (m, 2H, CH₂); 2.39 (s, 6H, CH₃); 2.17 (m, 2H, CH₂); 1.61 (m, 1H, CH).

### Chemical Example C

### N-Benzyl-N-(5-methoxy-1,2,3,4-tetrahydronaphthalen-2-yl)amine

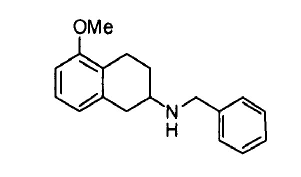

To a solution of 5-methoxy-2-tetralone (30 g, 170.24 mmol) dissolved in CH₂Cl₂ (250 mL) were added benzylamine (23 mL, 212.80 mmol) and AcOH (0.97 mL, 17.02 mmol), and the mixture was stirred for 4 h at room temperature. It was then cooled to 0 °C and NaB(OAc)₃H (0.38 eq, 13.71 g, 64.69 mmol) was added over a period of 20 min. After 1 h stirring at 0 °C, NaB(OAc)₃H (1.07 eq, 38.61 g, 182.16 mmol) was added over a period of 30 min. It was added CH₂Cl₂ (100 mL), the reaction mixture warmed to room temperature and stirred for 15 h. The mixture was cooled again to 0°C, and H₂O (200 mL) was added slowly. The pH of the solution was adjusted to 8.0 by adding NaHCO₃ saturated aqueous solution (300 mL), and the mixture was stirred at 0 °C for 15 min. The layers were separated, and the aqueous phase was extracted with CH₂Cl₂ (2x150 mL). All organic phases were combined, dried over anhydrous Na₂SO₄ and concentrated in vacuo. The residue (58.7 g) was purified by flash chromatography on silica gel (40:60:1-100:0:1 AcOEt/Hexane/Et₃N), followed by trituration with hexane, affording 33.87 g of the title compound (R*f*= 0.5 (10% MeOH/CH₂Cl₂), yellow solid, 74% yield).
¹H-NMR (CDCl₃, 250 MHz, δ): 7.26-7.12 (m, 5H, ArH); 7.00 (dd, J = 8.0 y 7.7 Hz, 1 H, ArH); 6.60 (m, 2H, ArH); 3.82 (s, 2H, CH₂); 3.72 (s, 3H, CH₃); 2.88 (m, 2H, CH₂); 2.51 (m, 2H, CH₂); 1.99 (m, 1H, CH); 1.48 (m, 2H, CH₂).

### Chemical Example D

### N-Benzyl-N-[(2R)-5-methoxy-1, 2,3,4-tetrahydronaphthalen-2-yl]amine

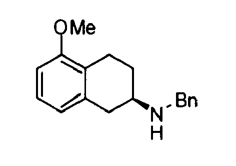

N-Benzyl-N-(5-methoxy-1,2,3,4-tetrahydronaphthalen-2-yl)amine (25.0 g, 93.507 mmol) was dissolved in Et₂O (800 mL). The reaction mixture was warmed up to reflux and (*R*)-(-)-mandelic acid (14.23 g, 93.507 mmol) was added. It was added CH₂Cl₂ (360 mL), and the mixture was refluxed for 2 h. The mixture was then cooled at room temperature and stirred for 15 h. The resulting solid was filtered, washed with Et₂O (3x40 mL) and dried to give 9.34 g of the diastereosiomeric salt as a white solid. The diastereosiomeric salt was recrystallized from Et₂O to improve the ee. The salt was suspended in AcOEt (300 mL), and K₂CO₃ aqueous solution (20%, 100 mL) was added. The mixture was stirred at room temperature for 2.5 h and the layers were separated. The organic phase was dried over anhydrous Na₂SO₄ and concentrated in vacuo, affording 6.0 g of the title compound (R*f*= 0.5 (10% MeOH/CH₂Cl₂), white solid, 24% yield).
¹H-NMR (CDCl₃, 250 MHz, δ): 7.26-7.12 (m, 5H, ArH); 7.00 (dd, J = 8.0 y 7.7 Hz, 1H, ArH); 6.60 (m, 2H, ArH); 3.82 (s, 2H, CH₂); 3.72 (s, 3H, CH₃); 2.88 (m, 2H, CH₂); 2.51 (m, 2H, CH₂); 1.99 (m, 1H, CH); 1.48 (m, 2H, CH₂)

### Chemical Example E

### (6R)-6-[Benzyl(methyl)amino]-5,6,7,8-tetrahydronaphthalen-1-ol

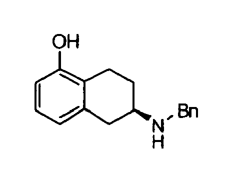

*N*-Benzyl-*N*-[(2*R*)-5-methoxy-1,2,3,4-tetrahydronaphthalen-2-yl]amine (10.0 g, 37.40 mmol) was suspended in HBr aq. (48%, 250 mL), and the reaction mixture was refluxed for 4 h. The mixture was allowed to reach room temperature, and it was then cooled to -78 °C. The pH of the solution was adjusted to 9.0 by adding NH₃ aqueous solution (25%). The mixture was allowed to reach room temperature, and was stirred for 30 min. The aqueous phase was extracted with AcOEt (2x300 mL). All organic phases were combined, dried over anhydrous Na₂SO₄ and concentrated in vacuo to afford 9.4 g of the title compound (R*f*= 0.4 (10% MeOH/CH₂Cl₂), white solid, 99% yield).
¹H-NMR (CDCl₃, 250 MHz, δ): 9.13 (sa, 1H, OH); 7.38-7.17 (m, 5H, ArH); 6.86 (m, 1H, ArH); 6.54 (d, J = 8.0 Hz, 1H, ArH); 6.47 (d, J = 7.4 Hz, 1H, ArH); 3.80 (s, 2H, CH₂); 2.92-2.67 (m, 2H, CH₂); 2.41 (m, 2H, CH₂); 2.00 (m, 1H, CH); 1.45 (m, 2H, CH₂).

### Chemical Example F

### (6R)-6-Amino-5,6,7,8-tetrahydronaphthalen-1-ol

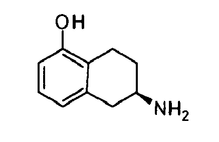

A solution of (6*R*)-6-[Benzyl(methyl)amino]-5,6,7,8-tetrahydronaphthalen-1-ol (4.80 g, 18.946 mmol) in THF (150 mL) was added to Pd/C (1.90 g, 10% weight of Pd on activated carbon), and the reaction mixture was stirred under H₂ (g) atmosphere for 16 h. It was filtered through Celite washing with AcOEt (1x200 mL) and the solvent was concentrated off to afford 3.0 g of the title product (R*f*= 0.1 (20% MeOH/CH₂Cl₂), off-white solid, 97% yield).
¹H-NMR (CDCl₃, 250 MHz, δ): 6.99 (m, 1 H, ArH); 6.61 (dd, J = 7.4, 8.2 Hz, 1H, ArH); 3.18 (m, 1H, CH); 3.02-2.82 (m, 2H, CH₂); 2.70-2.52 (m, 2H, CH₂); 2.05 (m, 1H, CH); 1.24 (m, 1H, CH)

### Chemical Example G

### (6R)-6-(Dimethylamino)-5,6,7,8-tetrahydronaphthalen-1-ol

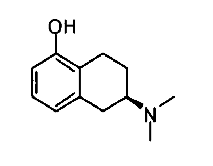

Formaldehyde (10 mL 37% aqueous solution, 133.481 mmol) was added to a solution of (6*R*)-6-Amino-5,6,7,8-tetrahydronaphthalen-1-ol (3.0 g, 18.38 mmol) in MeOH (100 mL) and THF (15 mL). The reaction mixture was stirred at room temperature for 15 min, and NaBH₄ (2.0 g, 52.868 mmol) was added. The reaction mixture was stirred at room temperature for 2 h, poured into H₂O (300 mL) and extracted with CH₂Cl₂ (2x300 mL). The organic layer was dried over anhydrous Na₂SO₄, filtered and concentrated. The product was purified by flash chromatography on silica gel (0-5-10-15-20% Et₃N/AcOEt) to furnish 2.74 g of the title product (R*f*= 0.5 (20% Et₃N/AcOEt), off-white solid, 78% yield).
¹H-NMR (CDCl₃, 250 MHz, δ): 6.99 (m, 1 H, ArH); 6.68 (d, J= 7.6 Hz, 1 H, ArH); 6.58 (d, J= 7.6 Hz, 1H, ArH); 2.76 (m, 2H, CH₂); 2.58 (m, 2H, CH₂); 2.39 (s, 6H, CH₃); 2.17 (m, 2H, CH₂); 1.61 (m, 1 H, CH)

### Chemical Example H

### (6R)-6-(Dimethylamino)-5,6,7,8-tetrahydronaphthalen-1-yl trifluoromethanesulfonate

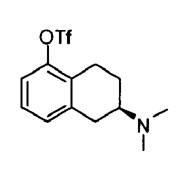

Tf₂O (4.3 mL, 25.452 mmol) was dropwise added to a -78 °C cooled solution of (6R)-6-(dimethylamino)-5,6,7,8-tetrahydronaphthalen-1-ol (4.25 g, 22.218 mmol) and Et₃N (8.0 mL, 57.396 mmol) in CH₂Cl₂ (90 mL). The reaction was completed after 5 min at low temperature. The reaction mixture was poured into brine (200 mL) and extracted with CH₂Cl₂ (2x200 mL). The organic layer was dried over anhydrous Na₂SO₄, filtered and concentrated. The crude residue was flash chromatographed on silica gel (5-10% MeOH/CH₂Cl₂) to furnish 5.10 g of the title product (R*f*= 0.4 (10% MeOH/CH₂Cl₂), pale brown colored oil, 71% yield).
¹H-NMR (CDCl₃, 250 MHz, δ): 7.14 (m, 3H, ArH); 3.02 (m, 2H, CH₂); 2.79 (m, 2H, CH₂); 2.60 (m, 1H, CH); 2.38 (s, 6H, CH₃); 2.18 (m, 1H, CH); 1.62 (m, 1H, CH).

### Chemical Example I

### (2R)-Dimethyl-[5-(1,3,5-trimethyl-1H-pyrazol-4-yl)-1,2,3,4-tetrahydro-naphthalen-2-yl]-amine

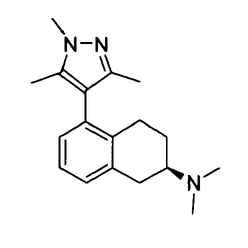

(6*R*)-6-(Dimethylamino)-5,6,7,8-tetrahydronaphthalen-1-yl trifluoromethanesulfonate (2.5 g, 7.731 mmol), 1,3,5-trimethyl-1H-pyrazole-4-boronic acid pinacol ester (2.10 g, 8.893 mmol) and Pd(PPh₃)₄ (1.2 g, 1.038 mmol) were added to a solution of K₂CO₃ (2.15 g, 15.556 mmol) in a mixture of 1,2-dimethoxyethane (60 mL) and H₂O (8 mL). The reaction mixture was purged with N₂ (g) for 10 min, and warmed up to reflux. The reaction was completed in 2 h. It was allowed to reach room temperature, diluted with H₂O (200 mL) and extracted with AcOEt (1x400 mL). The organic layer was dried over anhydrous Na₂SO₄, filtered and concentrated. The residue was purified by flash chromatography on silica gel (0-10-20% Et₃N/AcOEt) to afford the desired product as a brown-colored oil. The material was dissolved in CH₂Cl₂ (200 mL) and acidified with HCl aqueous solution (6 N). The organic layer was discarded, and the aqueous layer was taken to pH > 13 with NaOH aqueous solution (6 N). It was extracted with CH₂Cl₂ (3x300 mL), and the organic layer was dried over anhydrous Na₂SO₄, filtered and concentrated, to give 1.45 g of the coupling product (R*f*= 0.2 (10% Et₃N/AcOEt), colorless oil, 66% yield).
¹H-NMR (CDCl₃, 250 MHz, δ): 7.16-7.06 (m, 2H, ArH); 6.90 (m, 1H, ArH); 3.77 (d, 3H, J = 1.4 Hz, CH₃); 3.02 (m, 1 H, CH); 2.83 (m, 1 H, CH); 2.68-2.28 (m, 4H, CH₂); 2.37 (s, 6H, CH₃); 2.04 (s, 3H, CH₃); 2.00 (d, J = 2.7 Hz, 3H, CH₃); 1.51 (m, 1H, CH)

### Chemical Example J

### (2R)-Dimethyl-[5-(1,3,5-trimethyl-1H-pyrazol-4-yl)-1,2,3,4-tetrahydro-naphthalen-2-yl]-amine hydrochloride

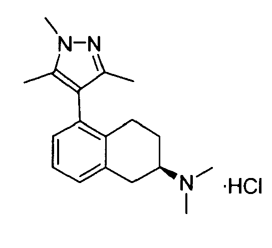

HCl (9.2 mL, 4 M solution in dioxane, 18.51 mmol) was dropwise added to a suspension of (2*R*)-dimethyl-[5-(1,3,5-trimethyl-1H-pyrazol-4-yl)-1,2,3,4-tetrahydro-naphthalen-2-yl]-amine (1.75 g, 6.17 mmol) in Et₂O (10 mL). The reaction mixture was stirred at room temperature for 3 h and then the solvent was concentrated off. The resulting solid was suspended in Et₂O (25 mL) and concentrated, in order to remove excess of HCl. This operation was done for three times, to give 1.95 g of the title product (R*f*= 0.2 (10% Et₃N/AcOEt), white solid, 99% yield).
¹H NMR (250 MHz, DMSO-*d*₆) δ ppm 1.74 (m, 1 H) 2.01 (d, J= 5.08 Hz, 3 H) 2.05 (d, J= 5.08 Hz, 3 H) 2.24 (d, *J*=12.08 Hz, 1 H) 2.44 (m, 2 H) 2.74 (s, 3 H) 2.76 (s, 3 H) 3.00 - 3.31 (m, 2 H) 3.58 (br. s., 1 H) 3.84 (s, 3 H) 6.96 (t, *J*=5.49 Hz, 1 H) 7.10 - 7.28 (m, 2 H). MS-EL+ m/z: 283.21 (M-HCl)

Other examples were or are prepared according to the reaction schemes and descriptions given above and are given in the following table:

| **Chemical Example** | **Structure** | **Name** | **¹H-NMR** |
|---|---|---|---|
| J | 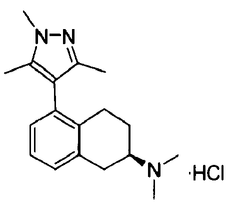 | (2*R*)-Dimethyl-[5-(1,3,5-trimethyl-1H-pyrazol-4-yl)-1,2,3,4-tetrahydro- naphthalen-2-yl]-amine hydrochloride | ¹H NMR (250 MHz, DMSO-*d*₆) δ ppm 1.74 (m, 1 H) 2.01 (d, *J*= 5.08 Hz, 3 H) 2.05 (d, *J*= 5.08 Hz, 3 H) 2.24 (d, *J*=12.08 Hz, 1 H) 2.44 (m, 2 H) 2.74 (s, 3 H) 2.76 (s, 3 H) 3.00 - 3.31 (m, 2 H) 3.58 (br. s., 1 H) 3.84 (s, 3 H) 6.96 (t,*J*=5.49 Hz, 1 H) 7.10, 7.28 (m,2 |
| K | 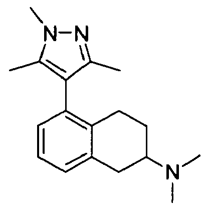 | Dimethyl-[5-(1,3,5-trimethyl-1 H-pyrazol-4-yl)-1,2,3,4-tetrahydronaphthalen-2-yl]-amine | |
| L | 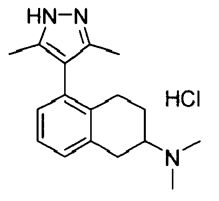 | [5-(3,5-Dimethyl-1H-pyrazol-4-yl)-1,2,3,4-tetrahydro-naphthalen-2-yl]-dimethyl-amine | |
| M | 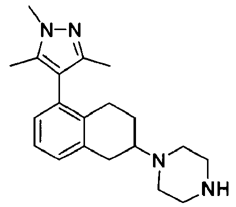 | 1-[5-(1,3,5-Trimethyl-1H-pyrazol-4-yl)-1,2,3,4-tetrahydro-naphthalen-2-yl]-piperazine | |
| N | 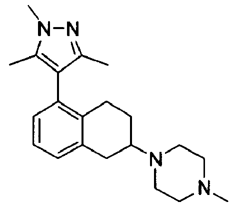 | 1-Methyl-4-[5-(1,3,5-trimethyl-1 H-pyrazol-4-yl)-1,2,3,4-tetrahydro-naphthalen-2-yl]-piperazine | |
| O | 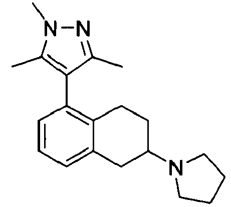 | 1,3,5-Trimethyl-4-(6-pyrrolidin-1-yl-5,6,7,8-tetrahydro-naphthalen-1-yl)-1H-pyrazole | |
| P | 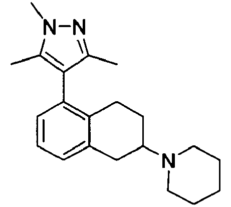 | 1-[5-(1,3,5-Trimethyl-1H-pyrazol-4-yl)-1,2,3,4-tetrahydro-naphthalen-2-yl]-piperidine | |
| Q | 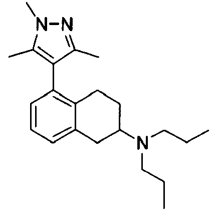 | Dipropyl-[5-(1,3,5-trimethyl-1 H-pyrazol-4-yl)-1,2,3,4-tetrahydronaphthalen-2-yl]-amine | |
| R | 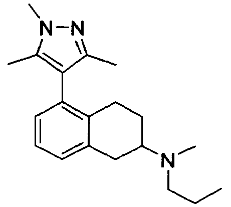 | Methyl-propyl-[5-(1,3,5-trimethyl-1 H-pyrazol-4-yl)-1,2,3,4-tetrahydronaphthalen-2-yl]-amine | |
| S | 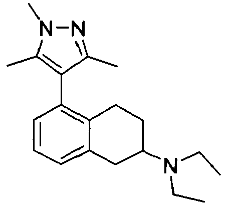 | Diethyl-[5-(1,3,5-trimethyl-1 H-pyrazol-4-yl)-1,2,3,4-tetrahydronaphthalen-2-yl]-amine | |
| T | 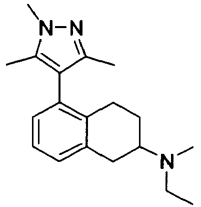 | Ethyl-methyl-[5-(1,3,5-trimethyl-1 H-pyrazol-4-yl)-1,2,3,4-tetrahydronaphthalen-2-yl]-amine | |
| U | 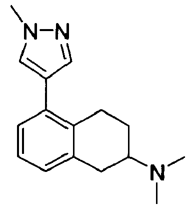 | Dimethyl-[5-(1-methyl-1 H-pyrazol-4-yl)-1,2,3,4-tetra hyd ro-na phtha len-2-yl]-amine | |
| V | 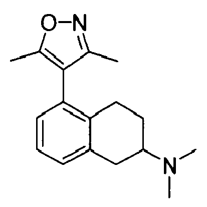 | [5-(3,5-Dimethyl-isoxazol-4-yl)-1,2,3,4-tetrahydro-naphthalen-2-yl]-dimethyl-amine | |
| W | 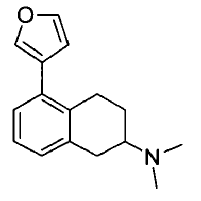 | (5-Furan-3-yl-1,2,3,4-tetra hydro-naphthalen-2-yl)-dimethyl-amine | |
| X | 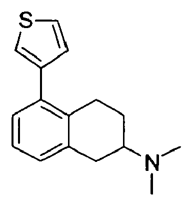 | Dimethyl-(5-thiophen-3-yl-1,2,3,4-tetrahydronaphthaten-2-yl)-amine | |
| Y | 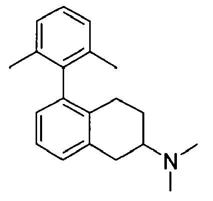 | [5-(2,6-Dimethyl-phenyly 1,2,3,4-tetrahydronaphthalen-2-yl]-dimethyl-amine | |
| Z | 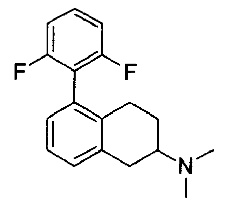 | [5-(2,6-Difluoro-phenyl)-1,2,3,4-tetrahydronaphthalen-2-yl]-dimethyl-amine | |
| AA | 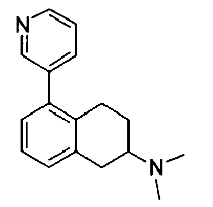 | Dimethyl-(5-pyridin-3-yl-1,2,3,4-tetrahydronaphthalen-2-yl)-amine | |

### PHARMACOLOGICAL EXAMPLES:

### P-Example 1: In-vitro binding to 5HT7-Receptor:

### AS-19:

The Ki-value of AS-19 for the 5HT7-recptor from literature is 0.6 nM. Other Ki values for other 5HT-receptors were:
- 89.7 nM for human 5HT1A,
- 48.1 nM for rat 5-HT1 (non-selective)
- 98.5 nM for human 5-HT5A
and no significant binding for:
human 5HT2B, 5HT2c, 5HT2(non-selective), 5HT3 or 5HT6 or guinea pig 5HT4.

### Chemical Example Group 1:

### Chemical example 1:

The results for chemical example 1 can be seen in table 1:

**Table 1:**

| **5-HT₇ IC50 (nM)** | **5-HT₇ EC50 (nM)** | **5-HT₇ Emax (%)** | **5-HT₆ inhib. (10⁻⁷M) (%)** | **5-HT₁ IC50 (nM)** |
|---|---|---|---|---|
| 6.1 ±0.7 | 25.5 ±2 | 94% | 5.4% | > 1000 |

### Chemical Example Group 2:

| **Chemical EXAMPLE** | **5-HT₇ IC₅₀ (nM)** | **5-HT₇ %-Inhib. (10⁻⁶ M)** | **5-HT₇ %-Inhib. (10⁻⁷ M)** | **5-HT₇ %-Inhib. (10⁻⁸ M)** |
|---|---|---|---|---|
| H | 2.3 | 98.1 | 97.8 | 86.1 |
| F | | 109.9 | 44.7 | 16.3 |

### Chemical Example Group 3:

| **Chemical EXAMPLE** | **5-HT₇ IC₅₀ (nM)** | **5-HT₇ %-Inhib. (10⁻⁶ M)** | **5-HT₇ %-Inhib. (10⁻⁷ M)** | **5-HT₇ %-Inhib. (10⁻⁸ M)** |
|---|---|---|---|---|
| J | | 59.6 | 9.6 | -1.8 |
| K | 3.9 | | 91.3 | 38.8 |

### P-Example 2: Hot-Plate-Test (Combination of Morphine + AS-19):

Male CD1 Mice were receiving s.c. injections of the test substances/combinations A to F.
A = saline (vehicle/baseline)
B = AS-19, [10 mg/kg]
C = morphine [2 mg/kg]
D = morphine [2 mg/kg] + AS-19 [10 mg/kg]
E = morphine [8 mg/kg]
F = morphine [8 mg/kg] + AS-19 [10 mg/kg]
The mice were placed 30 minutes after injection on the hot plate heated to 50° C. The signs of pain shown were divided by Front Paw Licking (FPL), Hind Paw Licking (HPL) and Jumping and measured/counted. The Baseline was determined based on saline injection. Significance values were determined vs. the saline group and vs. the respective morphine group.
The results are shown in Fig. 1A), Fig. 1B) and Fig. 2). The Combination according to the invention did show a highly super-additive effect in all three signs of pain.

### P-Example 3: Tail-Flick-Test (Combination of Morphine + AS-19):

Male CD1 Mice were receiving s.c. injections of the test substances/combinations A to F.
A = saline (vehicle/baseline)
B = AS-19, [10 mg/kg]
C = morphine [2 mg/kg]
D = morphine [2 mg/kg] + AS-19 [10 mg/kg]
E = morphine [8 mg/kg]
F = morphine [8 mg/kg] + AS-19 [10 mg/kg]
The mice were placed 45 minutes after injection in the tail-flick apparatus. The Baseline was determined based on saline injection. Significance values were determined vs. the saline group and vs. the respective morphine group.
The results are shown in Fig. 3). The Combination according to the invention did show a highly super-additive effect.

### P-Example 4: Hot-Plate-Test (Combination of Morphine + Chemical Example 1):

Male CD1 Mice were receiving s.c. injections of the test substances/combinations including
- saline (vehicle/baseline)
- Example 1, Dimethyl-{2-[3-(1,3,5-trimethyl-1H-pyrazol-4-yl)-phenyl]-ethyl)-amine [10 mg/kg]
- morphine [2 mg/kg]
- morphine [2 mg/kg] + Example 1, Dimethyl-{2-[3-(1,3,5-trimethyl-1H-pyrazol-4-yl)-phenyl]-ethyl}-amine [10 mg/kg],
- morphine of varying concentrations of 1, 2, 4, 8 or 16 mg/kg
- morphine of varying concentrations of 1, 2, 4, 8 or 16 mg/kg + Example 1, Dimethyl-{2-[3-(1,3,5-trimethyl-1H-pyrazol-4-yl)-phenyl]-ethyl}-amine [10 mg/kg].

The mice were placed 30 minutes after injection on the hot plate heated to 50° C. The signs of pain shown were divided by Front Paw Licking (FPL), Hind Paw Licking (HPL) and Jumping and measured/counted. The Baseline was determined based on saline injection. Significance values were determined vs. the saline group and vs. the respective morphine group.
The results are shown in Fig. 4), Fig. 5), Fig. 6), Fig. 7), and Fig. 8). In Fig. 4) the Combination according to the invention did show a clearly super-additive effect in jumping in fixed dose of morphine. Figures 5) to 8) taken together did in all three signs of pain show a highly pronounced lowering of the ED50 values due to the combination compared to morphine alone, whereas especially for FPL only the combination was able to show any ED50 at all.

### P-Example 5: Hot-Plate-Test (Combination of Morphine + Chemical Example 1), after tolerance development:

Male CD1 Mice were devided in 2 groups, the group receiving s.c. injections of morphine in high doses to develop tolerance (hereinafter called Tolerants), and the group receiving only vehicle (callede Non-Tolerants). The following treatment scheme was followed:

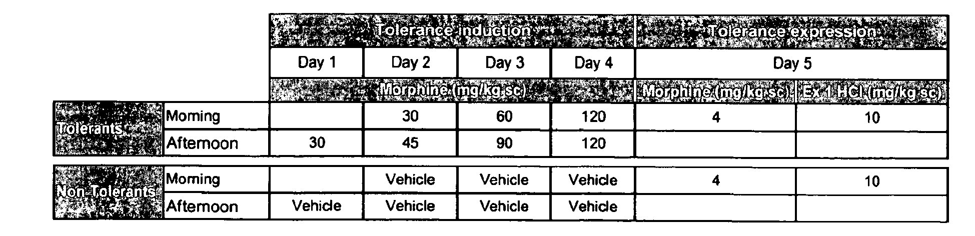

According to past experience which was later proven in the experiments after 4 days of treatment the Tolerants group were expressing tolerance towards morphine both groups Tolerants and Non-Tolerants were tested on day 5.

Male CD1 Mice (Tolerants and Non-Tolerants) in two groups each (N=10) were receiving s.c. injections of morphine [4 mg/kg] or a test combination of 4 mg/kg morphine and 10 mg/kg of Chemical Example 1, Dimethy)-{2-[3-(1,3,5-trimethy)-1H-pyrazo)-4-yl)-phenyl]-ethyl}-amine.

The mice were placed 30 minutes after injection on the hot plate heated to 50° C. The signs of pain shown were divided by Front Paw Licking (FPL), Hind Paw Licking (HPL) and Jumping and measured/counted. The Baseline was determined based on saline injection. Significance values were determined vs. respective "non tolerant" group, the "Morphine-tolerant group" or the "Morphine-non tolerant group" with *** p<0.001 vs. respective "non tolerant" group, # # # p<0.001 vs. "Morphine-tolerant group" and α α α p<0.001 *vs.* "Morphine-non tolerant group".

The results are shown in Fig. 9A and 9B), and Fig. 10). In all Figures the analgesic effect of morphine alone was reduced in all tolerant groups. In Fig. 9A) and Fig 9B) the Combination according to the invention did show no effect on the tolerant group compared to the group treated only with morphine, but again an effect in FPL and even a clearly super-additive effect in HPL in the Non-Tolerant group. In Fig. 10) in Jump the Combination according to the invention did show a clear and significant effect on both the Tolerant and the Non-Tolerant Group compared to the respective groups treated only with morphine.

### P-Example 6: Tail-Flick-Test (Combination of Morphine + Chemical Example 1), after tolerance development:

Male CD1 Mice were devided in 2 groups, the group receiving s.c. injections of morphine in high doses to develop tolerance (hereinafter called Tolerants), and the group receiving only vehicle (callede Non-Tolerants). The following treatment scheme was followed:

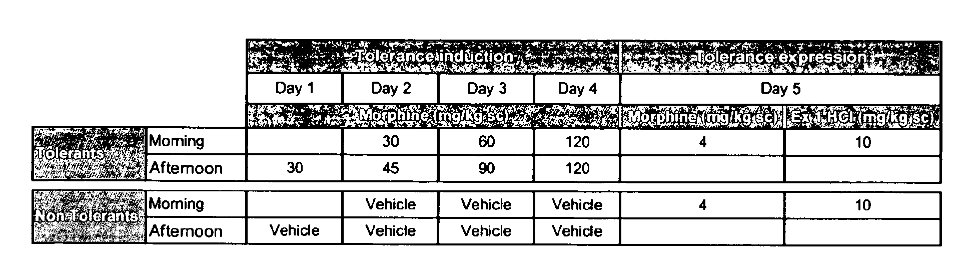

According to past experience which was later proven in the experiments after 4 days of treatment the Tolerants group were expressing tolerance towards morphine both groups Tolerants and Non-Tolerants were tested on day 5.

Male CD1 Mice (Tolerants and Non-Tolerants) in two groups each (N=10) were receiving s.c. injections of morphine [4 mg/kg] or a test combination of 4 mg/kg morphine and 10 mg/kg of Chemical Example 1, Dimethyl-{2-[3-(1,3,5-trimethyl-1H-pyrazol-4-yl)-phenyl]-ethyl}-amine.

The mice were placed 45 minutes after injection in the tail-flick apparatus. The Baseline was determined based on saline injection. Significance values were determined vs. respective "non tolerant" group, the "Morphine-tolerant group" or the "Morphine-non tolerant group" with *** p<0.001 vs. respective "non tolerant" group, # # # p<0.001 *vs.* "Morphine-tolerant group" and α α α p<0.001 vs. "Morphine-non tolerant group".

The results are shown in Fig. 11). The analgesic effect of morphine alone was significantly reduced in the tolerant group. The Combination according to the invention did show a clear and significant effect on both the Tolerant and the Non-Tolerant Group compared to the respective groups treated only with morphine.

## Claims

1. A combination of compounds comprising
c) at least one compound A selected from compounds binding to the 5-HT7 receptor;
d) at least one compound B selected from compounds binding to the µ-opoid receptor.

2. Combination according to claim 1, in which the compound A is acting as an agonist, preferably a full or partial agonist on the 5-HT7 receptor, preferably as a full agonist.

3. Combination according to claim 1, **characterized in that** the compound B is acting as an agonist, preferably a full or a partial agonist or mixed agonist/antagonist on the µ-opioid receptor.

4. Combination according to claim 1, in which
the compound A is acting as an agonist, preferably a full or partial agonist on the 5HT7 receptor;
and
the compound B is acting as an agonist, preferably a full or partial agonist or mixed agonist/antagonist on the µ opioid receptor.

5. Combination according to any of claims 1, 3 or 4, **characterized in that** the compound B binding to the µ-opioid receptor is binding to the µ-opioid receptor with a Ki-value lower than 1000 nM, preferably lower than 200 nM, more preferably lower than 100 nM, even more preferably lower than 50 nM.

6. Combination according to any of claims 1, 3, 4 or 5, **characterized in that** the compound B binding to the µ-opioid receptor
is acting as a full or partial agonist on the µ opioid receptor;
and
is binding to the µ-opioid receptor with a Ki-value lower than 1000 nM, preferably lower than 200 nM, more preferably lower than 100 nM, even more preferably lower than 50 nM.

7. Combination according to any of claims 1, or 3 to 6, **characterized in that** the compound B binding to the µ-opioid receptor is selected from
• natural products including semi-synthetic derivatives of natural products, like morphine, codeine and thebain;
• fully synthetic compounds;
• peptides.

8. Combination according to any of claims 1, or 3 to 7, **characterized in that** the compound B binding to the µ-opioid receptor is selected from
• morphine, codeine, thebain, papaverin, narcotine,
• heroin, hydromorphone, dihydrocodeine, thebacon, hydrocodone, oxymorphone, oxycodone, ketobemidone, pethidine, anileridine, piminodine, phenoperidine, furethidine, α-prodin, trimeperidine, meptazinol, profadol, methadone, dextromoramide, levomethadyl acetate, phenadoxone, dipipanone, themalon, dextropropoxyphene, N-methylmorphinan, levorphanol, dextrometorphane, butorphanol, pentazocine, phenazocine, ketocyclazocine, bremazocine, sufentanil, carfentanil, fantanyl, lofentanil, alfentanil, ohmefentanil, remifentanil, pitramide, benztriamide, diphenoxylate, loperamide, tramadol, tilidine, U-50488, 1-Benzyl-4-(4-bromo-phenyl)-4-dimethylamino-cyclohexanol;
• alfentanil, buprenorphine, butorphanol, codeine, dextromoramide, dextropropoxyphene, dezocine, diamorphine, dihydrocodeine, diphenoxylate, ethylmorphine, etorphine, fentanyl, hydrocodone, hydromorphone, ketobemidone, levomethadone, levomethadyl-acetate, levorphanol, loperamide, meptazinol, morphine, nalbuphine, nalorphine, oxycodone, oxymorphone, pentazocine, pethidine, piritramide, remifentanil, sufentanil, tilidine, tramadol, tapentadol,
• Met-enkephalin, Leu-enkephalin, nociceptin, β-endorphin, endomorphin-1, endomorphin-2, metorphamid, dynorphin-A, dynorphin-B, α-neoendorphin.

9. Combination according to any of claims 1, 2 or 4, **characterized in that** the compound A binding to the 5-HT7 receptor is binding with a higher affinity - expressed as a Ki-value - to the 5-HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

10. Combination according to any of claims 1, 2, or 4, **characterized in that** the compound A binding to the 5-HT7 receptor is binding with a higher affinity - expressed as a Ki-value - to the 5-HT7 receptor than to the any other 5-HT receptor; especially binding with an affinity higher by a factor of at least 10, preferably with an affinity higher by a factor of at least 30, more preferably with an affinity higher by a factor of at least 50, most preferably with an affinity higher by a factor of at least 100.

11. Combination according to any of claims 1, 2, 4, or 9 to 10, **characterized in that** the compound A binding to the 5-HT7 receptor is binding to the 5-HT7 receptor with a Ki-value lower than 1000 nM, preferably lower than 200 nM, more preferably lower than 100 nM, even more preferably lower than 50 nM, very preferably lower than 25 nM, most preferably lower than 10 nM.

12. Combination according to any of claims 1, 2, 4 or 9 to 11, **characterized in that** the compound A binding to the 5-HT7 receptor
is binding to the 5-HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a Ki-value - to the 5-HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100.

13. Combination according to any of claims 1, 2, 4, or 9 to 12, **characterized in that** the compound A binding to the 5-HT7 receptor
is binding to the 5-HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a Ki-value - to the 5-HT7 receptor than to the 5-HT1A receptor; especially binding with an affinity higher by a factor of at least 10, preferably with an affinity higher by a factor of at least 30, more preferably with an affinity higher by a factor of at least 50, most preferably with an affinity higher by a factor of at least 100;
and
is acting as an agonist, preferably as a full or partial agonist on the 5-HT7 receptor, more preferably as a full agonist.

14. Combination according to any of claims 1, 2, 4, or 9 to 13, **characterized in that** the compound binding to the 5-HT7 receptor is binding to the 5-HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a Ki-value - to the 5-HT7 receptor than to any other 5-HT receptor; especially binding with an affinity higher by a factor of at least 10, preferably with an affinity higher by a factor of at least 30, more preferably with an affinity higher by a factor of at least 50, most preferably with an affinity higher by a factor of at least 100.

15. Combination according to any of claims 1, 2, 4 or 9 to 14, **characterized in that** the compound binding to the 5-HT7 receptor is binding to the 5-HT7 receptor with a Ki-value lower than 1000 nM, preferably lower than 200 nM, more preferably lower than 100 nM, even more preferably lower than 50 nM, very preferably lower than 25 nM, most preferably lower than 10 nM
and
is binding with a higher affinity - expressed as a Ki-value - to the 5-HT7 receptor than to any other 5-HT receptor; especially binding with an affinity higher by a factor of at least 10, preferably with an affinity higher by a factor of at least 30, more preferably with an affinity higher by a factor of at least 50, most preferably with an affinity higher by a factor of at least 100;
and
is acting as an agonist, preferably as a full or partial agonist on the 5-HT7 receptor, more preferably as a full agonist.

16. Combination according to any of claims 1, 2, 4 or 9 to 15, **characterized in that** the compound A binding to the 5-HT7 receptor is AS-19 or MSD5a, especially AS-19, optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate.

17. Combination according to any of claims 1, 2, 4 or 9 to 16, **characterized in that** the compound A binding to the 5-HT7 receptor is selected from
Compounds of Chemical Group 1, Heterocyclyl-substituted-ethylamino-phenyl derivative of general formula (l/1) 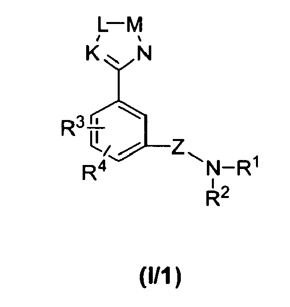 wherein
K-L-M-N together form
• =CH-X-Y=CH-; in which any suitable H may be substituted by R⁶ and/or R⁷, and in which X is selected from NR⁸, O or S, while Y is selected from N or CH;
• =CH-X-Y-C(O)-; in which any suitable H may be substituted by R⁶ and in which one of X and Y is NR⁸, while the other is selected from NR^{8a}, S or O;
• =CH-X-Y-C(O)-; in which one of X and Y is CH₂, while the other is selected from NR⁸, S or O, in which any suitable H may be substituted by R⁶ and/or R⁷;
• =CR⁶-N=N-C(O)-;
• =CR⁹-CH=CH-CH=CH-; in which any suitable H may be substituted by R⁶;
• =CR⁹-CH=CH-CH=CR^{9a}-; in which any suitable H may be substituted by R⁶;
• =CH-X=Y-CH=CH-; in which any suitable H may be substituted by R⁶ and/or R⁷, and in which one of X or Y is selected from N, while the other is selected from N or CH;
• =CH-X=Y-CH₂-CH₂-; in which any suitable H may be substituted by R⁶ and/or R⁷, and in which one of X or Y is selected from N, while the other is selected from N or CH;
• =CH-X-Y-CH=CH-; in which any suitable H may be substituted by R⁶ and/or R⁷, and in which one of X or Y is selected from NR⁸, O or S while the other is selected from NR^{8a} or CH₂;
• =CH-X-Y-CH₂-CH₂-; in which any suitable H may be substituted by R⁶ and/or R⁷, and in which one of X or Y is selected from NR⁸, O or S while the other is selected from NR^{8a} or CH₂;
• =CH-X-CH₂-Y=CH-; in which any suitable H may be substituted by R⁶ and/or R⁷, and in which X is selected from NR⁸, O or S while Y is selected from N or CH;
• =CH-X-CH=Y-CH₂-; in which any suitable H may be substituted by R⁶ and/or R⁷, and in which X is selected from NR⁸, O or S while Y is selected from N or CH;
• =CH-N=CH-Y=CH-; in which any suitable H may be substituted by R⁶ and/or R⁷;
• =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
• -(CH₂)ₙ-, with n being 1, 2, 3 or 4;
• -O-(CH₂)ₙ-, with n being 1, 2, 3 or 4;
• -S-(CH₂)ₙ-, with n being 1, 2, 3 or 4;
• (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
or
Compounds of Chemical Group 2, heterocyclyl-substituted- tetrahydro-naphthalen derivative of general formula (I/2) or its benzyl-substituted analogue of general formula (Iₚᵣₒₜ/2) 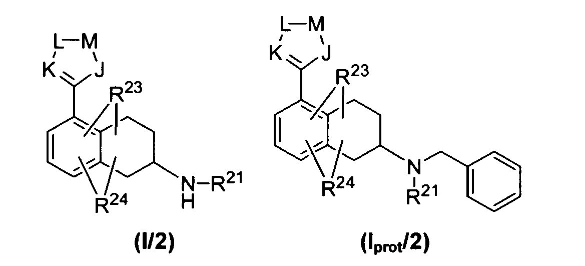 wherein
K-L-M-J together form
• =CH-X-Y=CH-, in which any suitable H may be substituted by R²⁶ and/or R²⁷, and in which X is selected from NR²⁸, O or S, while Y is selected from N
or
CH;
• =CH-X-Y-C(O)-, in which any suitable H may be substituted by R²⁶ and in which one of X and Y is NR²⁸, while the other is selected from NR^{28a}, S or O;
• =CH-X-Y-C(O)-, in which one of X and Y is CH₂, while the other is selected from NR²⁸, S or O, in which any suitable H may be substituted by R²⁶ and/or R²⁷;
• =CR²⁶-N=N-C(O)-; or
• =CR²⁹-X₁=Y-X₂=CR^{29a}-, in which two of Y, X₁ and X₂ are CH, while the other is selected from CH or N, in which any suitable H may be substituted by R²⁶;
R²¹ is selected from the group consisting of hydrogen; a linear or branched, saturated or unsaturated, optionally at least mono-substituted aliphatic radical; or an optionally at least mono-substituted alkyl-aryl;
R²³ and R²⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R²⁶ and R²⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
R²⁸ and R^{28a} are independently from each other selected from hydrogen; or an aliphatic radical, which is linear or branched, saturated or unsaturated, and optionally at least mono-substituted by F, Cl, Br, I, SH or OH;
R²⁹ and R^{29a} are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
**or**
Compounds of Chemical Group 3, heterocyclyl-substituted-tetrahydro-naphthalen-amine derivatives of general formula (I/3) 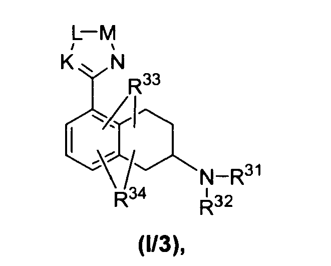 wherein
K-L-M-J together form
• =CH-X-Y=CH-, in which any suitable H may be substituted by R³⁶ and/or R³⁷, and in which X is selected from NR³⁸, O or S, while Y is selected from N
or
CH;
• =CH-X-Y-C(O)-, in which any suitable H may be substituted by R³⁶ and in which one of X and Y is NR³⁸, while the other is selected from NR^{38a}, S or O;
• =CH-X-Y-C(O)-, in which one of X and Y is CH₂, while the other is selected from NR³⁸, S or O, in which any suitable H may be substituted by R³⁶ and/or R³⁷;
• =CR³⁶-N=N-C(O)-; or
• =CR³⁹-X₁=Y-X₂=CR^{39a}-, in which two of Y, X₁ and X₂ are CH, while the other is selected from CH or N, in which any suitable H may be substituted by R³⁶;
R³¹ and R³² are independently from each other a linear or branched, saturated or unsaturated, optionally at least mono-substituted aliphatic radical;
or
R³¹ and R³² together with their connecting nitrogen are forming an optionally at least mono-substituted heterocyclic ring system;
R³³ and R³⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R³⁶ and R³⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
R³⁸ and R^{38a} are independently from each other selected from hydrogen; or an aliphatic radical, which is linear or branched, saturated or unsaturated, and optionally at least mono-substituted by F, Cl, Br, I, SH or OH;
R³⁹ and R^{39a} are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

18. Combination according to any of claims 1, 2, 4 or 9 to 17, **characterized in that** the compound A binding to the 5-HT7 receptor is selected from
compounds of Chemical Group 1, preferably from compounds of Chemical Group 1 according to formula la/1 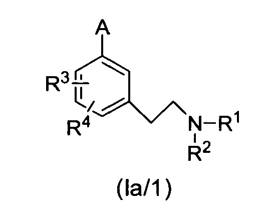 , wherein
A is a compound selected from the following group 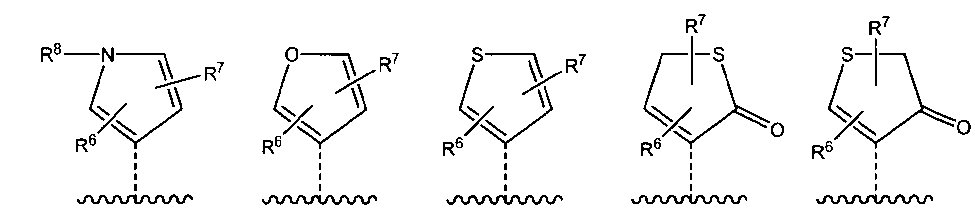 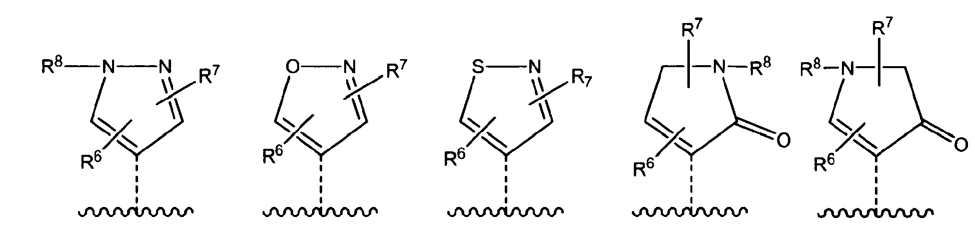 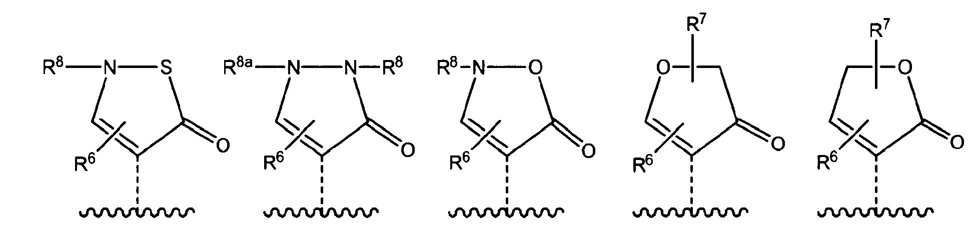 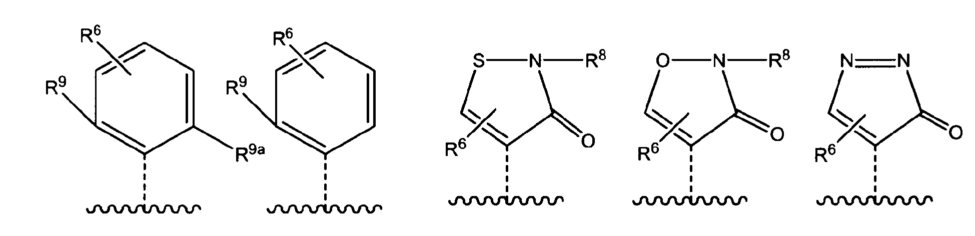 R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
most preferably from compounds of Chemical Group 1, selected from
• Dimethyl-{2-[3-(1,3,5-trimethyl-1H-pyrazol-4-yl)-phenyl]-ethyl}-amine.
• Methyl-{2-[3-(1,3,5-trimethyl-1H-pyrazol-4-yl)-phenyl]-ethyl}-amine,
• Diethyl-{2-[3-(1,3,5-trimethyl-1H-pyrazol-4-yl)-phenyl]-ethyl}-amine,
• Dipropyl-{2-[3-(1,3,5-tdmethyl-1H-pyrazol-4-yl)-phenyl]-ethyl}-amine,
• {2-[3-(3,5-Dimethyl-1H-pyrazol-4-yl)-phenyl]-ethyl}-dimethyl-amine,
• {2-[3-(3,5-Dimethyl-1H-pyrazol-4-yl)-phenyl]-ethyl}-methyl-amine,
• {2-[3-(3,5-Dimethyl-1H-pyrazol-4-yl)-phenyl]-ethyl}-diethyl-amine,
• {2-[3-(3,5-Dimethyl-1H-pyrazol-4-yl)-phenyl]-ethyl}-dipropyl-amine,
• Dimethyl-{2-[3-(1-methyl-1H-pyrazol-4-yl)-phenyl]-ethyl}-amine,
• Methy)-{2-[3-(1-rnethy)-1H-pyrazo)-4-yl)-phenyl]-ethyl}-amine,
• Diethyl-{2-[3-(1-methyl-1H-pyrazol-4-yl)-phenyl]-ethyl}-amine,
• {2-[3-(1-Methyl-1H-pyrazol-4-yl)-phenyl]-ethyl}-dipropyl-amine,
• {2-[3-(3,5-Dimethyl-isoxazol-4-yl)-phenyl]-ethyl}-dimethyl-amine,
• {2-[3-(3,5-Dimethyl-isoxazol-4-yl)-phenyl]-ethyl}-methyl-amine,
• {2-[3-(3,5-Dimethyl-isoxazo1-4-yl)-phenyl]-ethyl}-diethyl-amine, or
• {2-[3-(3,5-Dimethyl-isoxazol-4-yl)-phenyl]-ethyl}-dipropyl-amine,
optionally in form of a salt, preferably a physiologically acceptable salt, more preferably in form of a physiologically acceptable acid addition salt, most preferably a hydrochloride salt, or a corresponding solvate.

19. Combination according to any of claims 1, 2, 4 or 9 to 17, **characterized in that** the compound A binding to the 5-HT7 receptor is selected from
compounds of Chemical Group 2, preferably from compounds of Chemical Group 2 according to formula la/2 or laₚᵣₒₜ/2 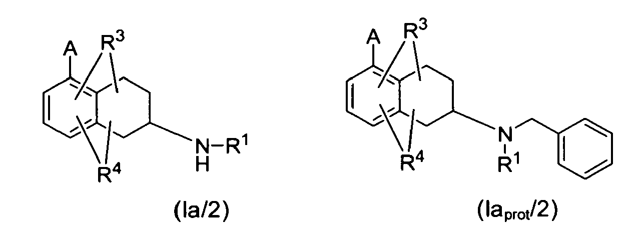 , wherein
A is a compound selected from the following group 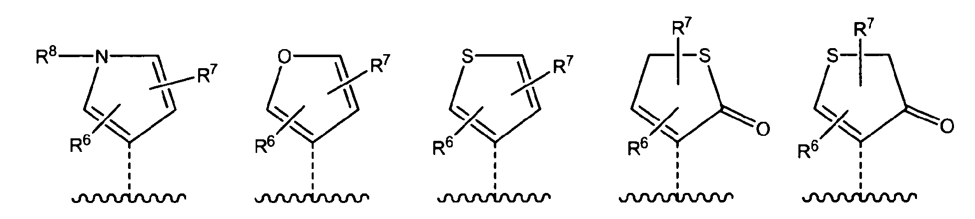 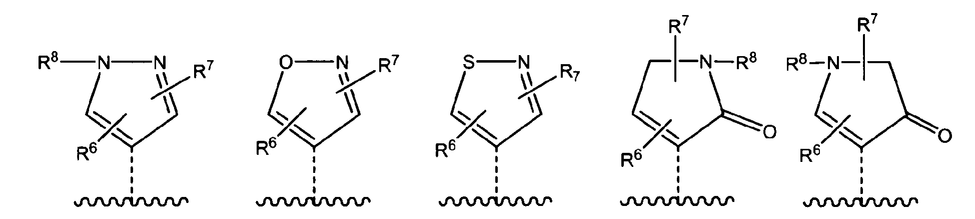 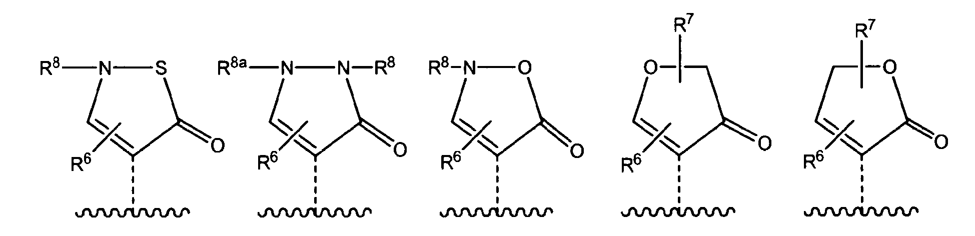 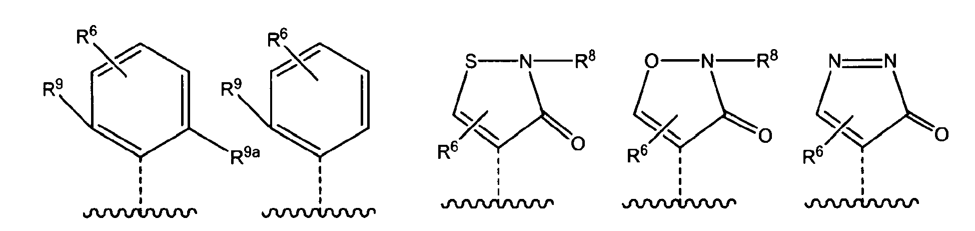 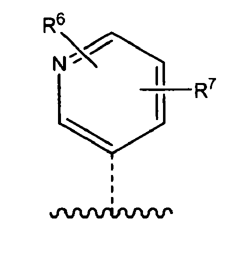 R²¹ is selected from the group consisting of hydrogen; a linear or branched, saturated or unsaturated, optionally at least mono-substituted aliphatic radical; or an optionally at least mono-substituted alkyl-aryl;
R²³ and R²⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R²⁶ and R²⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
R²⁸ and R^{28a} are independently from each other selected from hydrogen; or an aliphatic radical, which is linear or branched, saturated or unsaturated, and optionally at least mono-substituted by F, Cl, Br, I, SH or OH;
R²⁹ and R^{29a} are independently from each other selected from halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
most preferably from compounds of Chemical Group 2 selected from
• Methyl-[5-(1,3,5-trimethyl-1H-pyrazol-4-yl)-1,2,3,4-tetrahydro-naphthalen-2-yl]-amine,
• (2S)-Methyl-[5-(1,3,5-trimethyl-1H-pyrazol-4-yl)-1,2,3,4-tetrahydro-naphthalen-2-yl]-amine,
• Benzyl-[5-(1,3,5-trimethyl-1H-pyrazol-4-yl)-1,2,3,4-tetrahydro-naphthalen-2-yl]-amine,
• (2*S*)-Benzyl-[5-(1,3,5-trimethyl-1H-pyrazol-4-yl)-1,2,3,4-tetrahydro-naphthalen-2-yl]-amine,
• Benzyl-methyl-[5-(1,3,5-trimethyl-1H-pyrazol-4-yl)-1,2,3,4-tetrahydro-naphthalen-2-yl]-amine,
• (2S)-Benzyl-methyl-[5-(1,3,5-trimethy)-1H-pyrazol-4-yl)-1,2,3,4-tetrahydro-naphthalen-2-yl]-amine;
• 5-(1,3,5-Trimethyl-1H-pyrazol-4-yl)-1,2,3,4-tetrahydro-naphthalen-2-ylamine hydrochloride;
or
• (2S)-5-(1,3,5-Trimethy)-1H-pyrazol-4-yl)-1,2,3,4-tetrahydro-naphthalen-2-ylamine hydrochloride;
optionally in form of a salt, preferably a physiologically acceptable salt, more preferably in form of a physiologically acceptable acid addition salt, most preferably a hydrochloride salt, or a corresponding solvate.

20. Combination according to any of claims 1, 2, 4 or 9 to 17, **characterized in that** the compound A binding to the 5-HT7 receptor is selected from
compounds of Chemical Group 3, preferably from compounds of Chemical Group 3 according to formula la/3 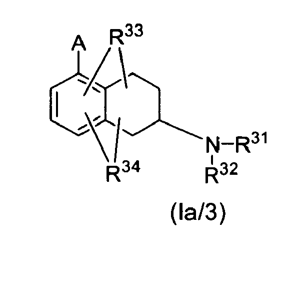 , wherein
A is a compound selected from the following group 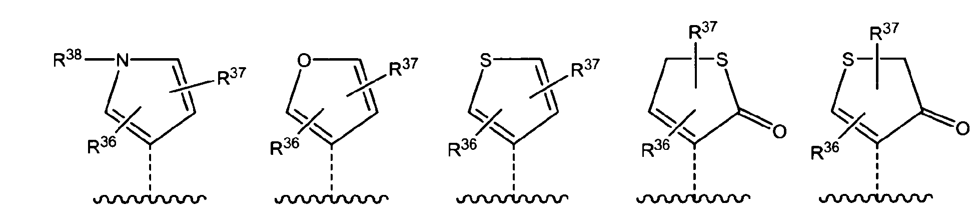 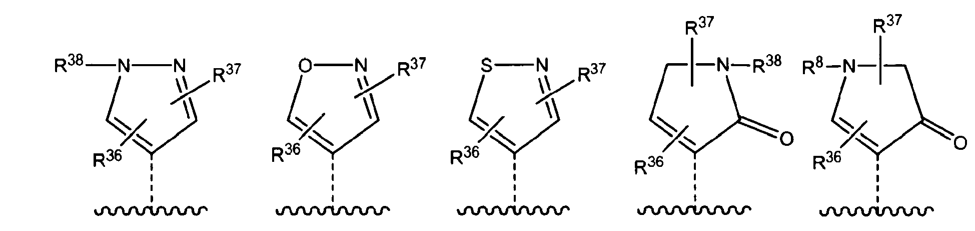 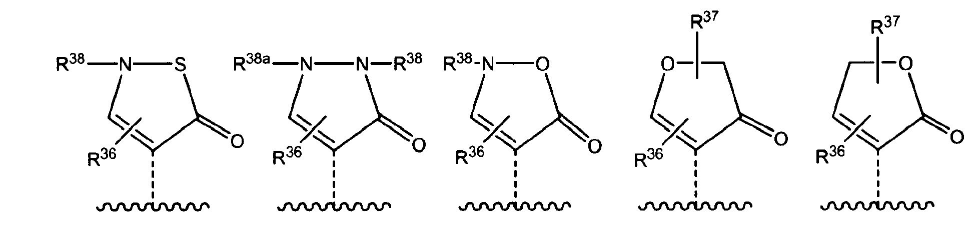 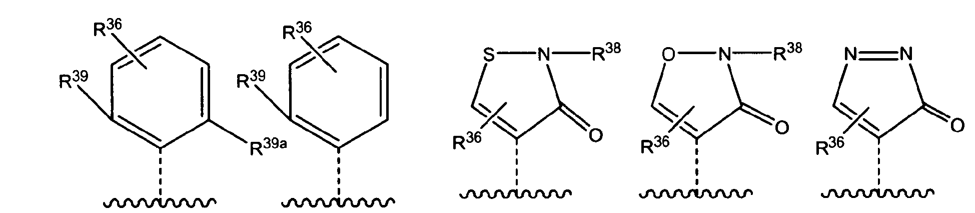 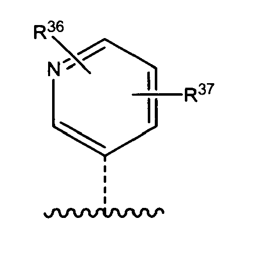 R³¹ and R³² are independently from each other a linear or branched, saturated or unsaturated, optionally at least mono-substituted aliphatic radical;
or
R³¹ and R³² together with their connecting nitrogen are forming an optionally at least mono-substituted heterocyclic ring system;
R³³ and R³⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R³⁶ and R³⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or OR with R being an aliphatic radical, which is linear or branched, saturated or unsaturated, and optionally at least mono-substituted by F, Cl, Br, I, SH or OH;
R³⁸ and R^{38a} are independently from each other selected from hydrogen; or an aliphatic radical, which is linear or branched, saturated or unsaturated, and optionally at least mono-substituted by F, Cl, Br, I, SH or OH;
R³⁹ and R^{39a} are independently from each other selected from halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
most preferably from compounds of Chemical Group 3 selected from
• 1-[5-(1,3,5-Trimethyl-1H-pyrazol-4-yl)-1,2,3,4-tetrahydro-naphthalen-2-yl]-piperazine;
• 1-Methyl-4-[5-(1,3,5-trimethyl-1H-pyrazol-4-yl)-1,2.3,4-tetrahydro-naphthalen-2-yl]-piperazine;
• 1,3,5-Trimethyl-4-(6-pyrrolidin-1-yl-5,6,7,8-tetrahydro-naphthalen-1-yl)-1H-pyrazole;
• 1-[5-(1,3,5-Trimethyl-1H-pyrazol-4-yl)-1,2,3,4-tetrahydro-naphthalen-2-yl]-piperidine;
• Dipropyl-[5-(1,3,5-trimethyl-1H-pyrazol-4-yl)-1,2,3,4-tetrahydro-naphthalen-2-yl]-amine;
• Methyl-propyl-[5-(1,3,5-trimethyl-1H-pyrazol-4-yl)-1,2,3,4-tetrahydro-naphtha len-2-yl]-a mine;
• Diethyl-[5-(1,3,5-trimethyl-1H-pyrazol-4-yl)-1,2,3,4-tetrahydro-naphthalen-2-yl]-amine;
• Ethyl-methyl-[5-(1,3,5-trimethyl-1H-pyrazol-4-yl)-1,2,3,4-tetrahydro-naphthalen-2-yl]-amine;
• Dimethyl-[5-(1-methyl-1H-pyrazol-4-yl)-1,2,3,4-tetrahydro-naphthalen-2-yl]-amine;
• [5-(3,5-Dimethyl-isoxazol-4-yl)-1,2,3,4-tetrahydro-naphthalen-2-yl]-dimethyl-amine;
• (5-Furan-3-yl-1,2,3,4-tetrahydro-naphthalen-2-yl)-dimethyl-amine;
• Dimethyl-(5-thiophen-3-yl-1,2,3,4-tetrahydro-naphthalen-2-yl)-amine;
• [5-(2,6-Dimethyl-phenyl)-1,2,3,4-tetrahydro-naphthalen-2-yl]-dimethyl-amine;
• [5-(2,6-Difluoro-phenyl)-1,2,3,4-tetrahydro-naphthalen-2-yl]-dimethyl-amine;
• Dimethyl-(5-pyridin-3-yl-1,2,3,4-tetrahydro-naphthalen-2-yl)-amine;
optionally in form of one of its stereoisomers, preferably enantiomers or diastereomers, its racemate or in form of a mixture of at least two of its stereoisomers, preferably enantiomers or diastereomers, in any mixing ratio, optionally in form of a salt, preferably a physiologically acceptable salt, more preferably in form of a physiologically acceptable acid addition salt, most preferably a hydrochloride salt, or a corresponding solvate.

21. Medicament comprising a combination according to any of claims 1 to 20, and optionally one or more pharmaceutically acceptable adjuvants.

22. Use of at least one combination according to claims 1 to 20, for the manufacture of a medicament for the treatment of pain, preferably visceral pain, chronic pain, cancer pain, migraine, acute pain or neuropathic pain, allodynia or hyperalgesia.

23. Use according to claim 22 wherein the stimulus evoking the pain is mechanical.

24. Use according to claim 22 wherein the stimulus evoking the pain is thermal.

25. Use, according to any of claims 22 to 24, **characterized in that** the neuropathic pain, is selected from central pain, hyperpathia, peripheral neuropathic pain or peripheral neurogenic pain, causalgia, hyperesthesia, neuralgia, neuritis, or neuropathy.

26. Use of at least one combination according to claims 1 to 20, for the manufacture of a medicament for the treatment of sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.
